# FASCICULE DE BREVET EUROPEEN

(11) **EP 1 165 491 B1**
(45) Date de publication et mention de la délivrance du brevet: **11.02.2004**
(21) Numéro de dépôt: 00917145.5
(22) Date de dépôt: 06.04.2000
(51) Int. Cl.: C07C 229/12, C07F 9/38, A61P 29/00

(54) **INHIBITEURS DE LTA4 HYDROLASE**
LTA4 HYDROLASE INHIBITOREN
LTA4 HYDROLASE INHIBITORS

(30) Priorité: 06.04.1999 FR 9904271
(43) Date de publication de la demande: 02.01.2002
(73) Titulaire: INSTITUT NATIONAL DE LA SANTE ET DE LA RECHERCHE MEDICALE (INSERM), 75013 Paris (FR); BIOPROJET, 75003 Paris (FR)
(72) Inventeur: DANVY, Denis, F-76190 Yvetot (FR); MONTEIL, Thierry, F-76690 Saint Georges sur Fontaine (FR); PLAQUEVENT, Jean-Christophe, F-76960 Notre-Dame de Bondeville (FR); DUHAMEL, Pierre, F-76130 Mont-Saint-Aignan (FR); DUHAMEL, Lucette, F-76130 Mont-Saint-Aignan (FR); NOEL, Nadine, F-68480 Moernach (FR); GROS, Claude, F-75015 Paris (FR); CHAMARD, Olivier, F-93600 Aulnay sous Bois (FR); SCHWARTZ, Jean-Charles, F-75014 Paris (FR); LECOMTE, Jeanne-Marie, F-75003 Paris (FR); PIETTRE, Serge, F-76270 Saint Martin l'Hortier (FR)
(74) Mandataire: Bernasconi, Jean Raymond
(86) Numéro de dépôt international: PCT/FR2000/000876
(87) Numéro de publication internationale: WO 2000/059864

(56) Documents cités:
- WO-A-94/00420
- WO-A-98/17627
- WO-A-98/40364
- US-A- 4 071 552
- CHEMICAL ABSTRACTS, vol. 99, no. 23, 5 décembre 1983 (1983-12-05) Columbus, Ohio, US; abstract no. 195369, WITCZUK, BARBARA ET AL: "Resolution of racemic 2-amino-4-(aryloxy)butanoic acids into enantiomers and determination of their absolute configuration" XP002130916 & POL. J. CHEM. (1981), 55(7-8), 1511-18,
- TINLAND, B.: "Structure-activity relations for homoserine derivatives as inhibitors of the enzymic synthesis of S-adenosyl-L-methionine" FARMACO, ED. SCI. (1976), 31(8), 596-8, XP000876749
- HULBERT, PETER B.: "Multiple regression analyses of the relations between structure and inhibitory effects of a series of substituted O-phenyl-DL- homoserines on adenosine triphosphate:L-methionine S-adenosyltransferase. Addendum" MOL. PHARMACOL. (1974), 10(2), 315-18, XP000879018
- COULTER, A. W. ET AL: "Structural and conformational analogs of L-methionine as inhibitors of the enzymic synthesis of S-adenosyl-L-methionine. II. Aromatic amino acids" MOL. PHARMACOL. (1974), 10(2), 305-14, XP000879197
- SARDA, N. ET AL: "New synthesis of 3-aminochroman. Synthesis and absolute configuration of its enantiomers" TETRAHEDRON LETT. (1976), (4), 271-2, XP000867682
- AMBLARD, MURIEL ET AL: "Synthesis and biological evaluation of cholecystokinin analogs in which the Asp-Phe-NH2 moiety has been replaced by a 3-amino-7-phenylheptanoic acid or a 3-amino-6-(phenyloxy)hexanoic acid" J. MED. CHEM. (1993), 36(20), 3021-8, XP000876721

## Description

La présente invention concerne des composés tels que définis ci-après, constituant une classe de médicaments présentant principalement une activité anti-inflammatoire et/ou agissant par inhibition de la LTA₄ (leucotriène A₄) hydrolase, enzyme responsable de la biosynthèse du leucotriène LTB₄, un médiateur pro-inflammatoire important.

Elle concerne encore de tels composés utiles sous forme de prodrogues.

Elle concerne également des procédés de préparation de ces composés.

La LTA₄ hydrolase (EC 3.3.2.6.) est une enzyme notamment présente dans les neutrophiles dont on a récemment montré que la séquence (Funck et coll., P.N.A.S., 1987, 89: 6677) s'apparentait à celle d'une métallopeptidase à zinc, l'aminopeptidase M (Malfroy et al., B.B.R.C., 1989, 161: 236). En accord avec la suggestion de Malfroy et coll., la LTA₄ hydrolase a été reconnue posséder un atome de zinc essentiel à son activité catalytique, une activité de type aminopeptidasique et une sensibilité à l'action de certains inhibiteurs de métallopeptidases (Heggstrom et coll., B.B.R.C., 1990, 173: 431 ; Minami et coll., B.B.R.C., 1990, 173: 620).

L'inhibition de la LTA₄ hydrolase est de nature à prévenir la formation de LTB₄, un médiateur responsable de l'adhésion des neutrophiles aux cellules endothéliales et de leur chimiotactisme. Il paraît impliqué dans l'étiologie ou la symptomatologie d'une variété d'états inflammatoires et d'affections telles qu'arthrite rhumatoïde, inflammations chroniques de l'intestin, sclérose en plaques, goutte, psoriasis. Dans ces processus, le LTB₄ pourrait agir en synergie avec d'autres métabolites de l'acide arachidonique produits par la 5-lipoxygénase ou les cyclo-oxygénases dont l'inhibition est bien connue pour produire des effets anti-inflammatoires.

Certains composés inhibiteurs de la LTA₄ hydrolase ont été décrits, notamment dans les demandes de brevet WO 94/00420, WO 96/11192, WO 96/10999, WO 96/27585, WO 96/41625, WO 98/40354, WO 98/40364, WO 98/40370, WO 98/09943 et WO 98/43954.

L'objectif de la présente invention est de fournir de nouveaux composés susceptibles d'inhiber la LTA₄ hydrolase.

L'objectif de la présente invention est également de fournir des composés pouvant être utilisés comme médicaments.

A cette fin, l'invention a pour objet l'utilisation de composés de formule (I) telle que définie ci-dessous comme inhibiteur de l'activité de la LTA₄ hydrolase, notamment comme anti-inflammatoires.

L'invention a aussi pour objet l'utilisation de ces composés de formule (I) sous forme de prodrogues.

Ces composés répondent à la formule (I) suivante : 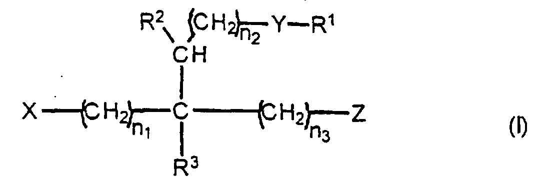 dans laquelle
- X est choisi parmi les groupes suivants :
   i)

      -NH₂
   ii) 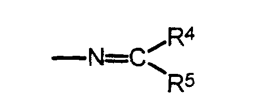
- n₁ et n₃ sont égaux à 0 ou 1, avec (n₁+n₃) égal à 0 ou 1
- n₂ varie de 0 à 10
- Y est choisi parmi les groupes suivants :
   i)

      ―O―
   ii)

      ―CH₂―
   iii)

      ―S―
   iv)

      ―NH―
   v)

      ―OCH₂―
- R¹ est choisi parmi les groupes suivants :
   i) un atome d'hydrogène
   ii) un groupe alkyle inférieur
   iii) un groupe cycloalkyle
   iv) un groupe phényle, non substitué, ou mono ou polysubstitué avec des substituants choisis parmi les atomes d'halogène et les groupes CF₃, alkyle inférieur, alcoxy inférieur, NH₂, NO₂, CN, OH, CO₂H, OPh, OCH₂Ph, SCH₃, SCH₂CH₃ et NHCOR⁶.
   v) un groupe α- ou β-naphtyle
   vi) un groupe anthracène
   vii)

      ―A²―(CH₂)ₙ₄―A¹

      où
      n₄ varie de 0 à 4.
      A¹ et A² sont indépendamment choisis parmi les groupes suivants :
      a) cycloalkyle
      b) phényle, non substitué, ou mono ou polysubstitué avec des substituants choisis parmi les atomes d'halogène et les groupes CF₃, alkyle inférieur et alcoxy inférieur,
      c) 2-, 3- ou 4-pyridyle
      d) 2- ou 3-thiényle
      e) 2- ou 3-furyle
      f) 2-, 3- ou 4-pipéridyle
      g) cycloalcène
   viii) un groupe 2-, 3- ou 4-pyridyle
   ix) un groupe 2- ou 3-thiényle
   x) un qroupe 2- ou 3-furyle
   xi) 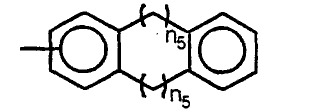
- Z est choisi parmi les groupes suivants :
   i)

      ―COOR⁷
   ii) 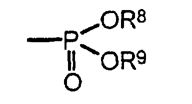
   iii) 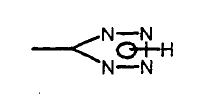
   iv) 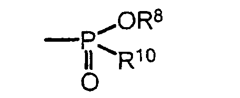
   v)

      ―SO₃H ;
   vi)

      ―SO₂NHR¹¹
   vii)

      ―CONHSO₂R¹¹
- R² et R³ sont indépendamment choisis parmi les groupes suivants :
   i) un atome d'hydrogène
   ii) un groupe alkyle inférieur
   iii) un groupe alkyle inférieur substitué par un atome d'halogène
   iv) un groupe CF₃
   v) un atome d'halogène
- R⁴ et R⁵ sont indépendamment choisis parmi un atome d'hydrogène, un groupe alkyle inférieur, un groupe phényle non substitué ou substitué par un atome d'halogène, un groupe CF₃, un groupe alkyle inférieur, un groupe alcoxy inférieur et un groupe OH.
- n₅ varie de 0 à 2
- R⁶ représente un groupe alkyle inférieur
- R⁷ représente un atome d'hydrogène, un groupe alkyle inférieur, un groupe ―(CH₂)ₙ₆―Ph, n₆ variant de 0 à 4 et Ph étant un groupe phényle non substitué ou mono ou polysubstitué par un atome d'halogène, un groupe CF₃, un alkyle inférieur, un alcoxy inférieur ou un groupe OH.
- R⁸ et R⁹ sont indépendamment choisis parmi un atome d'hydrogène, un groupe phényle, un groupe alkyle inférieur et un groupe acéthylthioalkylène inférieur.
- R¹⁰ représente un groupe alkyle inférieur, un groupe ―(CH₂)ₙ₇―Ph, n₇ variant de 1 à 6 et Ph étant un groupe phényle non substitué ou mono ou polysubstitué par un atome d'halogène, un groupe CF₃, un alkyl inférieur ou un alcoxy inférieur.
- R¹¹ représente un groupe alkyle inférieur ou un groupe phényle.

Par groupe alkyle inférieur, on entend un groupe alkyle à chaîne linéaire ou ramifiée contenant de 1 à 10 atomes de carbone, de préférence 1 à 4 atomes de carbone.

Par groupe alcoxy inférieur, on entend un groupe alcoxy contenant une chaîne linéaire ou ramifiée de 1 à 10 atomes de carbone, de préférence 1 à 4 atomes de carbone.

Par groupe cycloalkyle, on entend un cycle contenant de 5 à 7 atomes de carbone, de préférence 6 atomes de carbone, tels que le cyclopentane, cyclohexane ou cycloheptane.

Par groupe cycloalcène, on entend un cycle contenant de 5 à 7 atomes de carbone et contenant une double liaison, de préférence 6 atomes de carbone, tel que le cyclohexène.

Par groupe acétylthioalkylène inférieur on entend un groupe acétylthio à chaîne linéaire contenant de 1 à 4 atomes de carbone, de préférence 1 à 2 atomes de carbone.

Les atomes d'halogènes sont de préférence choisis parmi le chlore et le fluor.

L'invention comprend également les isomères des composés de formule (I) incluant les formes diastéréoisomères et énantiomères.

L'invention s'étend aussi aux sels thérapeutiquement acceptables de ces composés ainsi qu'aux sels de leurs isomères incluant les formes diastéréoisomères et énantiomères.

Par sels thérapeutiquement acceptables, on entend un sel n'altérant ni la structure chimique, ni les propriétés pharmacologiques des composés de la présente invention. De tels sels incluent les anions minéraux et organiques tels que le chlorhydrate, bromhydrate, acétate, trifluoroacétate, maléate, fumarate, oxalate, etc., bien connus dans la technique. Ces sels sont préparés de manière conventionnelle par neutralisation des composés de formule (I) avec l'acide désiré.

L'invention a également pour objet les composés de formule (I) en tant que tels, à l'exception :
(α) des composés dans lesquels Z est un groupement de type COOR⁷ et n₁=n₃=0 et R²=H et R¹ est un groupe iv) de type phényl non substitué, ou mono ou polysubstitué, et dans lesquels n₂=1, et
(β) des composés suivants : l'acide α-amino-β-phénoxy-propionique, l'acide 3-amino-7-phényl-heptanoïque, l'acide 3-amino-6-phénoxy-hexanoïque, l'acide α-amino-6-phényl-hexanoïque, et l'acide α-amino-5-phénoxy-pentanoïque.

L'invention a également pour objet des compositions pharmaceutiques comprenant au moins un tel composé.

Les inventeurs ont mis en évidence que les composés de formule (I) définie ci-dessus, ou leurs sels obtenus avec des acides minéraux ou organiques thérapeutiquement acceptables ou leurs stéréoisomères, possèdent une puissante activité inhibitrice de la LTA₄ hydrolase.

Les composés (I) présentent par ailleurs une bonne biodisponibilité et se sont révélés faiblement toxiques.

La présente invention décrit une série de composés capables d'inhiber puissamment la LTA₄ hydrolase.

Ces composés présentent en outre une activité biologique telle qu'indiquée ci-après qui leur confère un intérêt thérapeutique.

Selon un premier aspect de l'invention, un groupe préféré de composés de formule (I) précitée comprend ceux pour lesquels X représente NH₂ et/ou Z représente le groupe ―COOR⁷ avec R⁷ représentant un atome d'hydrogène.

Dans ce groupe, les composés de formule (I) dans laquelle X est NH₂ et Z est COOH, sont plus particulièrement préférés.

Les composés de formule (I) pour lesquels R² et/ou R³ représente un atome d'hydrogène constituent également un sous-groupe particulièrement préféré selon l'invention.

R² et R³ sont de préférence chacun un atome d'hydrogène.

Les composés de formule (I) avec R² et/ou R³ différent de l'hydrogène représente un autre sous-groupe selon l'invention.

Une sous-famille parmi les composés précités est formée par les composés pour lesquels n₁ et n₃ sont égaux à 0.

Une autre sous-famille est constituée par les composés pour lesquels n₁ ou n₃ est différent de 0.

Une sous-classe de composés selon l'invention est encore constituée par ceux pour lesquels n₂ = 0. Parmi ces composés Y représente de préférence ―O―.

Une autre sous-classe est formée par les composés pour lesquels n₂ varie de 1 à 5, de préférence de 2 à 5 et de manière plus particulièrement préférée pour les composés où n₂ = 3.

Une autre classe de composés selon l'invention est définie par ceux où n₂ est supérieur à 5.

Du point de vue du symbole Y, les composés pour lesquels celui-ci représente un atome d'oxygène sont particulièrement préférés selon l'invention.

D'autres sous-familles peuvent être définies selon que Y représente ―CH₂―, un atome de soufre, un motif ―NH― ou ―OCH₂―.

R¹ est choisi de préférence parmi un groupe phényle non substitué ou substitué, plus préférentiellement monosubstitué par un des substituants précités.

Lorsque R¹ symbolise un groupe phényle substitué, le ou les substituants sont de préférence choisis parmi les groupes alkyle inférieur, alcoxy inférieur, OPh et OCH₂Ph.

Les composés pour lesquels R¹ est un groupe phényle mono ou polysubstitué par un groupe OPh constituent une sous-famille préférée selon l'invention.

Lorsque R¹ représente un motif -A²-(CH₂)ₙ₄-A¹, A² est plus préférentiellement un groupe phényle, de préférence non-substitué.

Parmi ces composés, n₄ est de préférence égal à 0 ou 1 et A¹ est choisi préférentiellement parmi un groupe phényle, cycloalkyle et cycloalcène.

R¹ représentant un motif A²-(CH₂)ₙ₄-A¹ est de préférence un groupe phényle substitué par un groupe Ph, CH₂Ph, CH₂-cycloalkyle ou CH₂-cycloalcène, plus préférentiellement CH₂Ph ou CH₂-cycloalkyle.

Une autre sous-famille comprend les composés (I) pour lesquels R¹ représente un atome d'hydrogène ou un groupe alkyle inférieur.

Une autre sous-famille comprend les composés (I) pour lesquels R¹ est un groupe cycloalkyle.

Les composés (I) avec R¹ représentant un groupe α- ou β-naphtyle, ou un groupe anthracène forment encore une autre sous-famille.

Un autre groupe de composés selon l'invention comprend les composés (I) avec R¹ représentant un groupe 2-, 3 ou 4-pyridyle, 2- ou 3-thiényle et 2- ou 3-furyle.

Les composés (I) avec R¹ représentant 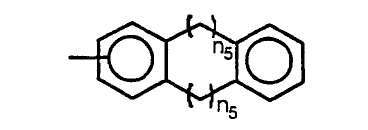 forment aussi un autre sous-groupe selon l'invention.

Pour toutes les sous-familles mentionnées ci-dessus, les substituants non précisées peuvent varier selon leurs définitions générales respectives.

Un groupe particulièrement préféré de composés selon l'invention est constitué des composés répondant à la formule (II) suivante : 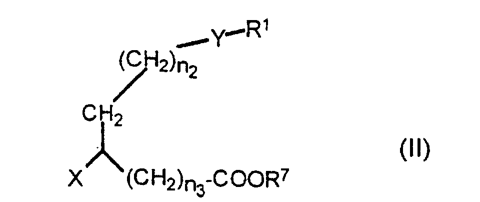 dans laquelle X, n₂, n₃, Y, R¹ et R⁷ ont la signification précédente.

Les préférences indiquées précédemment pour les composés de formule (I) s'appliquent également à ceux de formule (II).

Un groupe encore plus particulièrement préféré comprend les composés répondant à la formule (III) suivante : 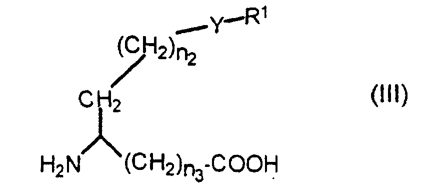 dans laquelle n₂, n₃, Y et R¹ ont la signification précédemment indiquée.

Les choix particuliers mentionnés pour les composés de formule (I) du point de vue des symboles Y et R¹ s'appliquent également aux composés de formules (III).

Parmi ces composés, ceux répondant à la formule (IV) suivante : 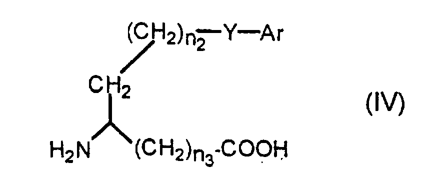 où Y, n₂ et n₃ ont la signification donnée précédemment et Ar symbolise le groupe R¹ représentant un groupe phényle iv) éventuellement substitué tel que défini précédemment ou R¹ représentant un groupe vii) -A²-(CH₂)ₙ₄-A¹, A² étant un groupe phényle (b) éventuellement substitué tel que défini précédemment, sont particulièrement préférés.

Selon un second aspect de l'invention, un groupe préféré de composés de formule (I) précitée comprend ceux pour lesquels X représente NH₂ et/ou Z représente le groupe 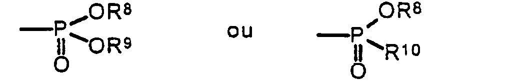 R⁸, R⁹ étant un atome d'hydrogène, et R¹⁰ ayant la signification précitée.

Dans ce groupe, les composés de formule (I) dans laquelle X est NH₂ et Z est 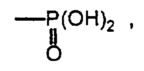 sont plus particulièrement préférés.

Les composés de formule (I) pour lesquels R² et/ou R³ représente un atome d'hydrogène constituent également un sous-groupe particulièrement préféré selon l'invention.

R² et R³ sont de préférence chacun un atome d'hydrogène.

Les composés de formule (I) avec R² et/ou R³ différent de l'hydrogène représente un autre sous-groupe selon l'invention.

Une sous-famille parmi les composés précités est formée par les composés pour lesquels n₁ et n₃ sont égaux à 0.

Une autre sous-famille est constituée par les composés pour lesquels n₁ et/ou n₃ sont différents de 0.

Une sous-classe de composés selon l'invention est encore constituée par ceux pour lesquels n₂ = 0. Parmi ces composés Y représente de préférence ―O―.

Une autre sous-classe est formée par les composés pour lesquels n₂ varie de 1 à 5, de préférence de 2 à 5 et de manière plus particulièrement préférée pour les composés où n₂ = 3.

Une autre classe de composés selon l'invention est définie par ceux où n₂ est supérieur à 5.

Du point de vue du symbole Y, les composés pour lesquels celui-ci représente un atome d'oxygène sont particulièrement préférés selon l'invention.

D'autres sous-familles peuvent être définies selon que Y représente ―CH₂―, un atome de soufre, un motif ―NH― ou ―OCH₂―.

R¹ est choisi de préférence parmi un groupe phényle non substitué ou substitué, plus préférentiellement monosubstitué par un des substituants précités.

Lorsque R¹ symbolise un groupe phényle substitué, le ou les substituants sont de préférence choisis parmi les atomes d'halogènes, les groupes CF₃, alkyles inférieur, alcoxy inférieur, NO₂, CN, NH₂, CO₂H, OPh, OCH₂Ph et NHCOR⁸.

Les composés pour lesquels R¹ est un groupe phényle mono ou polysubstitué par des atomes d'halogènes ou groupes alcoxy inférieur constituent une autre sous-famille- selon l'invention.

Lorsque R¹ représente un motif -A²-(CH₂)ₙ₄-A¹, A² est plus préférentiellement un groupe phényle, de préférence non-substitué.

Parmi ces composés, n₄ est de préférence égal à 0 ou 1 et/ou A¹ est de préférence un groupe phényle.

R¹ représentant un motif ―A²-(CH₂)ₙ₄-A¹ est de préférence un groupe phényle substitué par un groupe Ph ou CH₂Ph, plus préférentieilemsnt CH₂Ph.

Une autre sous-famille comprend les composés (I) pour lesquels R¹ représente un atome d'hydrogène ou un groupe alkyle inférieur.

Une autre sous-famille comprend les composés (I) pour lesquels R¹ est un groupe cycloalkyle.

Les composés (I) avec R¹ représentant un groupe α- ou β-naphtyle, ou un groupe anthracène forment encore une autre sous-famille.

Un autre groupe de composés selon l'invention comprend les composés (I) avec R¹ représentant un groupe 2-, 3 ou 4-pyridyle, 2- ou 3-thiényle et 2- ou 3-furyle.

Les composés (I) avec R¹ représentant 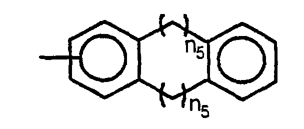 forment aussi un autre sous-groupe selon l'invention.

Pour toutes les sous-familles mentionnées ci-dessus, les substituants non précisés peuvent varier selon leurs définitions générales respectives.

Un groupe particulièrement préféré de composés selon l'invention est constitué des composés répondant à la formule (V) suivante : 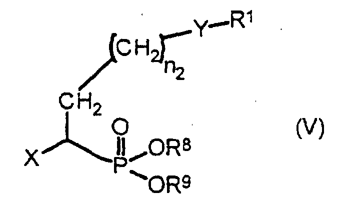 dans laquelle X, n₂, Y, R¹, R⁸ et R⁹ ont la signification précédente.

Les préférences indiquées précédemment pour les composés de formule (I) s'appliquent également à ceux de formule (V).

Un groupe encore plus particulièrement préféré comprend les composés répondant à la formule (VI) suivante : 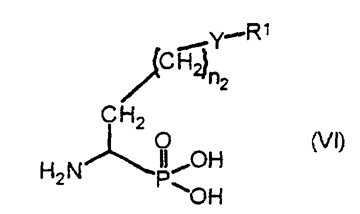 dans laquelle n₂, Y et R¹ ont la signification précédemment indiquée.

Les choix particuliers mentionnés pour les composés de formule (I) du point de vue des symboles Y et R¹ s'appliquent également aux composés de formules (VI).

Parmi ces composés, ceux répondant à la formule (VII) suivante : 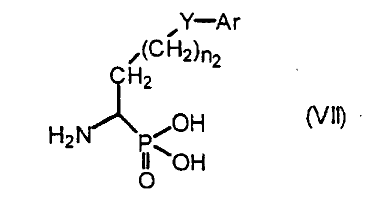 où Y et n₂ sont tels que définis précédemment, et Ar symbolise R¹ représentant un groupe phényle iv) éventuellement substitué tel que défini précédemment ou R¹ représentant un groupe vii) -A²-(CH₂)ₙ₄-A¹, A² étant un groupe phényle (b) éventuellement substitué tel que défini précédemment, sont particulièrement préférés.

Un autre groupe de composés selon le second aspect de la présente invention, comprend les composés répondant à la formule (VIII) suivante : 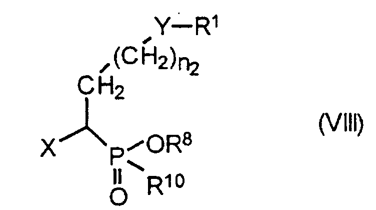 où X, Y, n₂, R¹, R⁸ et R¹⁰ ont la signification précédente.

Les préférences indiquées précédemment pour les composés de formule (I) s'appliquent également à ceux de formule (VIII).

Un troisième aspect de l'invention, concerne plus particulièrement les composés de formule (I) où Z représente le groupe

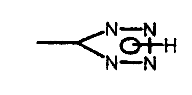

Un quatrième aspect de l'invention, est plus particulièrement relatif aux composés de formule (I) où Z représente un groupe -SO₃H, - SO₂NHR¹¹ ou -CONHSO₂R¹¹.

Parmi les composés de la présente invention sont particulièrement préférés les :
1) Chlorhydrate de la -(S)-O-4-benzyl phénoxy sérine 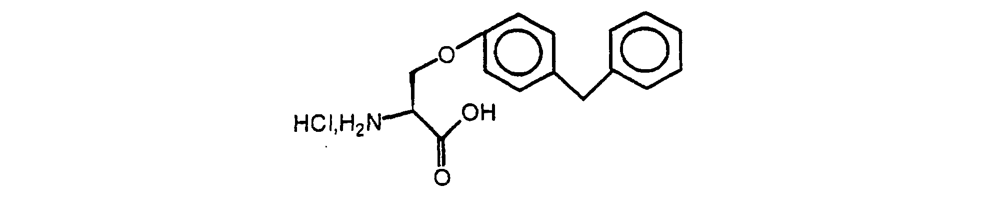
2) Bromhydrate de l'acide 2-(RS)-amino-6-(4 benzyl phénoxy) hexanoique 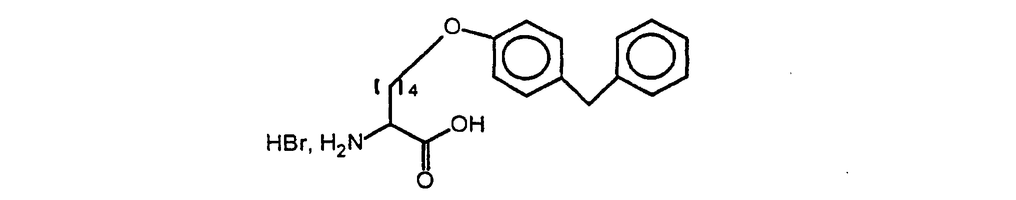
3) Bromhydrate de l'acide 2-(RS)-amino-5-(4 benzyl phénoxy) pentanoique 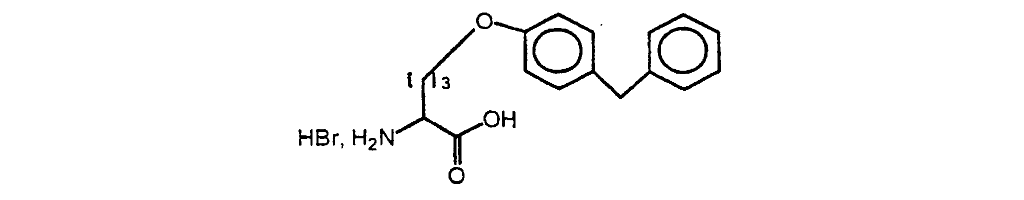
4) Bromhydrate de l'acide 2-(RS)-amino-5-(4-phénoxyphénoxy) pentanoique 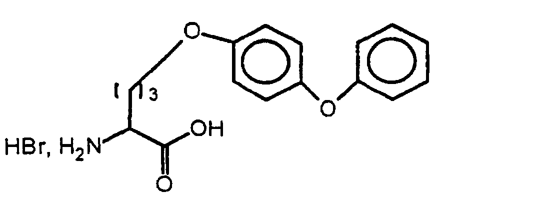
5) Bromhydrate de l'acide 2-(RS)-amino-7-(4-benzyl-phénoxy) heptanoique 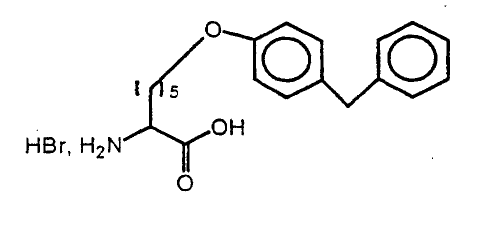
6) Bromhydrate de l'acide 2-(RS)-amino-6-(4-phénylphénoxy)-hexanoïque 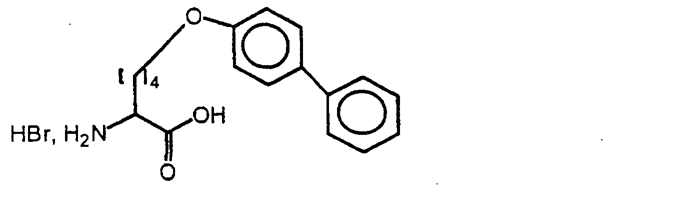
7) Bromhydrate de l'acide 2-(RS)-amino-6-(4-hexyloxyphénoxy)-hexanoïque 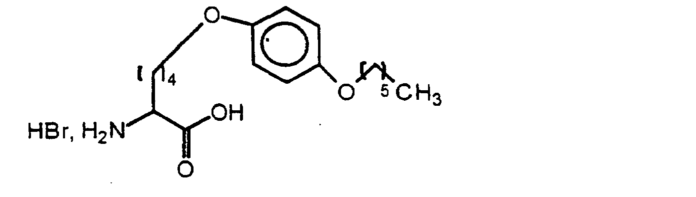
8) Bromhydrate de l'acide 2-(RS)-amino-8-(4-benzylphénoxy)-octanoïque 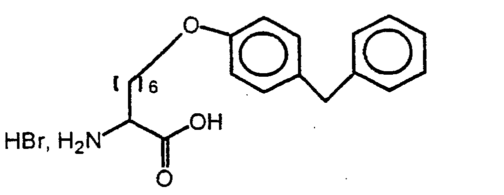
9) Bromhydrate de l'acide 2-(RS)-amino-6-(4-phénoxyphénoxy)-hexanoïque 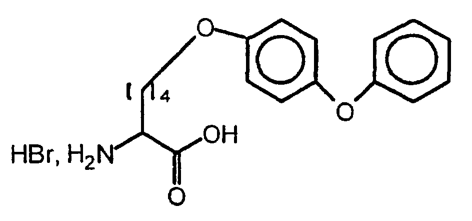
10) Acide 2-(RS)-aminométhyl-6-(4-benzyl-phénoxy)-hexanoïque 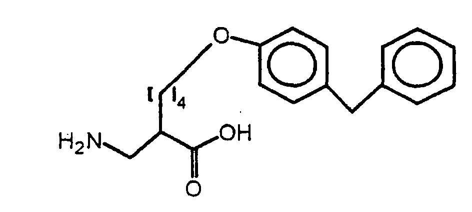
11) Bromhydrate de l'acide 1-(RS)-amino-5-(phénoxy)-pentylphosphonique 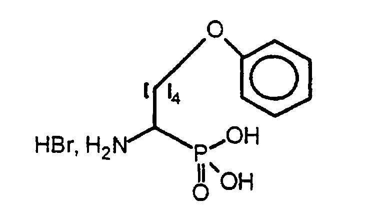
12) Bromhydrate de l'acide 1-(RS)-amino-6-(phénoxy)-hexylphosphonique 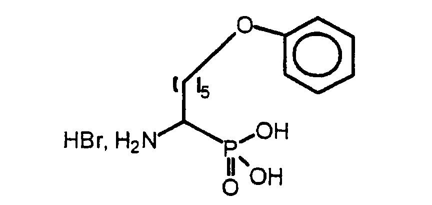
13) Bromhydrate de l'acide 1-(RS)-amino-5-(4-benzylphénoxy)-pentyl-phosphonique 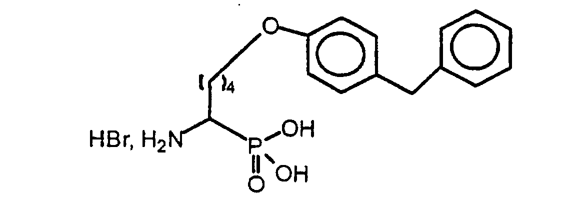
14) Bromhydrate de l'acide 1-(RS)-amino-4-(phénoxy)-butylphosphonique 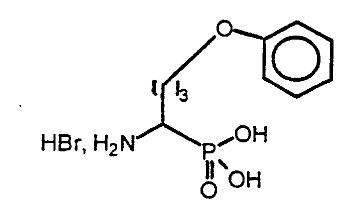
15) Bromhydrate de l'acide 1-(RS)-amino-7-(phénoxy)-heptylphosphonique 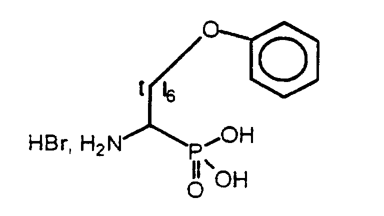
16) Bromhydrate de l'acide 2-(RS)-amino-6-(4 cyclohexylméthylphénoxy)-hexanoïque 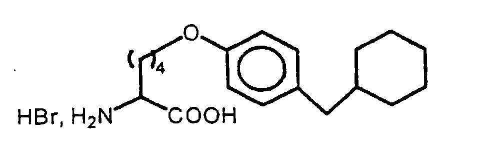
17) Acide 3-(RS)-amino-7-(4-benzyl-phénoxy)-heptanoïque 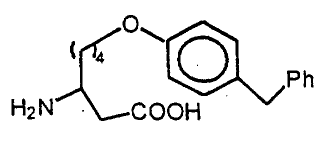
18) Bromhydrate de l'acide 2-(RS)-amino-2-méthyl-6(4-benzylphénoxy)hexanoïque 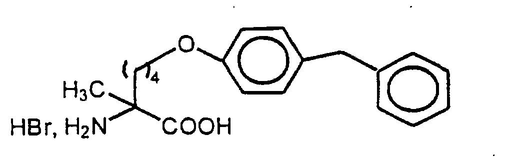
19) Bromhydrate de l'acide 1-(RS)-amino-tridécanyl phosphonique 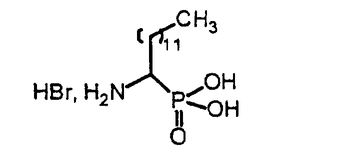
20) Acide 3-(RS)-amino-5-(4-benzyl-phénoxy)-pentanoïque 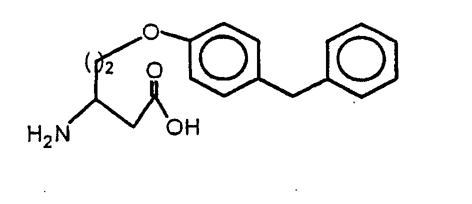
21) Acide 3-(RS)-amino-6-(4-benzyl-phénoxy)-hexanoïque 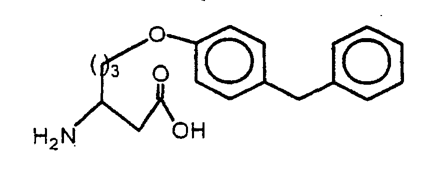
   Les composés de formule (I) ou (II) telle que précédemment définie avec X représentant NH₂ et R⁷ différent d'un atome d'hydrogène constituent des prodrogues.
   Les composés de formule (I) ou (II) telle que précédemment définie avec X représentant 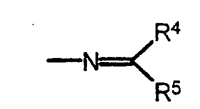 R⁷ étant un atome d'hydrogène constituent des prodrogues.
   Les composés de formule (I) ou (II) telles que précédemment définie avec X représentant 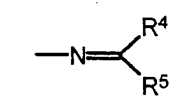 et R⁷ différent d'un atome d'hydrogène constituent des prodrogues.
   Les composés de formule (I) ou (V) telle que précédemment définie avec X représentant NH₂ et R⁸ et R⁹ différents d'un atome d'hydrogène constituent des prodrogues.
   Les composés de formule (I) ou (V) telle que précédemment définie où X est NH₂, R⁸ est l'hydrogène et R⁹ est différent d'un atome d'hydrogène constituent des prodrogues.
   Les composés de formule (I) ou (V) telle que précédemment définie où X est 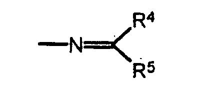 et R⁸ et R⁹ sont différents d'un atome d'hydrogène constituent des prodrogues.
   Les composés de formule (I) ou (V) telle que précédemment définie où X est 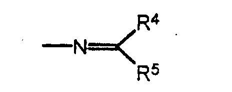 et R⁸ et R⁹ sont l'hydrogène constituent des prodrogues.
   Les composés de formule (I) ou (V) telle que précédemment définie où X est 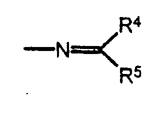 et R⁸ est l'hydrogène et R⁹ différent d'un atome d'hydrogène constituent des prodrogues.
   Les composés de formule (I) ou (VIII) telle que précédemment définie où X est NH₂ et R⁸ est différent d'un atome d'hydrogène constitue des prodrogues.
   Les composés de formule (I) ou (VIII) telle que précédemment définie où X est 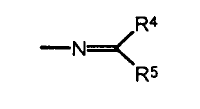 et R⁸ est différent d'un atome d'hydrogène constituent des prodrogues.
   Les composés de formule (I) ou (VIII) telle que précédemment définie où X est 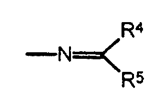 et R⁸ est l'hydrogène constituent des prodrogues.
   Des exemples de prodrogues selon l'invention sont les :
22) chlorhydrate du 2-(RS)-amino-7-(4 benzyl phénoxy) heptanoate d'éthyle 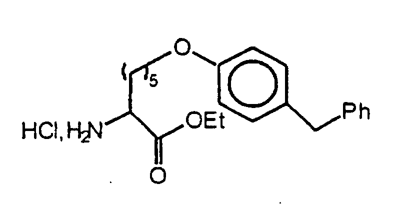
23) chlorhydrate du 2-(RS)-amino-6-(4-benzyl-phénoxy) hexanoate d'éthyle 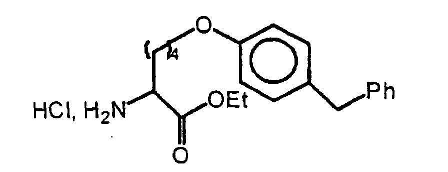
24) bromhydrate du diphényl 1-amino-5-phénoxy-pentylphosphonate 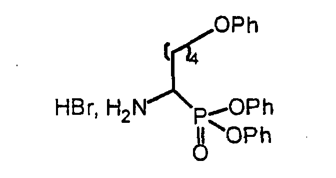
25) bromhydrate de l'éthyl-hydrogène-1-amino-5 phénoxy-pentylphosphonate

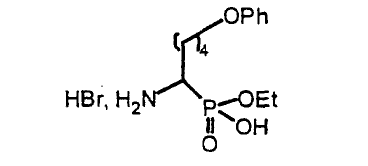

Les composés de la présente invention peuvent être préparés à partir de matières premières facilement disponibles, selon l'un des procédés indiqués ci-après.

Les schémas réactionnels donnés ci-après décrivent des procédés qui peuvent être employés pour la préparation des composés de formule (I), en indiquant les produits de départ, les intermédiaires ainsi que les conditions de synthèse.

Les abréviations utilisées dans la présente description correspondent aux définitions ci-dessous :

| | |
|---|---|
| Ac | acétyle |
| Bn | benzyle |
| DIAD | azodicarboxylate de diisopropyle |
| DMF | diméthylformamide |
| DPPA | diphénylphosphorylazide |
| Et | éthyle |
| EtOH | alcool éthylique |
| Et₂O | éther éthylique |
| Me | méthyle |
| NBu₄F | fluorure de tétrabutylammonium |
| Pd/C | palladium sur carbone |
| Ph | phényle |
| THF | tétrahydrofuranne |
| PCC | chlorochromate de pyridinium |

Les schémas 1 à 5 décrivent la préparation d'amino-acides substitués.

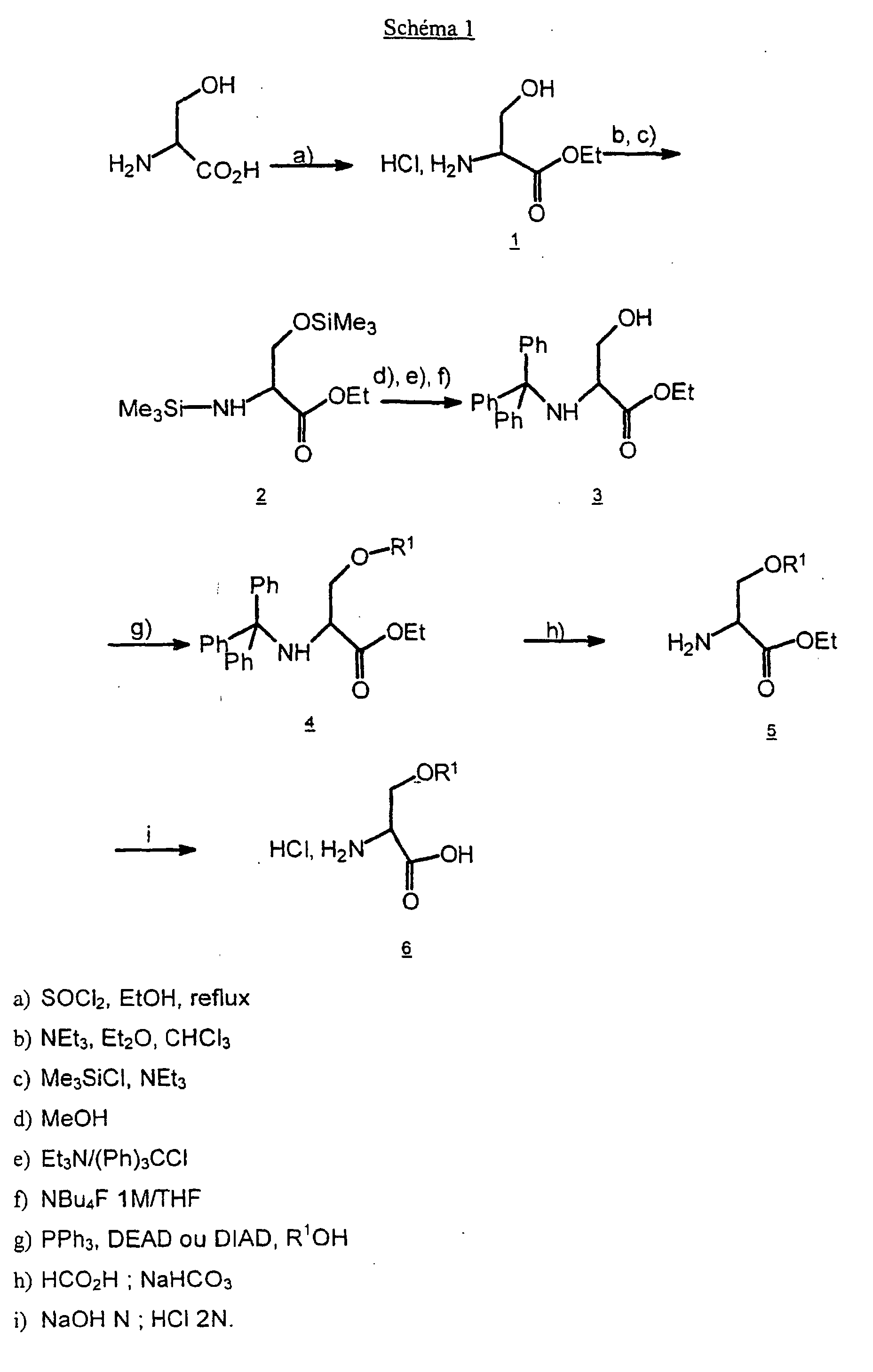

La sérine est estérifiée en présence de chlorure de thionyle et d'EtOH. Le chlorhydrate d'aminoester 1 obtenu est traité par la triéthylamine puis par le chlorure de triméthylsilyle en présence de NEt₃ pour conduire au composé 2. La fonction amino est déprotégée à l'aide de MeOH anhydre puis reprotégée par réaction avec le chlorure de trityle. La fonction hydroxy est ensuite libérée à l'aide du fluorure de tétrabutylammonium pour conduire au composé 3. La fonction hydroxy du composé 3 est substituée selon une réaction de type Mitsunobu par un dérivé phénolique de formule R¹OH pour conduire au composé 4. Les composés 5 sont obtenus par déprotection par l'acide formique suivie d'un traitement à l'aide d'hydrogénocarbonate de sodium. Le chlorhydrate d'amino-acide 6 est obtenu par saponification dans NaOH du composé 5 suivie d'une acidification dans HCl 2N.

Les dérivés amino-acides 13 et 15 sont préparés à partir des malonates 10 obtenus soit à partir des halogénures commerciaux, soit à partir des halogénures 9.

Le schéma 2 décrit la préparation des halogénures non commerciaux 9. 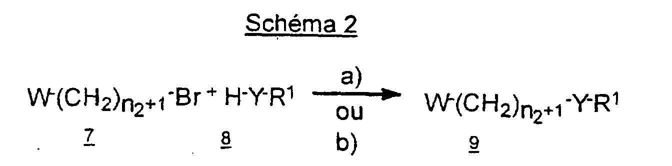
W = Cl, Br
Y = O, S
n₂ et R¹ sont tels que définis dans la formule (I)
a) NaOH 9N, THF reflux
b) K₂CO₃, DMF, température ambiante

Les composés 9 peuvent être obtenus selon deux voies : par traitement dans la soude 9N au reflux en présence de THF ou par l'utilisation de K₂CO₃ en poudre dans le DMF à température ambiante.

Le schéma 3 décrit la synthèse des sels d'aminoesters 13 et d'amino-acides 15.

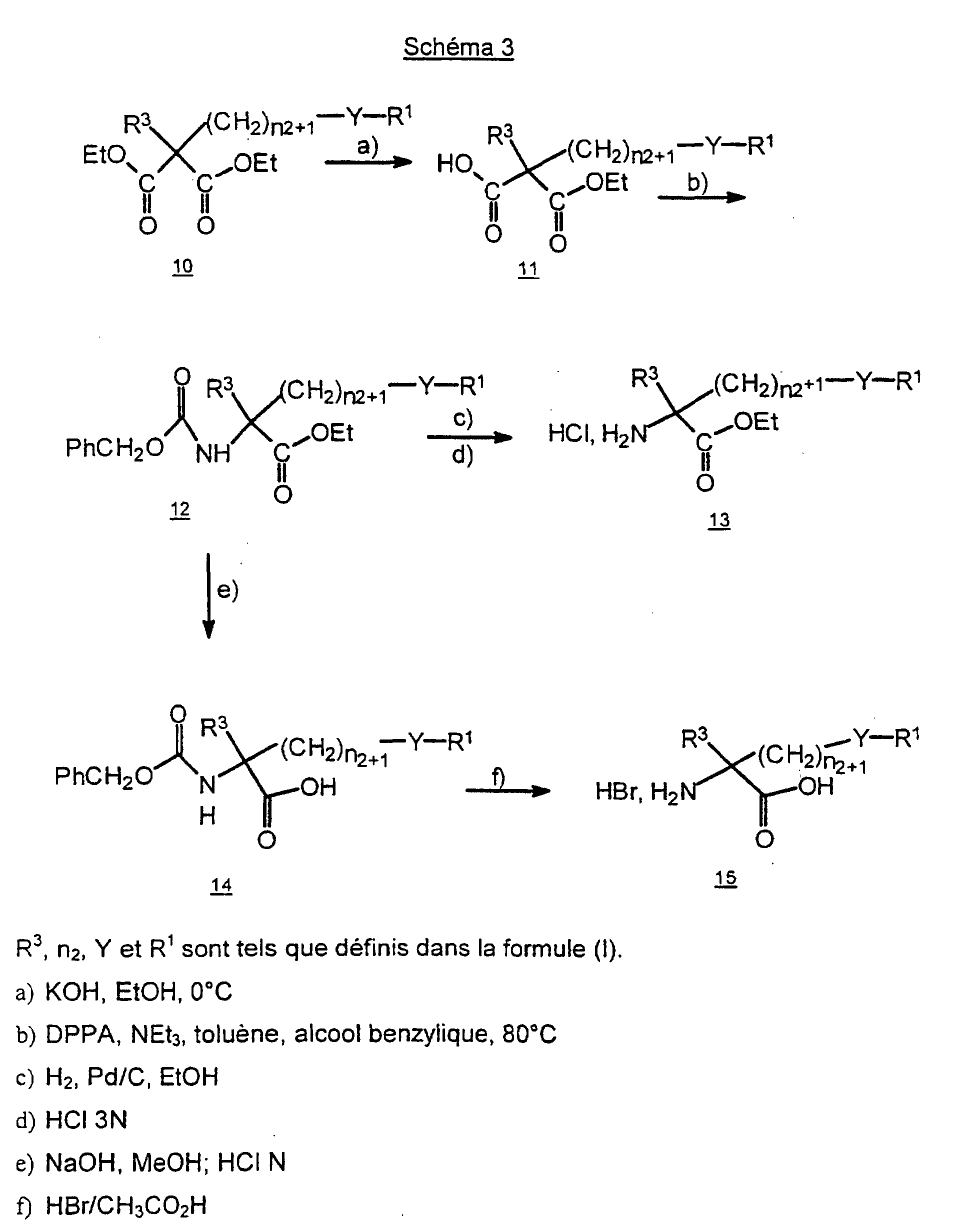

Les malonates de formule 10 sont obtenus par alkylation d'un malonate avec les dérivés bromés ou chlorés 9 correspondant en présence d'éthylate de sodium dans l'éthanol au reflux. Une monosaponification à l'aide d'une solution de KOH dans l'EtOH conduit aux composés 11 à qui l'on fait subir une réaction de Curtius en présence de DPPA, de NEt₃ et d'alcool benzylique dans le toluène à 80°C pendant une nuit.

La fonction benzyloxycarbonyle est déprotégée par hydrogénation catalytique dans l'éthanol à l'aide de Pd/C pour conduire aux aminoesters 13. Une saponification des composés 12 à l'aide d'une solution de NaOH dans le MeOH conduit aux dérivés 14 que l'on soumet à l'action du HBr dans l'acide acétique pour conduire aux amino-acides 15.

Les dérivés d'aminoacides 18 et 19 sont préparés à partir des malonates 10 (avec R³ = H) décrits dans le schéma 3.

Le schéma 4 décrit la synthèse des sels d'aminoesters 19 et des amino-acides 18.

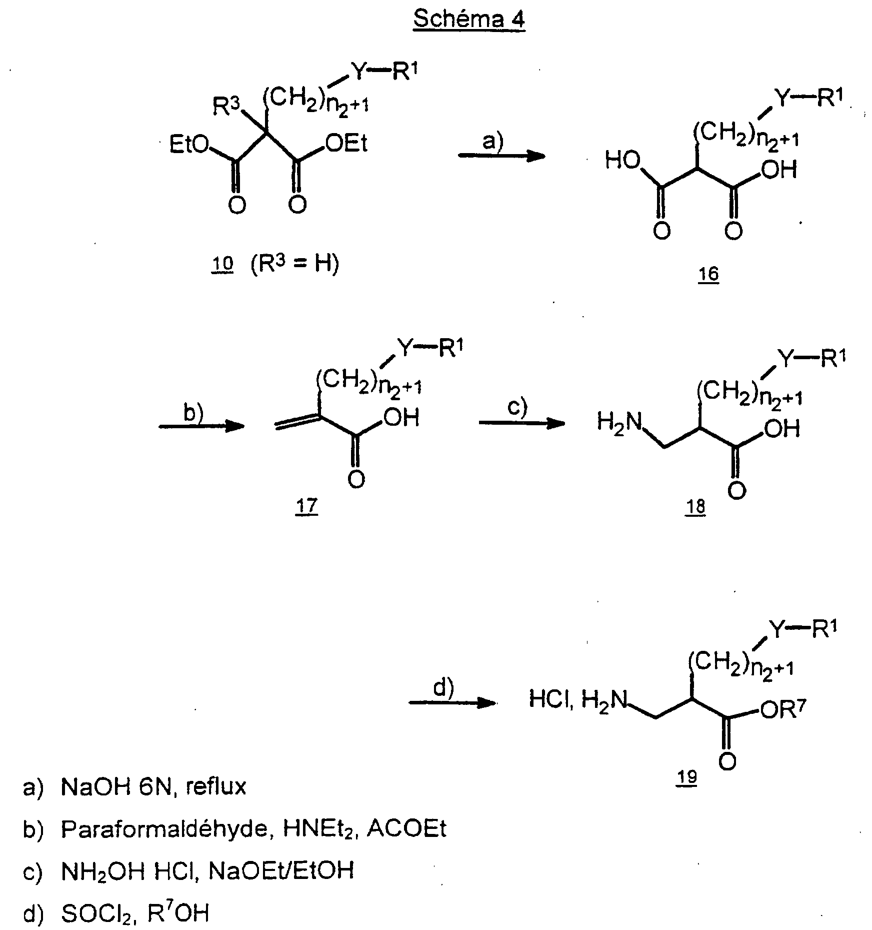

Les acides acryliques 17 sont préparés via les diacides 16 obtenus par saponification dans la soude 6N au reflux, puis une réaction de Mannich en présence de paraformaldéhyde, de diéthylamine dans l'acétate d'éthyle au reflux.

Les dérivés 18 sont obtenus par addition de l'hydroxylamine en présence d'éthylate de sodium sur les acides acryliques 17 au reflux. Les sels d'aminoesters 19 sont préparés à partir des dérivés 18 par réaction en présence de chlorure de thionyle dans un alcool R⁷OH.

Le schéma 5 montre la préparation de β-aminoacides 24 et de sels d'aminoacides 25 à partir de malonates.

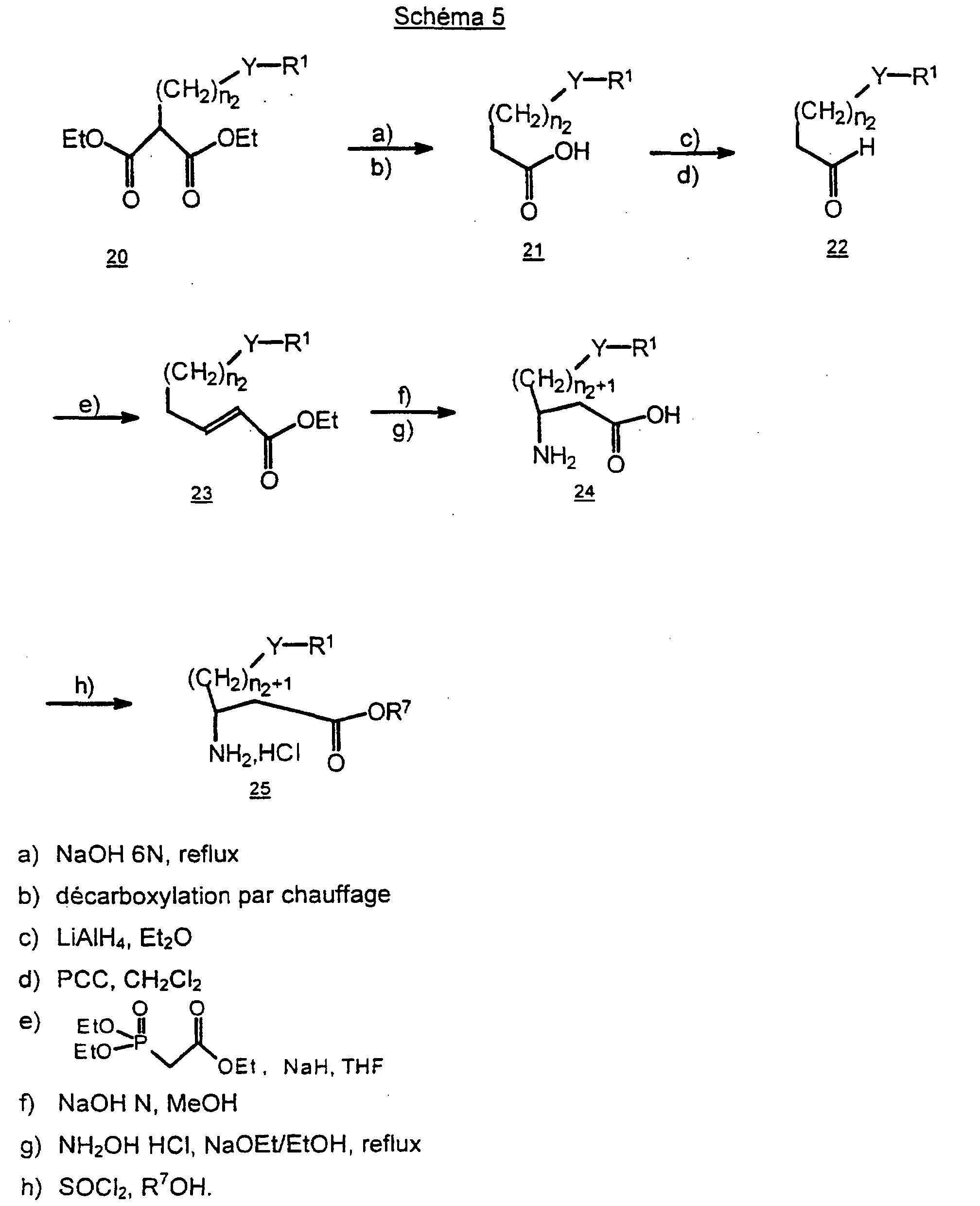

Les malonates de formule 20 sont obtenus par alkylation d'un malonate avec les dérivés bromés ou chlorés correspondants en présence d'éthylate de sodium dans l'éthanol au reflux. Les acides 21 sont préparés via les diacides obtenus par saponification dans la soude 6N au reflux, puis une décarboxylation thermique. Après une réduction à l'aide de l'aluminohydrure de lithium suivie d'une oxydation avec le chlorochromate de pyridinium (PCC), on obtient l'aldéhyde 22. Les aminoacides 24 sont préparés via une réaction de Wittig Horner à l'aide du triéthylphosphonoacétate suivie d'une saponification en présence de soude normale puis de l'addition d'hydroxylamine en présence d'éthylate de sodium sur les dérivés acryliques.

Les sels d'aminoesters 25 sont préparés à partir des dérivés 24 par réaction en présence de chlorure de thionyle dans un alcool R⁷OH.

Les schémas 6 et 7 décrivent la préparation de dérivés amino-phosphoniques 28.

Les dérivés amino-phosphoniques 28 sont obtenus selon deux voies :
- Voie A (schéma 6) 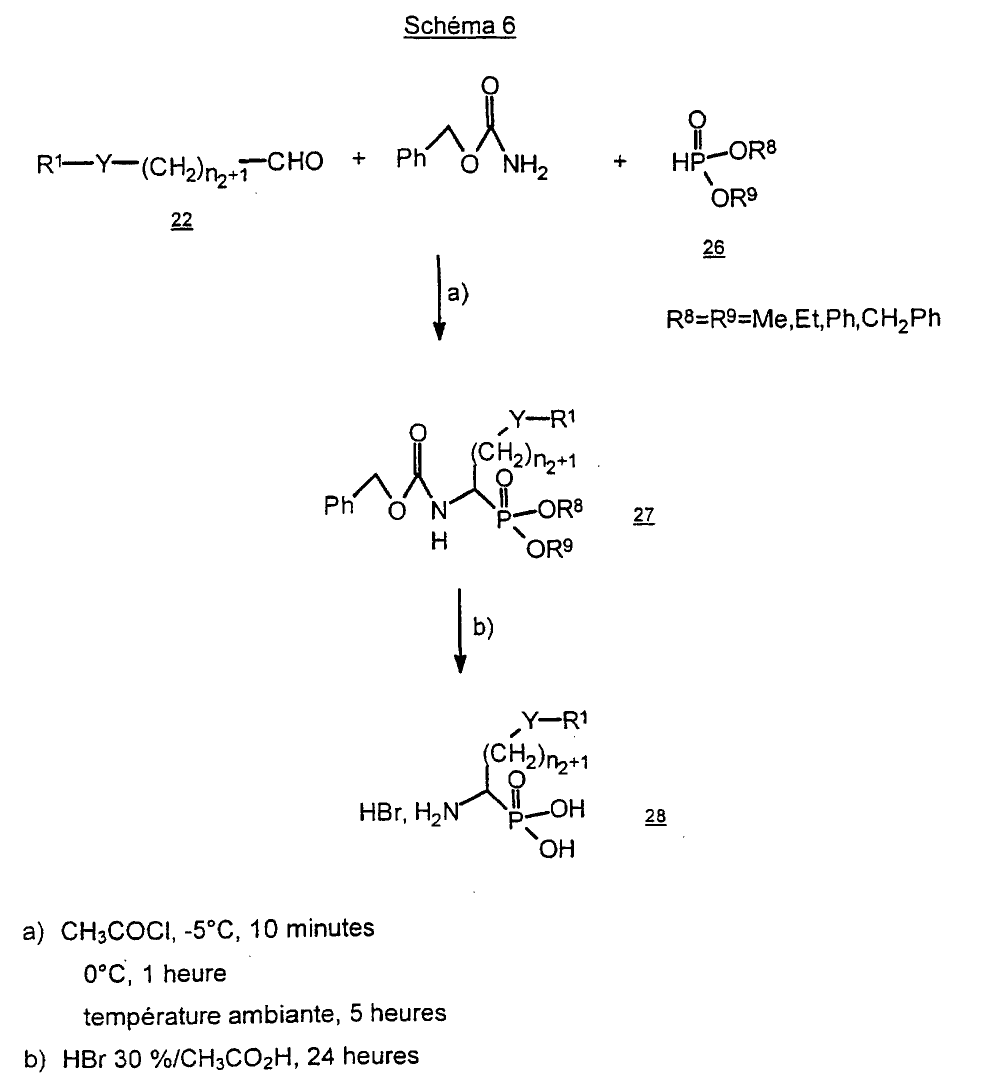
   La phosphite 26 réagit avec le carbamate de benzyle et les aldéhydes 22 pour conduire aux phosphonates 27. Une déprotection à l'aide d'HBr 30 % dans l'acide acétique permet d'accéder aux dérivés 28.
- Voie B (schéma 7) :

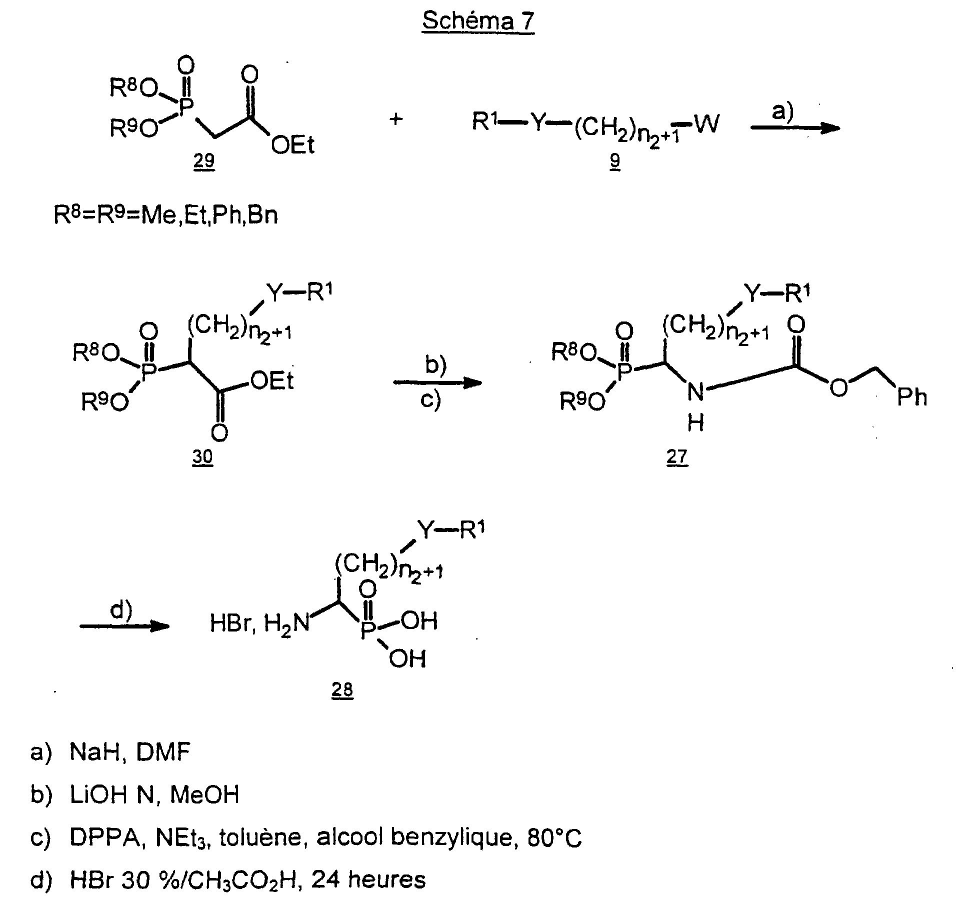

Les phosphonoacétates 29 sont alkylés par les dérivés halogénés 9 à l'aide du NaH dans le DMF. Après saponification et réaction de Curtius, on obtient les phosphonates 27. Une déprotection à l'aide d'HBr 30 % dans l'acide acétique permet d'accéder aux dérivés 28.

Le schéma 8 décrit la préparation de dérivés amino-phosphoniques 31 et 33.

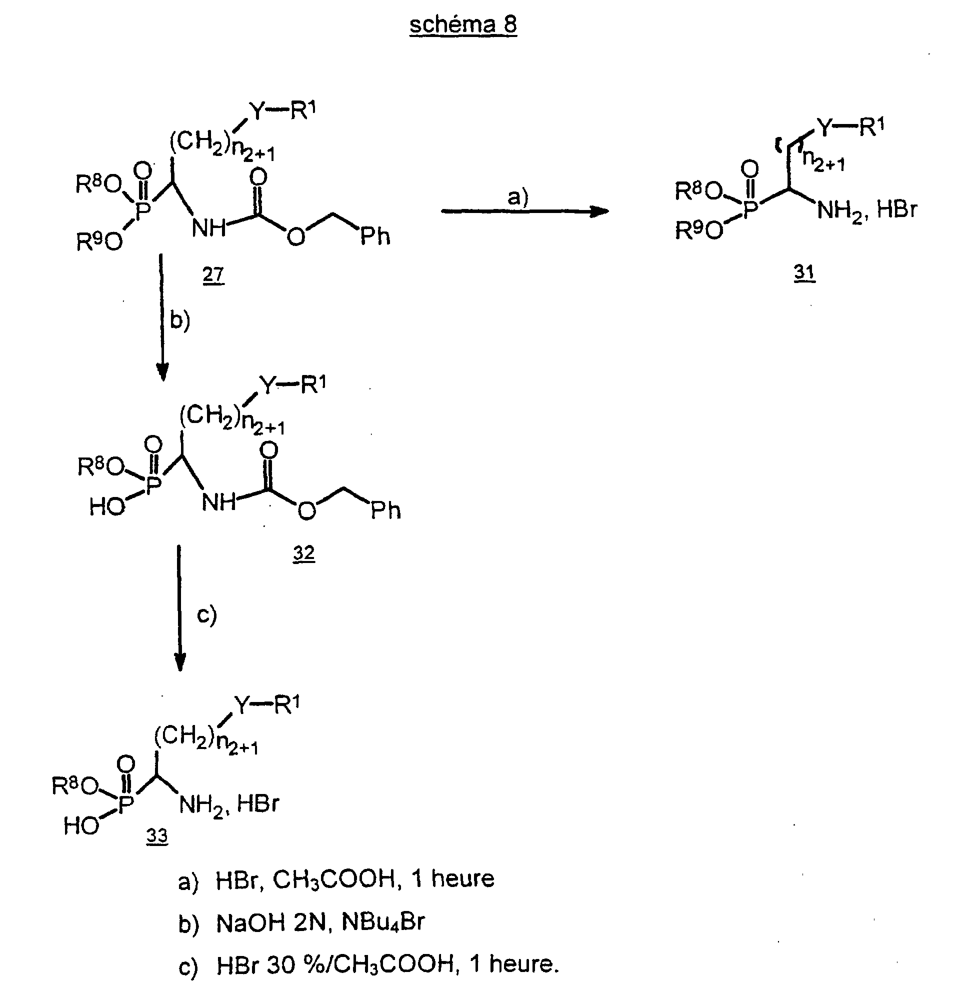

Le composé 27 est soumis à l'action d'une solution d'HBr à 30 % dans l'acide acétique pour conduire au produit 31.

Le composé 33 est obtenu en deux étapes à partir des dérivés 27 : une monosaponification en présence d'un agent de transfert de phase dans NaOH 2N puis déprotection par HBr 30 % dans l'acide acétique.

Les inventeurs ont montré que les composés (I) selon l'invention et notamment les composés répondant plus particulièrement à l'une des formules (II) à (VIII), présentent des propriétés inhibitrices de la LTA₄ hydrolase.

Ces propriétés s'appliquent aussi aux composés de formule (I) où Z est un groupement de type COOR⁷, n₁=n₃=O, R²=H, R¹ est un groupe iv) de type phényl non substitué ou mono ou polysubstitué et n₂=1, à l'acide α-amino-β-phénoxy-propionique, à l'acide 3-amino-7-phényl-heptanoïque à l'acide 3-amino-6-phénoxy-hexanoïque, à l'acide α-amino-6-phényl-hexanoïque, ou à l'acide α-amino-5-phénoxy-pentanoïque.

Ils possèdent une 'intéressante activité thérapeutique en particulier dans le domaine des traitements anti-inflammatoires.

Ils possèdent également une intéressante activité anti-arthritique.

Les composés de l'invention et ceux mentionnés ci-dessus présentent aussi des propriétés anti-psoriasiques.

Par ailleurs, les inventeurs ont montré que ces composés de l'invention préviennent l'augmentation des taux tissulaires de LTB₄ induite par les inhibiteurs de cyclooxygénase.

Ils sont ainsi utiles pour prévenir certains effets secondaires pro-inflammatoires paradoxaux des inhibiteurs de cyclooxygénase.

Enfin, le LTB₄ étant le ligand endogène du récepteur induisant une prolifération des peroxisomes, les composés de l'invention trouvent aussi des applications dans les domaines de l'hépatoprotection et de l'action antimitotique.

La présente invention a ainsi également pour objet l'utilisation des composés de formule (I) et notamment les composés répondant plus particulièrement à l'une des formules (II) à (VIII), et d'un composé de formule (I) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-proplonique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, en tant que médicaments agissant comme inhibiteurs de l'activité de la LTA₄ hydrolase, en particulier pour un traitement anti-inflammatoire, ou anti-arthritique.

Elle a aussi pour objet l'utilisation de ces composés en tant que médicaments anti-psoriasiques.

Elle a encore pour objet leur utilisation en tant que médicaments hépato-protecteurs ou antimitotiques.

Elle a encore pour objet l'utilisation de tels composés en tant que médicaments destinés au traitement d'une surproduction de LTB₄, induite notamment par des inhibiteurs de cyclooxygénase.

Elle a encore pour objet l'utilisation de tels composés et notamment les composés répondant plus particulièrement à l'une des formules (II) à (VIII), pour la préparation de médicaments destinés à inhiber l'activité de la LTA₄ hydrolase.

Elle a en particulier pour objet leur utilisation pour la préparation de médicaments destinés aux traitements pré-cités.

Ces composés et notamment les composés répondant plus particulièrement à l'une des formules (II) à (VIII), peuvent être administrés dans un véhicule ou excipient physiologiquement acceptable.

Aussi, la présente invention a encore pour objet des compositions pharmaceutiques comprenant une quantité thérapeutiquement efficace d'au moins un composé de formule (I) en combinaison avec un véhicule ou excipient physiologiquement acceptable.

Les composés (I) et notamment les composés répondant plus particulièrement à l'une des formules (II) à (VIII), de l'invention peuvent aussi être utilisés en combinaison avec des inhibiteurs de cyclooxygénase.

L'invention concerne ainsi des médicaments ou compositions pharmaceutiques contenant une quantité thérapeutiquement efficace d'un composé (I) et notamment les composés répondant plus particulièrement à l'une des formules (II) à (VIII), et une quantité thérapeutiquement efficace d'un composé inhibiteur de cyclooxygénase, éventuellement en combinaison avec un véhicule ou excipient physiologiquement acceptable.

Des exemples d'inhibiteurs de cyclooxygénase utiles selon l'invention sont l'aspirine (acide acétylsalicylique), l'ibuprofèrie, le dichlofénac.

Les médicaments ou compositions pharmaceutiques selon l'invention peuvent avantageusement être administrés par voies locales cutanées ou occulaires, par voie parentérale ou par voie orale, cette dernière étant préférée.

L'invention a également pour objet un procédé de traitement pour inhiber l'activité de la LTA₄ hydrolase chez l'homme.

Elle a encore pour objet un tel procédé pour les traitements indiqués précédemment.

Elle a aussi pour objet un procédé de traitement d'une surproduction de LTB₄, notamment induite par des inhibiteurs de cyclooxygénase.

D'autres avantages et caractéristiques de la présente invention apparaîtront à la lecture des exemples de préparation de composés de formule (1) donnés à titre illustratif et non limitatif, ainsi que des résultats biologiques donnés ci-après.

### EXEMPLES

Un tableau récapitulatif des exemples de composés de formule (I) est donné ci-dessous :

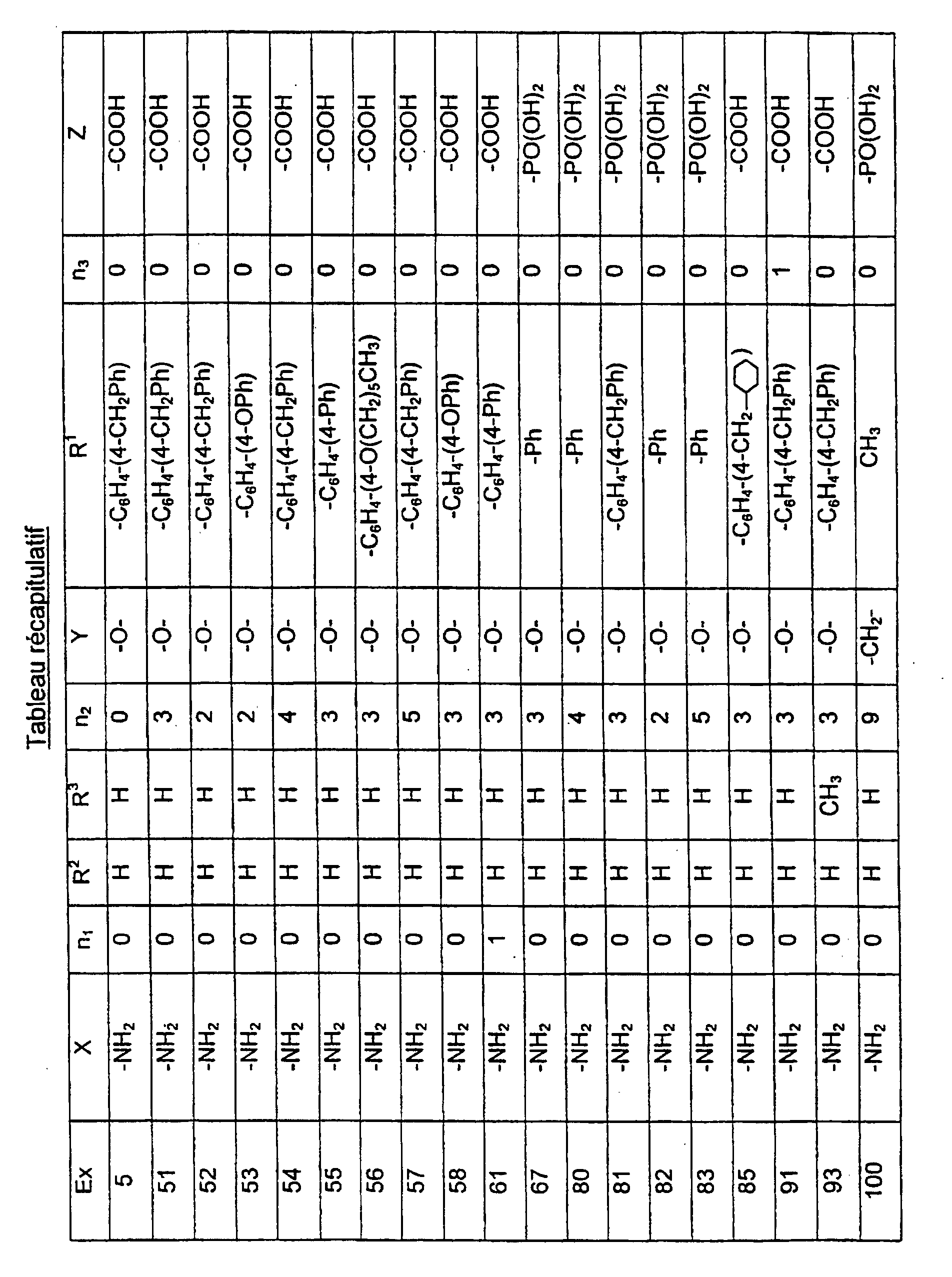

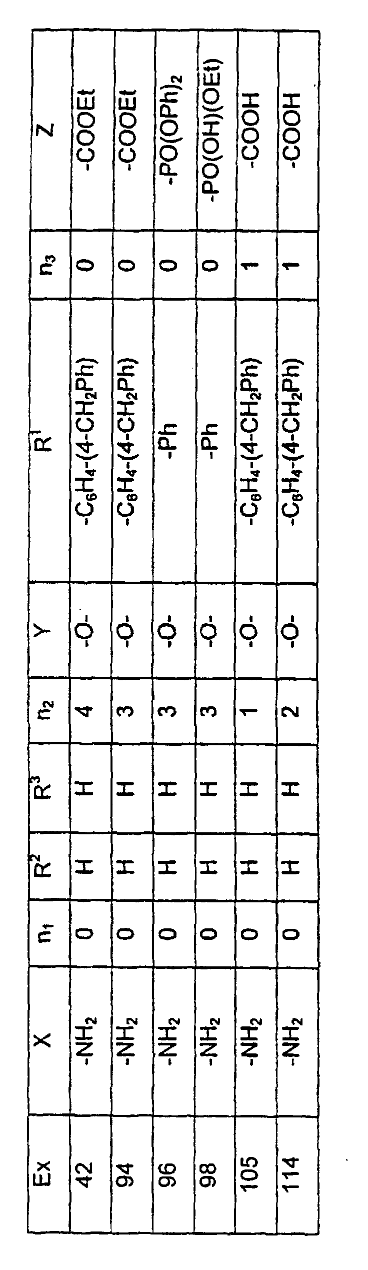

### Exemple 1

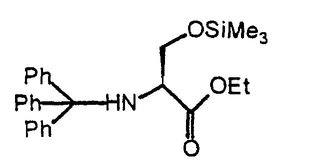

11,98 g (90 mmol) de sérinate d'éthyle sous forme chlorhydrate sont mis en solution dans 155 ml de CH₂Cl₂. On ajoute sous atmosphère inerte 22,78 g (209,68 mmol) de chlorure de triméthylsilyle.

On porte au reflux pendant 20 minutes puis on revient à température ambiante. On ajoute alors 21,2 g (209,90 mmol) de triéthylamine dans 60 ml de CH₂Cl₂ puis on porte au reflux pendant 45 minutes.

On refroidit ensuite à 0°C et on ajoute une solution de 5,4 ml (135 mmol) de méthanol anhydre dans 22 ml de CH₂Cl₂.

On laisse remonter le milieu à température ambiante puis on ajoute successivement 9,1 g (90 mmol) de NEt₃ et 25 g (90 mmol) de chlorure de trityle et on agite pendant une nuit à température ambiante.

On concentre sous vide, reprend avec 200 ml d'Et₂O. On lave à l'eau (une fois 30 ml).

On sèche sur MgSO₄, filtre et concentre sous vide.

On obtient 38,8 g du composé désiré.

### Exemple 2

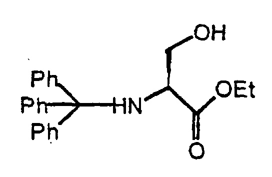

A une solution de 38,8 g de produit de l'exemple 1 dans 53 ml de THF, on ajoute à température ambiante 50 ml d'une solution molaire de fluorure de tétrabutylammonium (NBu₄F) dans le THF. On agite pendant 10 minutes à température ambiante.

On ajoute ensuite 500 ml d'Et₂O et on lave la phase organique successivement avec une solution aqueuse saturée d'hydrogénocarbonate de sodium (2 fois 60 ml) puis une solution aqueuse saturée de thiosulfate de sodium (2 fois 60 ml). La phase organique est séchée sur MgSO₄, filtrée et concentrée sous vide. Le résidu huileux obtenu est purifié par chromatographie éclair en utilisant le mélange éther de pétrole/Et₂O (1/1) puis Et₂O comme éluants.

On obtient 29,31 g (78 mmol) de composé désiré.

### Exemple 3

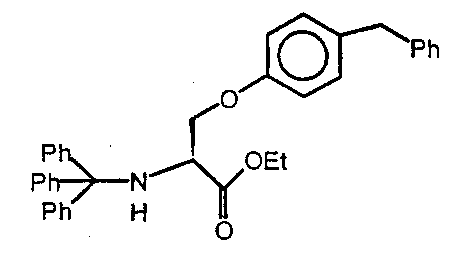

A une solution de 6,5 g (17,2 mmol) d'aminoester de l'exemple 2 dans 200 ml de toluène, on ajoute successivement 4,8 g (1,07 équivalents) de triphénylphosphine et 4,65 g (1,46 équivalent) de 4-phénylphénol. Le milieu réactionnel est agité vivement durant 5 minutes, puis on ajoute 3,70 g (1,07 équivalent) d'azodicarboxylate de diisopropyle.

On agite une nuit à température ambiante, filtre le milieu réactionnel et évapore à sec. Le résidu huileux est purifié par chromatographie éclair en utilisant le mélange éther-éther de pétrole (5/95) comme éluant. On obtient ainsi 6,6 g (12,15 mmol) du composé désiré.

### Exemple 4

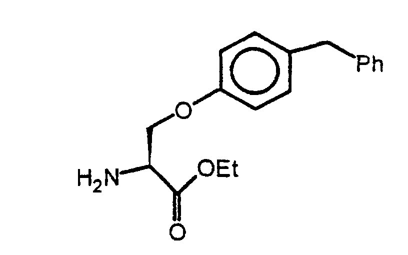

6,6 g (12,15 mmol) d'aminoester de l'exemple 3 sont agités vivement durant 5 heures à température ambiante en présence de 85 ml d'acide formique. Le milieu réactionnel est ensuite évaporé à sec, et l'on obtient un solide blanc que l'on reprend avec 100 ml d'eau. La phase aqueuse est lavée avec Et₂O (3 fois 20 ml) puis est basifiée à l'aide d'hydrogénocarbonate de sodium. La phase aqueuse basique est ensuite extraite avec de l'acétate d'éthyle (3 fois 20 ml). La phase organique est séchée sur MgSO₄, filtrée et concentrée sous vide. On obtient 1,86 g (6,6 mmol) du composé désiré.

### Exemple 5

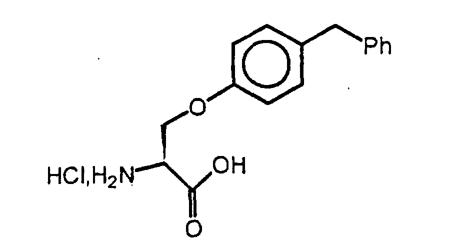

On mélange 1,86 (6,2 mmol) de produit de l'exemple 4 avec 6,5 ml de NaOH N et on agite pendant une nuit à température ambiante.

La phase aqueuse est lavée avec de l'éther-éthylique (1 fois 10 ml) puis concentrée sous vide. On ajoute ensuite 15 ml HCl N. Le solide blanc obtenu est filtré, lavé à l'eau et séché sous vide sur P₂O₅.

On obtient 1,26 g (3,75 mmol) de chlorhydrate d'amino-acide. (Fusion = 225°C).

La RMN¹H est en accord avec la structure chimique.

### Exemple 6 (méthode a)

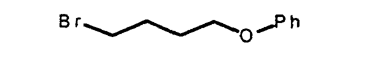

Dans un ballon, on introduit 17,41 g (185,25 mmol) de phénol, 14,5 ml de THF et 62 ml de NaOH 9N.

On ajoute goutte à goutte, 40 g (185,25 mmol) de 1,4-dibromobutane.

On agite et porte au reflux pendant 45 minutes.

Après refroidissement, on dilue avec 50 ml d'eau puis on extrait avec 100 ml d'Et₂O. La phase organique est lavée avec 30 ml d'eau, séchée sur MgSO₄, filtrée et concentrée. Le résidu huileux est distillé à la pompe à palette. On récupère la fraction distillant à 80-105°C sous 1 mm de Hg.

On obtient 15,96 g (37 %) d'huile incolore.

### Exemple 7 (méthode b)

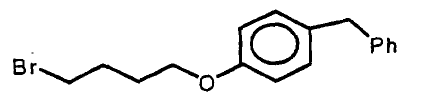

Dans un erlenmeyer, on introduit successivement 11 g (60 mmol) de 4-hydroxydiphénylméthane, 64,8 g (300 mmol) de 1,4-dibromobutane, 41,5 g (300 mmol) de K₂CO₃ en poudre et 94 ml de DMF anhydre.

On agite pendant une nuit à température ambiante. On filtre et reprend le filtrat avec 300 ml d'acétate d'éthyle. La phase organique est lavée avec une solution aqueuse saturée de NaCl (3 fois 100 ml), séchée sur MgSO₄, filtrée et concentrée.

On distille le 1,4-dibromobutane en excès, sous vide. On obtient 18 g (56,5 mmol) de résidu huileux correspondant au produit désiré.

Les exemples 8 à 17 sont préparés selon l'une des méthodes (a ou b) précédemment décrites.

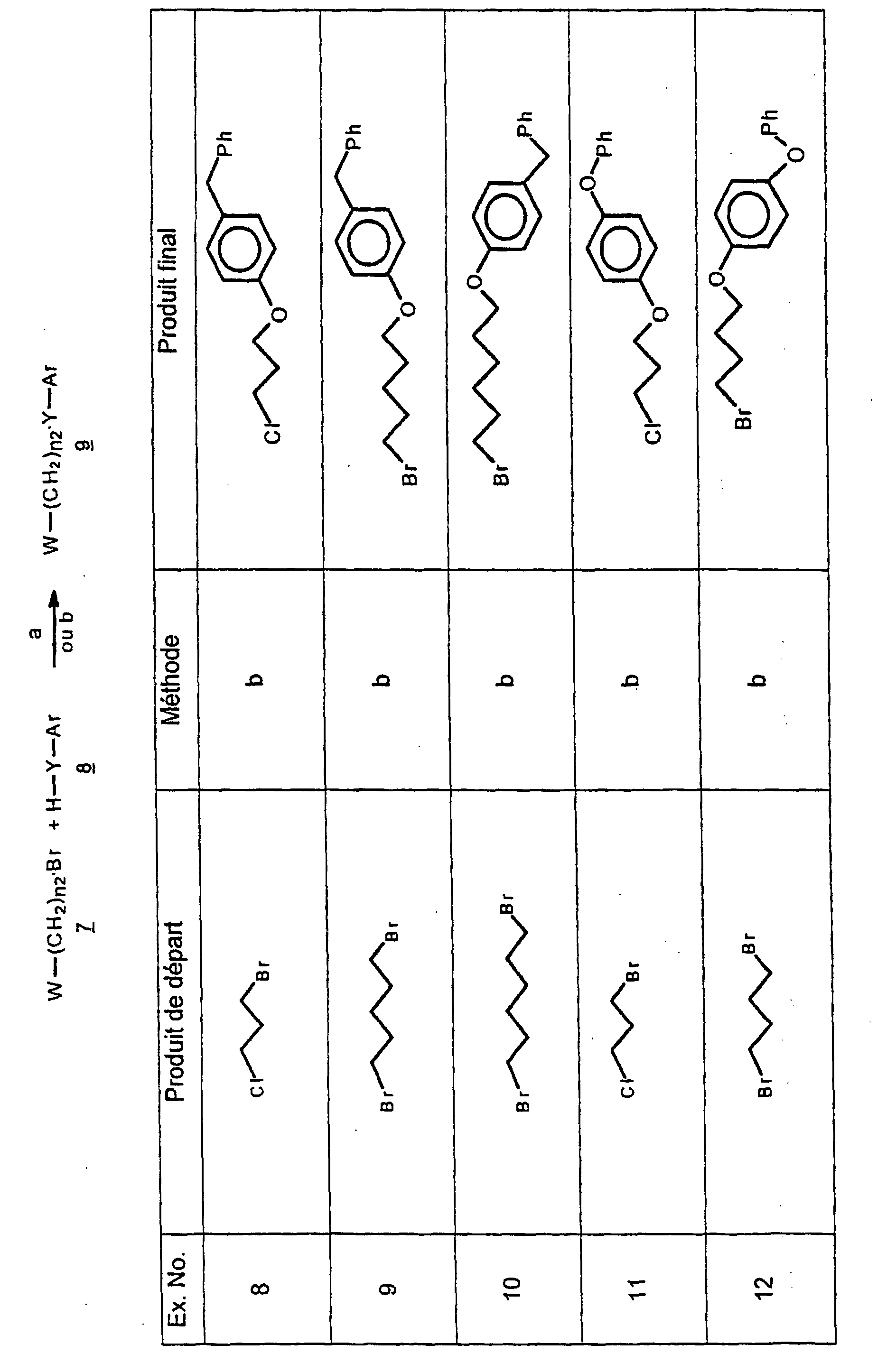

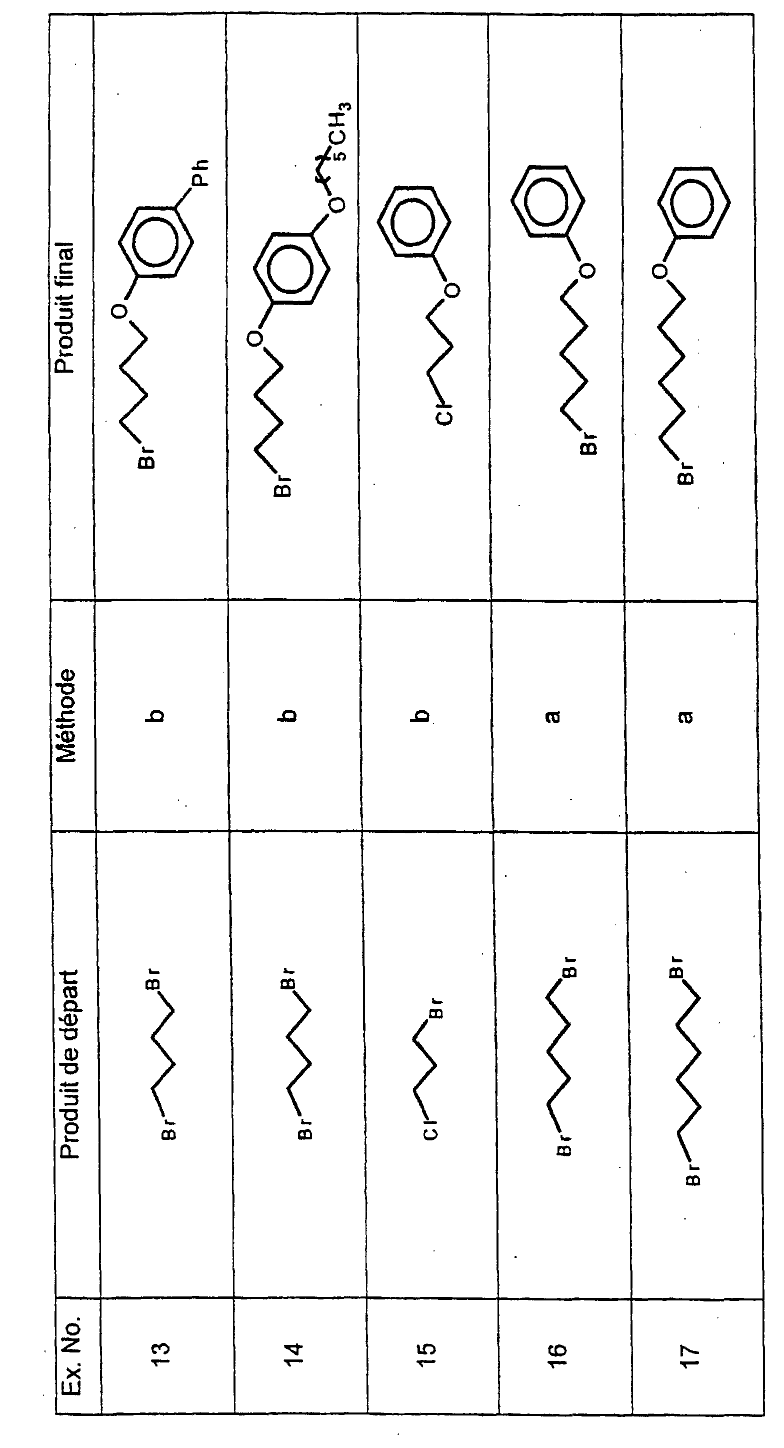

### Exemple 18

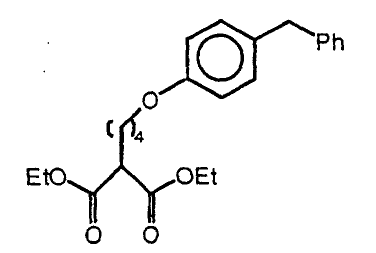

Une solution d'éthylate de sodium préparée à partir de 1,27 g (55,21 mmol) de sodium dans 32 ml d'EtOH est ajoutée à un mélange de 14,3 g (89,37 mmol) de malonate de diéthyle et de 6,78 g (21,27 mmol) du dérivé bromé de l'exemple 7. On porte au reflux pendant 4 heures.

On concentre sous vide, reprend le résidu avec de l'eau et extrait avec de l'Et₂O.

La phase éthérée est lavée 3 fois avec de l'eau, séchée sur MgSO₄, filtrée puis concentrée. L'excès de diéthylmalonate est éliminé par distillation sous vide.

On obtient 6,4 g (Rendement 76 %) d'huile jaune.

Les exemples 19 à 25 sont préparés selon le même mode opératoire que celui décrit pour l'exemple 18.

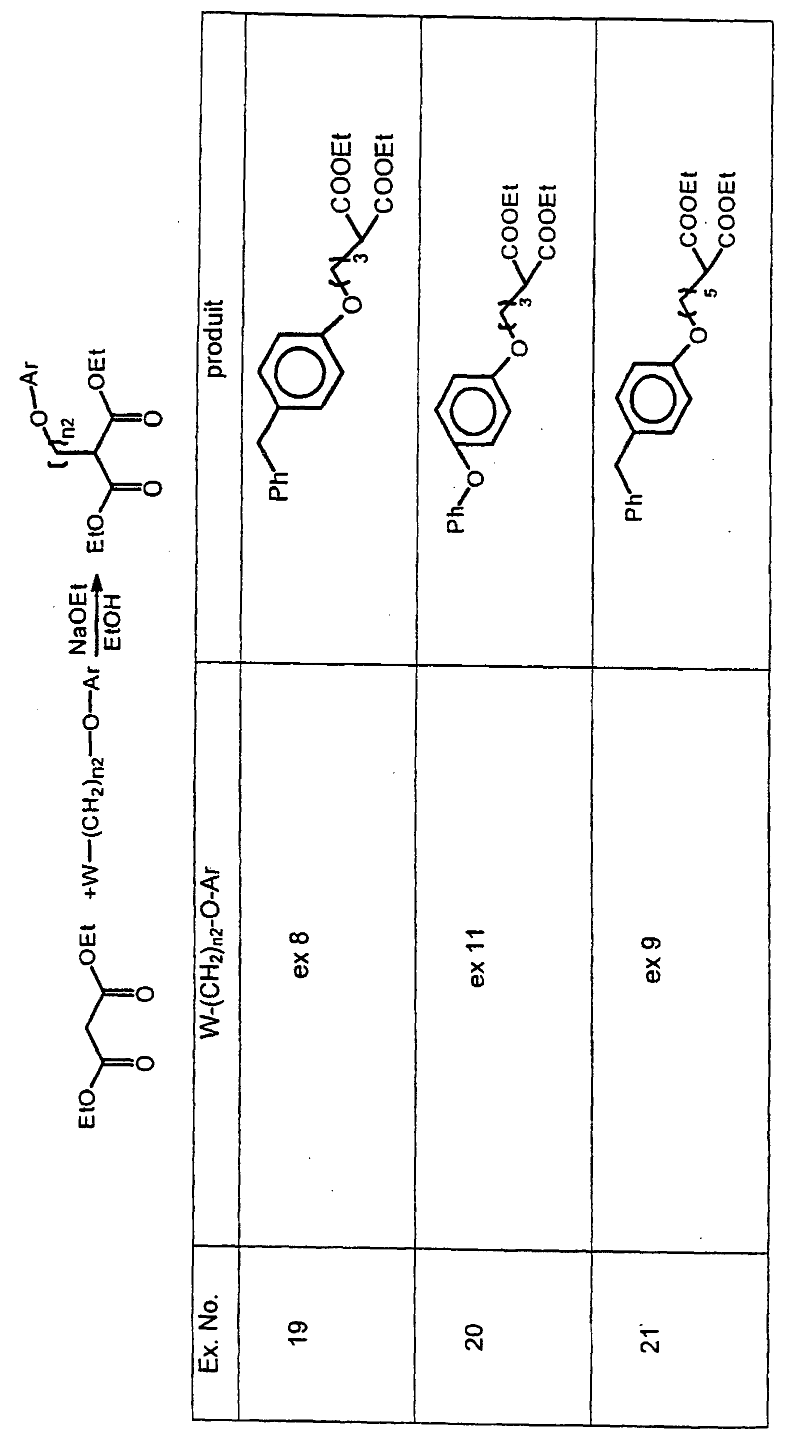

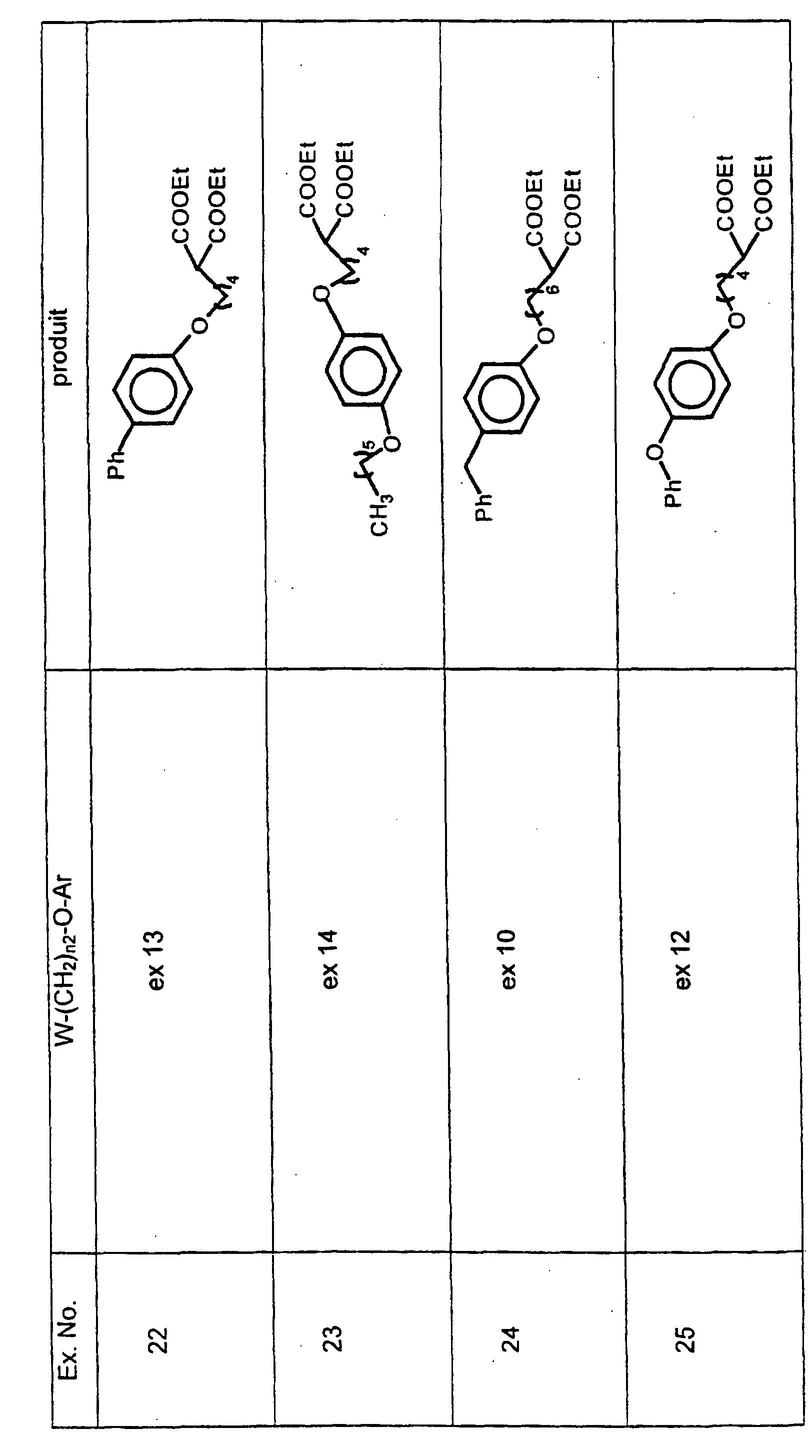

### Exemple 26

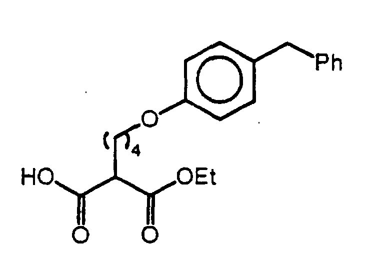

A une solution de 6,4 g (16,08 mmol) de diester de l'exemple 18 dans 3 ml d'EtOH, on ajoute à 0°C une solution de 1,09 g (16,51 mmol) de potasse dans 16 ml d'EtOH.

On agite pendant une nuit à 0°C.

Le milieu est ensuite concentré. Le résidu est repris avec 100 ml d'eau et lavé à l'Et₂O (2 fois 30 ml). La phase aqueuse est refroidie puis acidifiée par une solution d'acide chlorhydrique concentré. La phase aqueuse est extraite avec de l'éther (2 fois 40 ml). Les phases éthérées sont réunies, séchées sur MgSO₄, filtrées et concentrées. On obtient ainsi 4,71 g (79 %) d'huile jaune très visqueuse.

Les exemples 27 à 33 sont préparés selon le même procédé que celui décrit pour l'exemple 26.

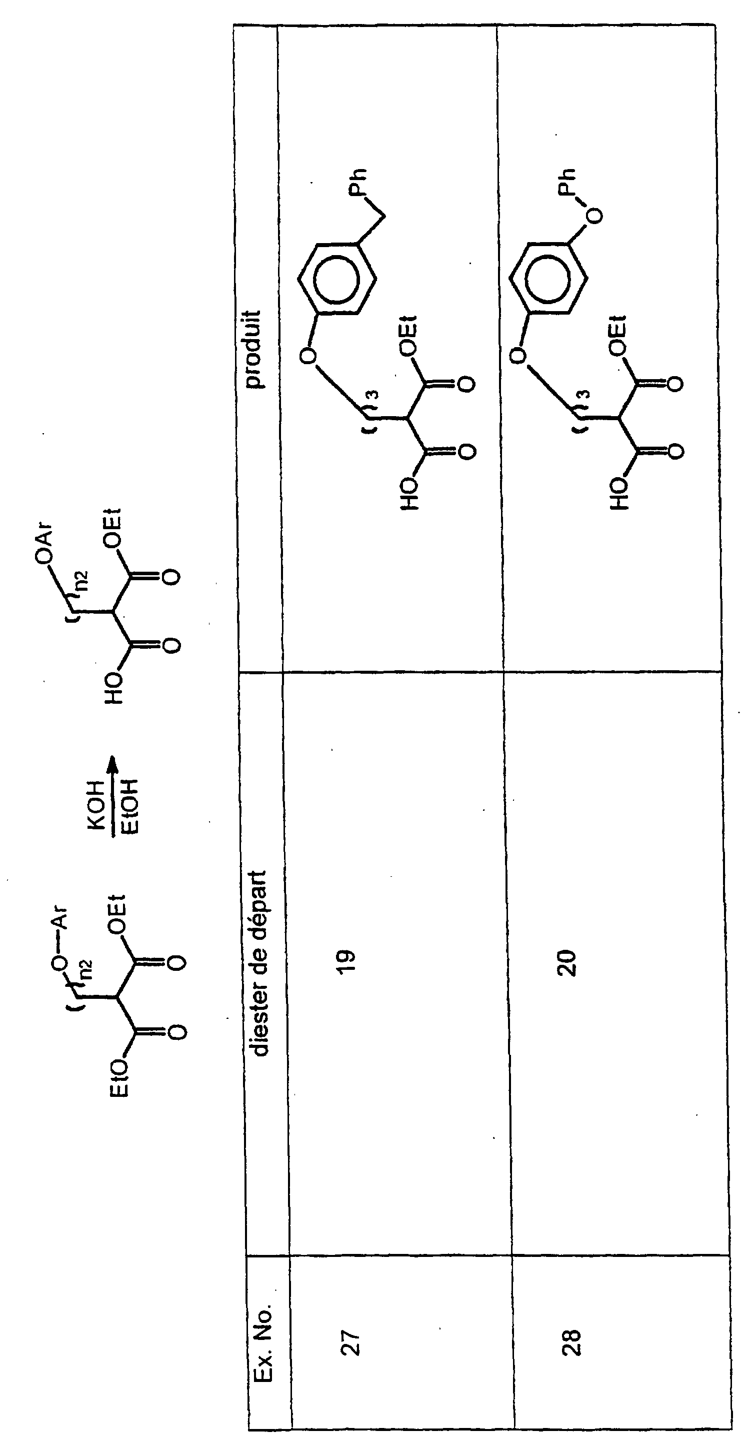

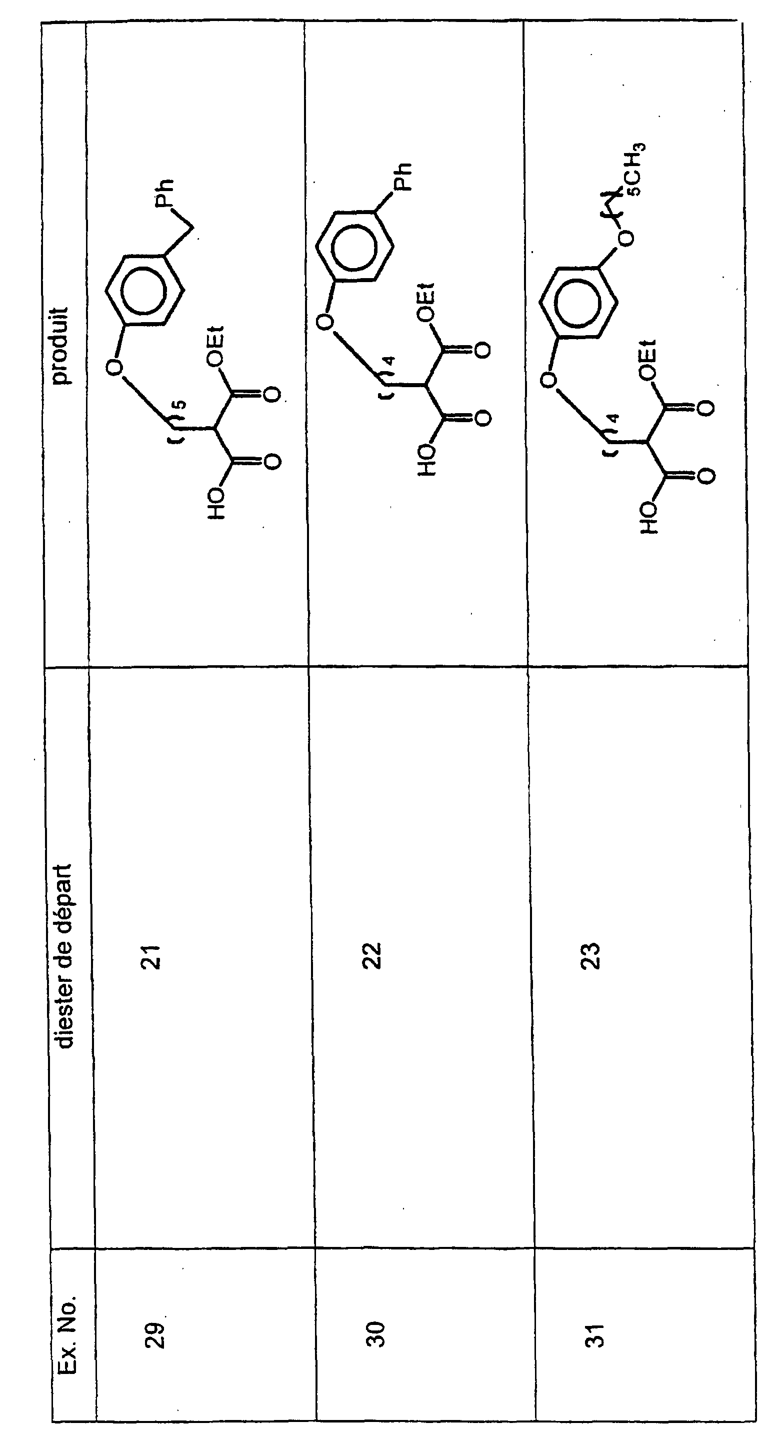

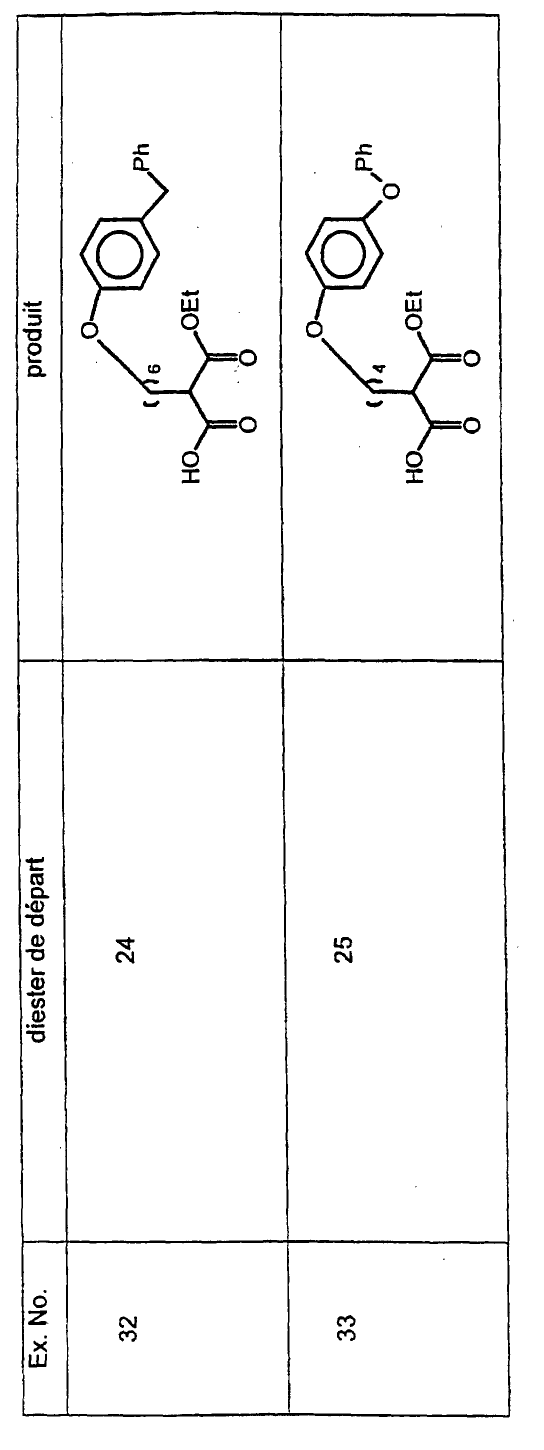

### Exemple 34

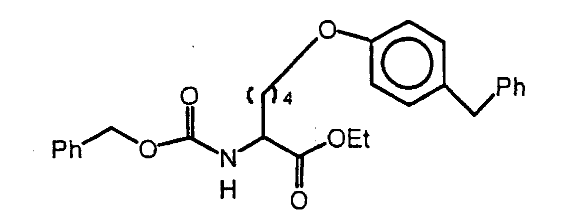

A une solution de 4,71 g du mono-acide de l'exemple 26 dans 20 ml de toluène, on ajoute goutte à goutte 3,67 g (13,33 mmol) de DPPA puis 1,34 g (13,33 mmol) de NEt₃. On chauffe à 80°C pendant 1 heure. On revient à température ambiante et on ajoute 1,65 g (15,27 mmol) d'alcool benzylique et on chauffe à 80°C pendant une nuit. La phase toluènique est successivement lavée à l'eau (1 fois 10 ml), par une solution aqueuse saturée d'hydrogénocarbonate de sodium (1 fois 10 ml) et à l'eau (1 fois 5 ml). On sèche la phase organique sur MgSO₄, filtre et concentre sous vide.

On obtient ainsi 6,5 g de produit brut. Celui-ci est purifié par chromatographie éclair sur silice avec le mélange éther éthylique-éther de pétrole (3/7) comme éluant. Nous obtenons 4,55 g (9,55 mmol ; Rendement = 75 %) de carbamate (huile incolore).

Les exemples 35 à 41 sont préparés selon le même procédé que celui décrit pour l'exemple 34.

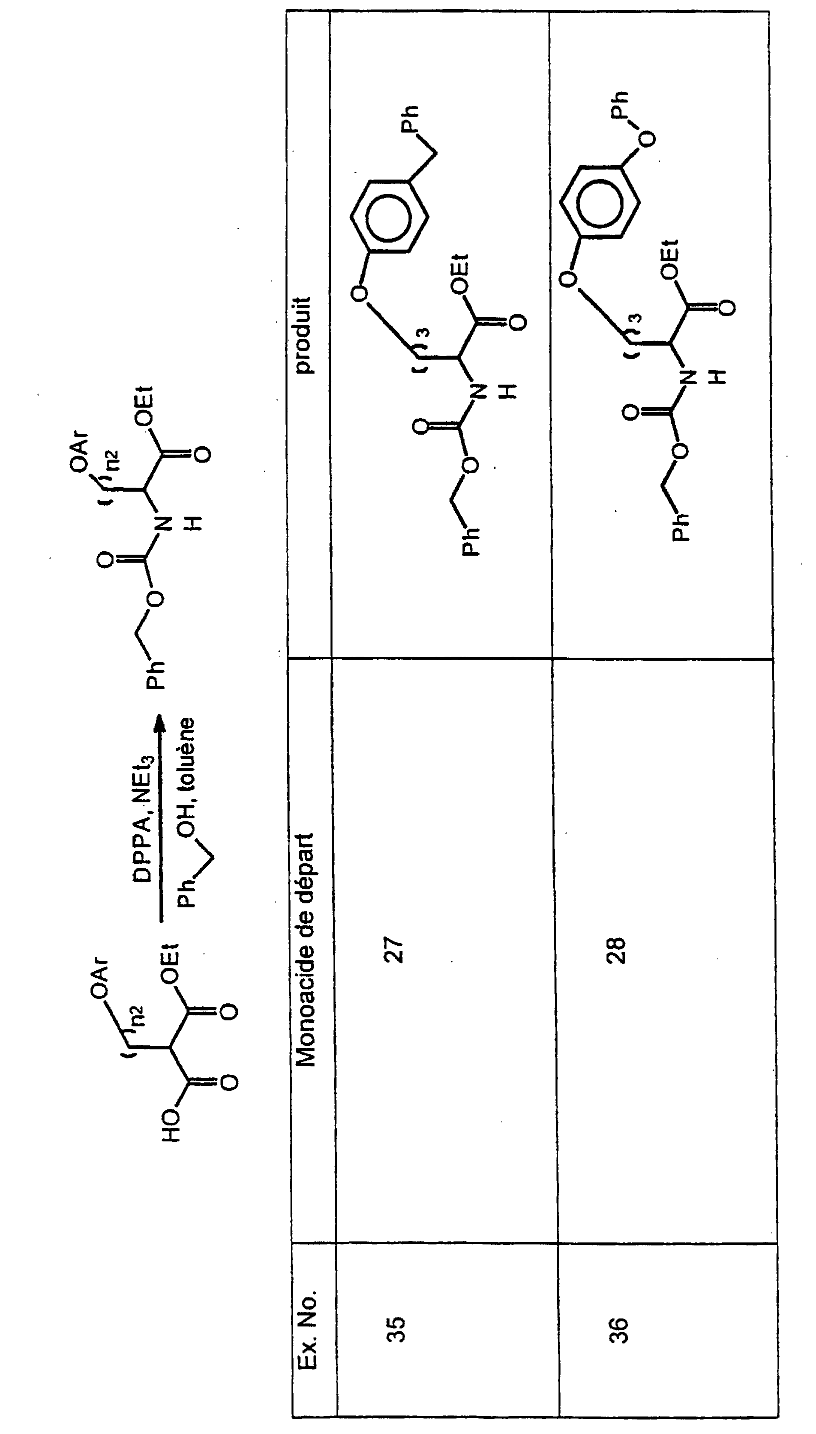

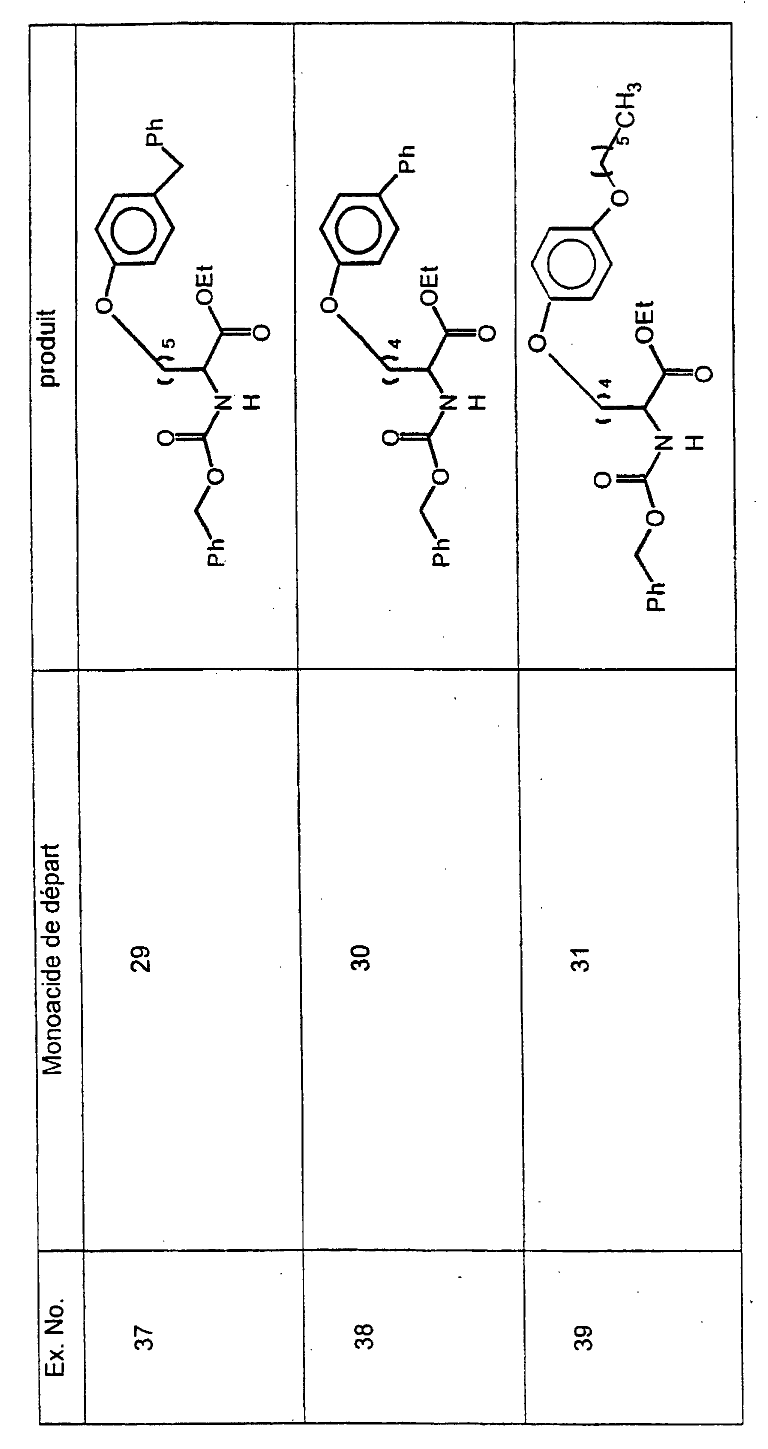

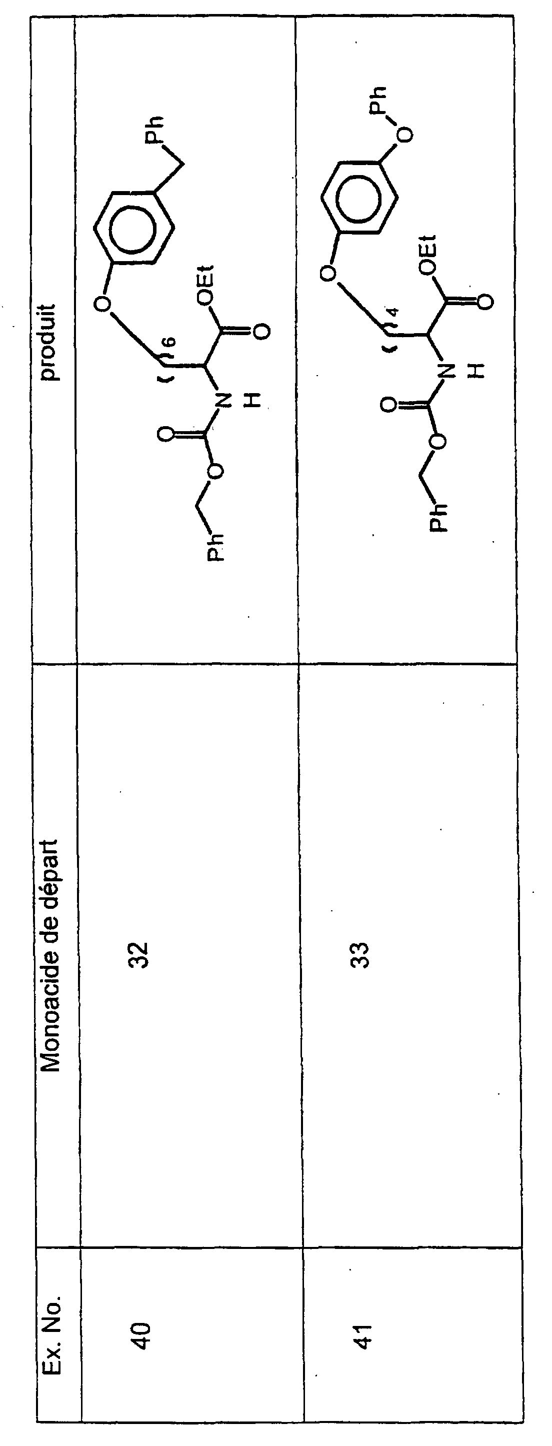

### Exemple 42

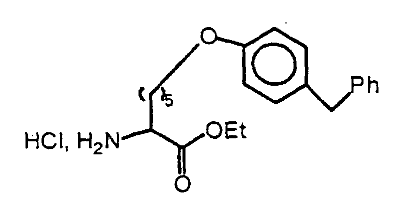

On met en solution 1 g (2,04 mmol) du carbamate de l'exemple 37 dans 20 ml d'EtOH. On ajoute ensuite 100 mg de Pd/C à 10 % puis on hydrogène à une pression d'environ 1 bar pendant une nuit à température ambiante.

La suspension est filtrée sur célite puis évaporée à sec. Le résidu huileux est repris par une solution aqueuse d'HCl concentré. La phase aqueuse acide est lavée à l'Et₂O (2 fois 20 ml). La phase aqueuse est évaporée à sec et le résidu est séché sous vide sur P₂O₅ jusqu'à masse constante. On obtient ainsi 0,64 g (Rendement 80 %) de solide blanc. La RMN¹H est en accord avec la structure chimique.

### Exemple 43

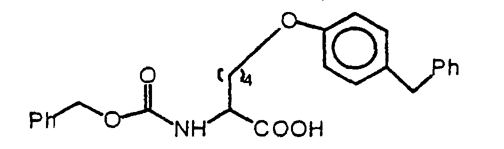

A une solution de 4,55 g (9,55 mmol) de l'ester de l'exemple 34 dans 20 ml de MeOH, on ajoute 11,5 ml d'une solution de NaOH N. On laisse agiter pendant une nuit.

On évapore le MeOH sous vide puis on lave la phase aqueuse résiduelle à l'éther éthylique (2 fois 15 ml).

La phase aqueuse basique est refroidie à environ 5°C, est acidifiée jusqu'à pH = 1 avec une solution d'HCl N et est extraite par de l'éther éthylique (2 fois 20 ml). Après séchage sur MgSO₄, filtration et concentration sous vide, on obtient 3,33 g (78 %) de solide blanc.

Les exemples 44 à 50 sont préparés selon le même mode opératoire que celui décrit pour l'exemple 43.

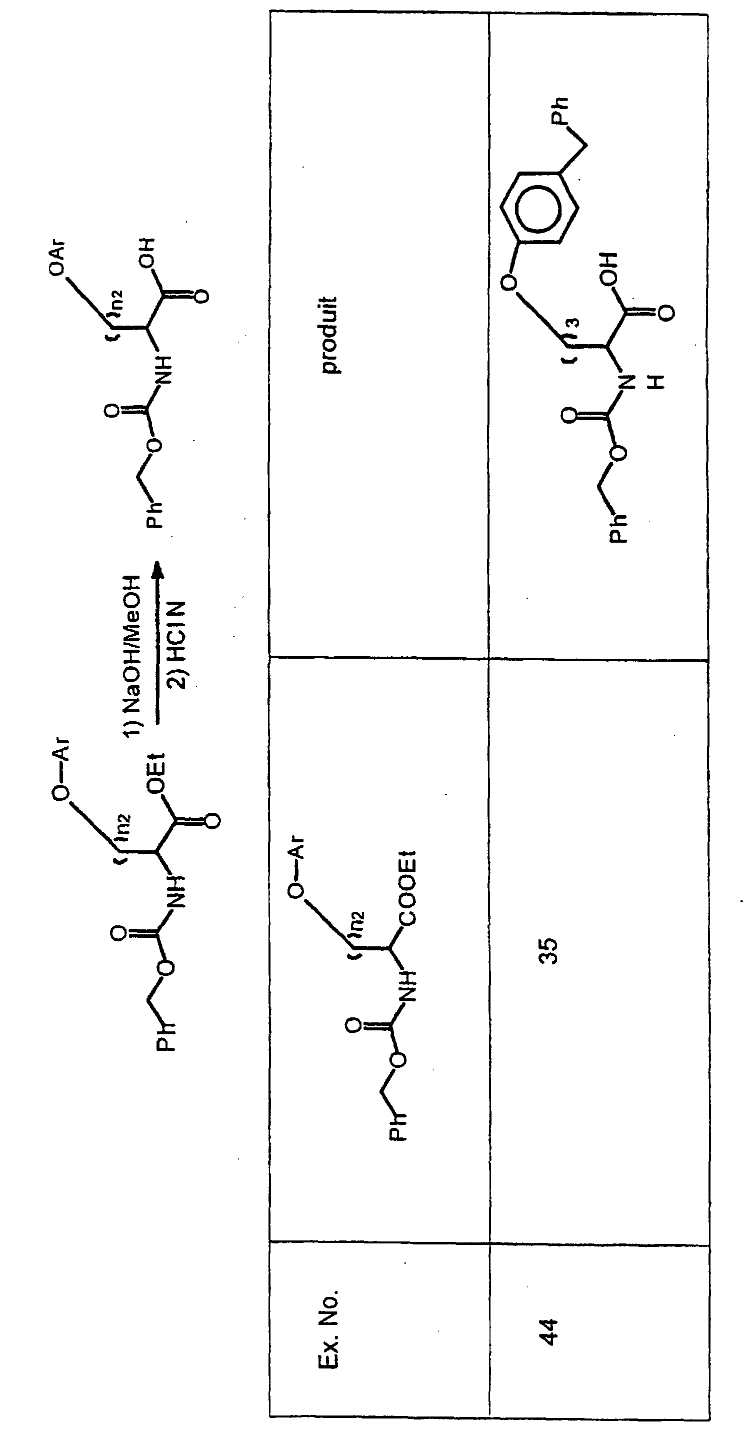

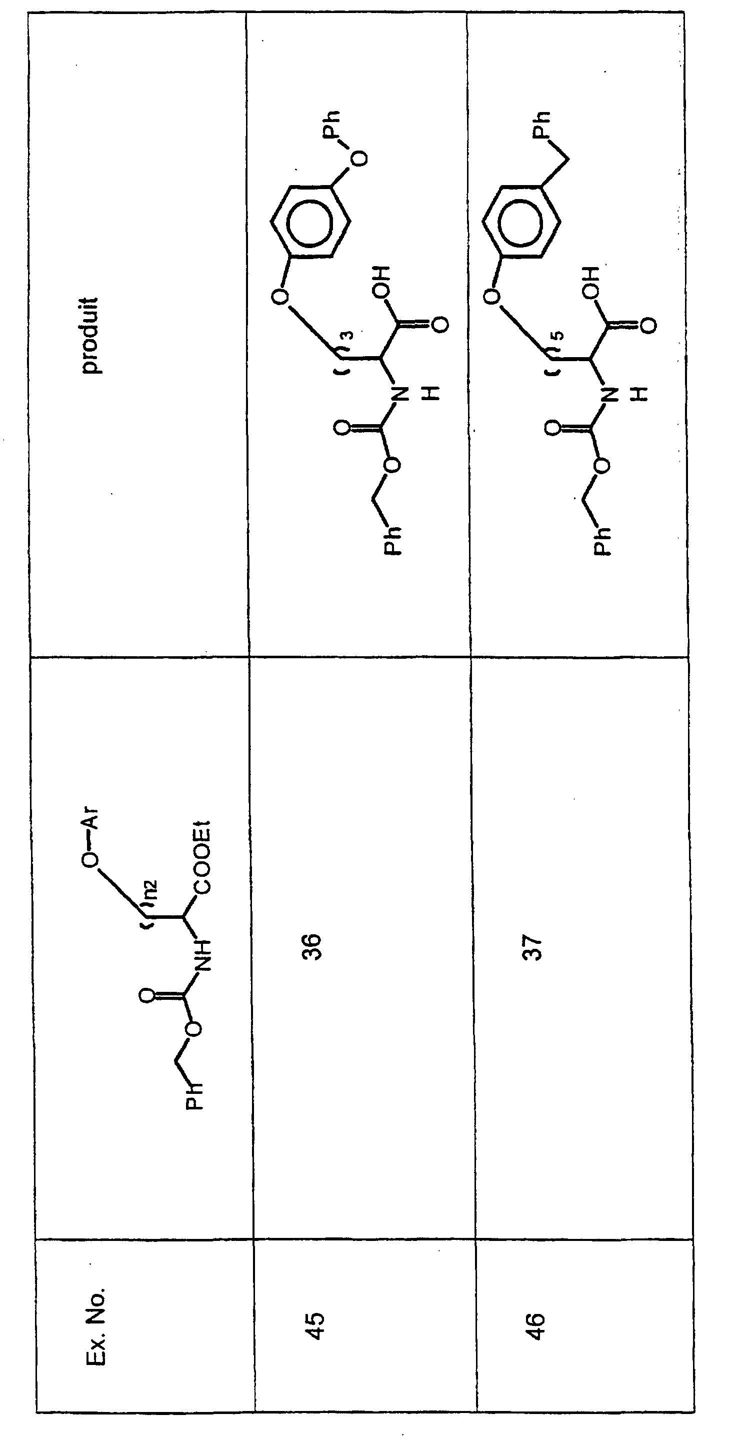

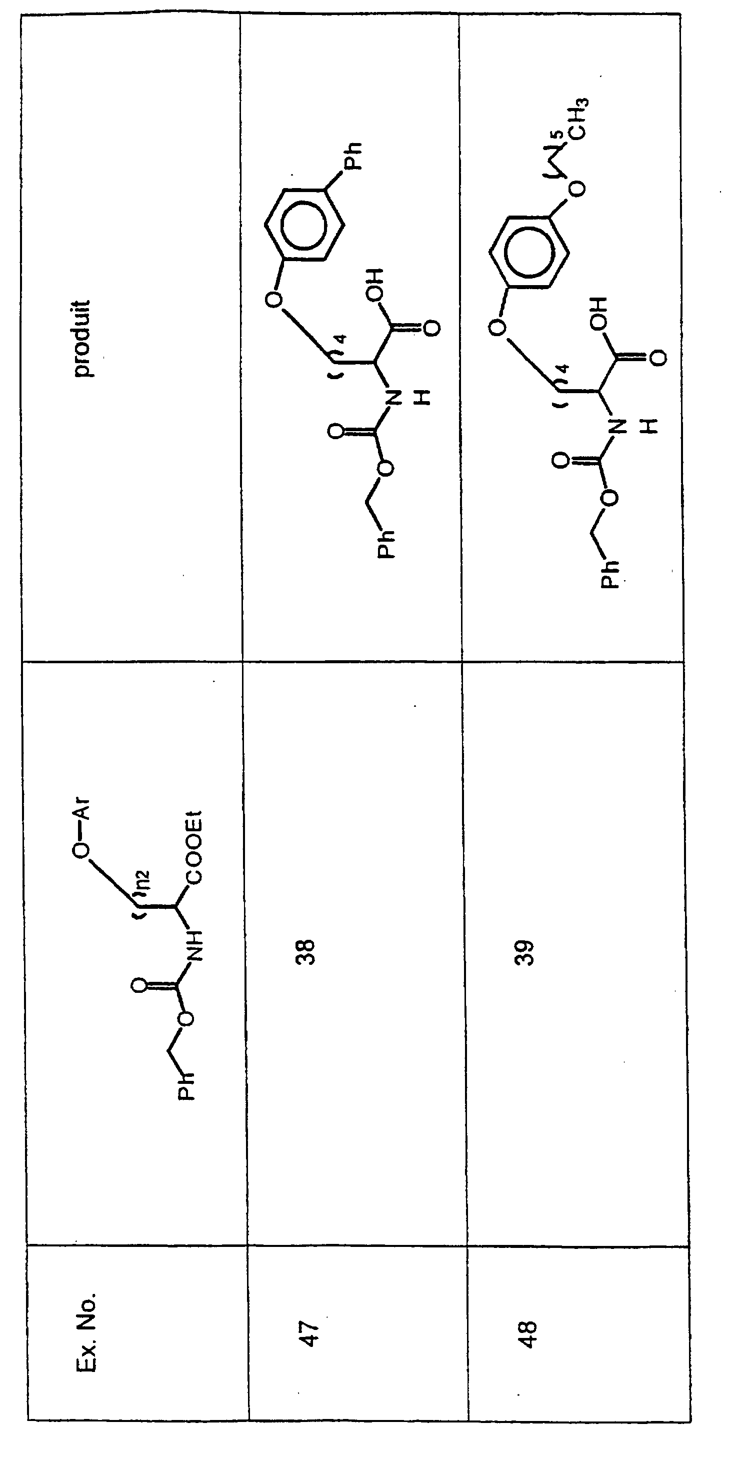

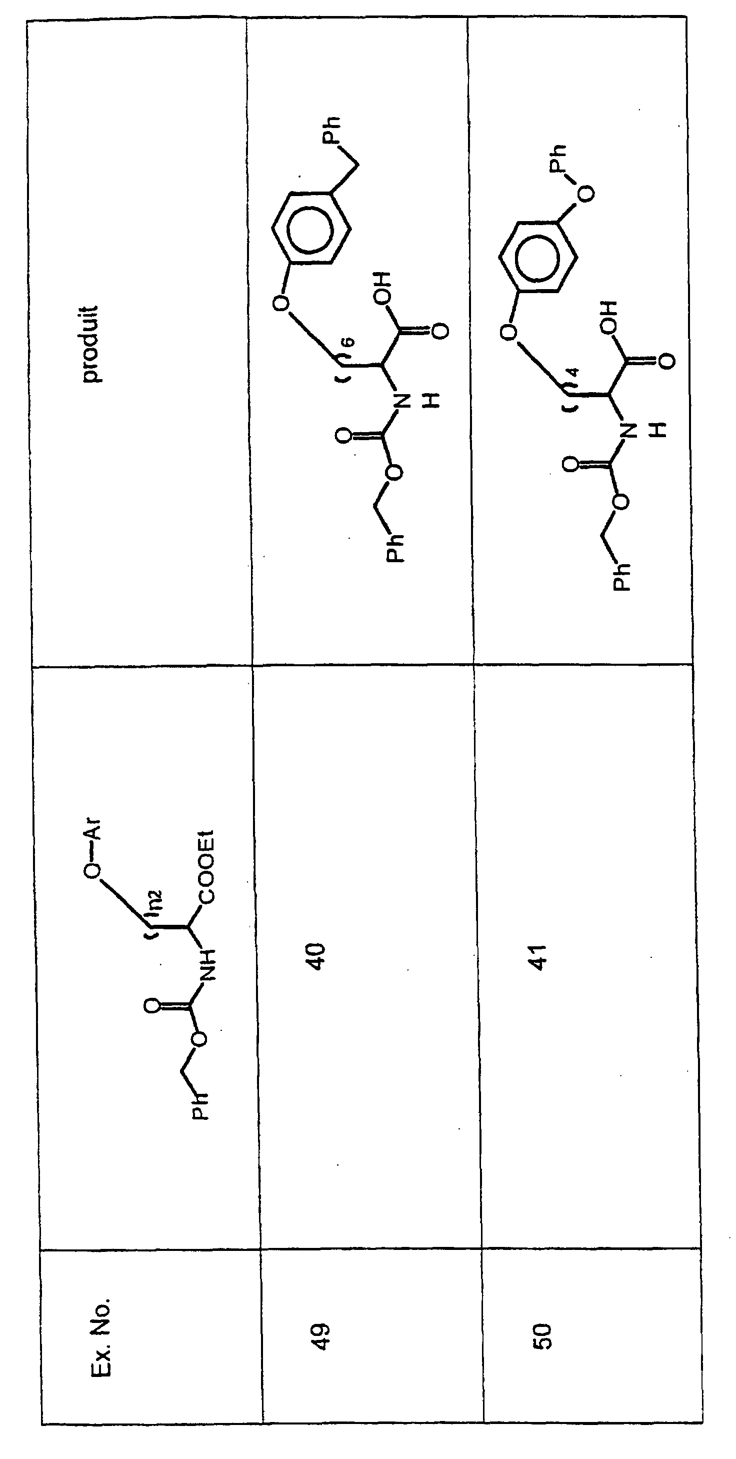

### Exemple 51

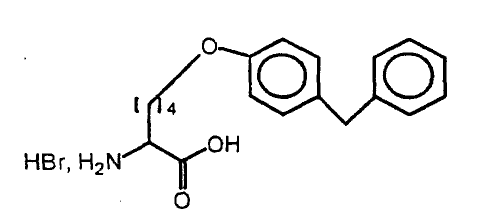

Dans un ballon, on introduit 0,89 g (1,99 mmol) du carbamate de l'exemple 43 et 5 ml d'une solution saturée d'HBr gazeux dans l'acide acétique. On maintient l'agitation pendant 2 heures.

On évapore l'acide acétique sous vide et on triture le résidu huileux dans de l'éther éthylique anhydre. On filtre et on lave à l'éther éthylique. Le solide blanc est séché sous vide sur P₂O₅. On obtient 0,52 g (66 %) de l'aminoacide désiré (Fusion > 200°C).

La RMN¹H est en accord avec la structure chimique.

Les exemples 52 à 58 sont préparés selon le même mode opératoire que celui décrit à l'exemple 51.

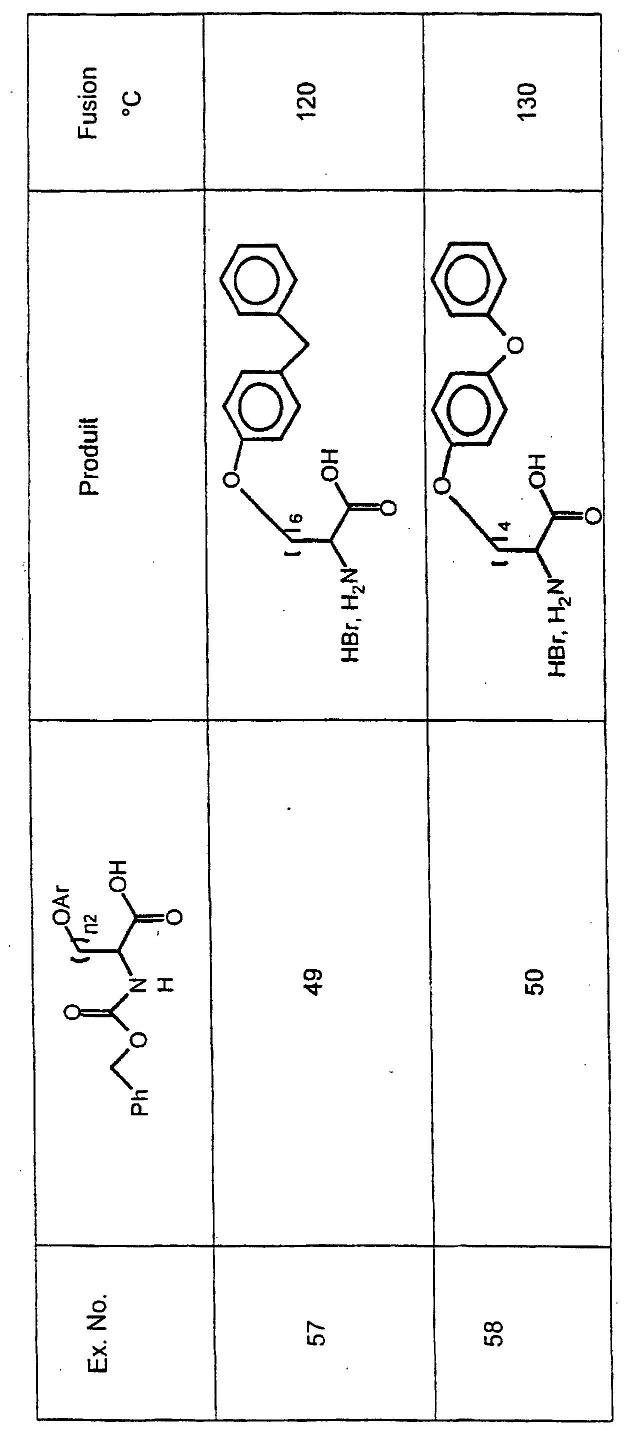

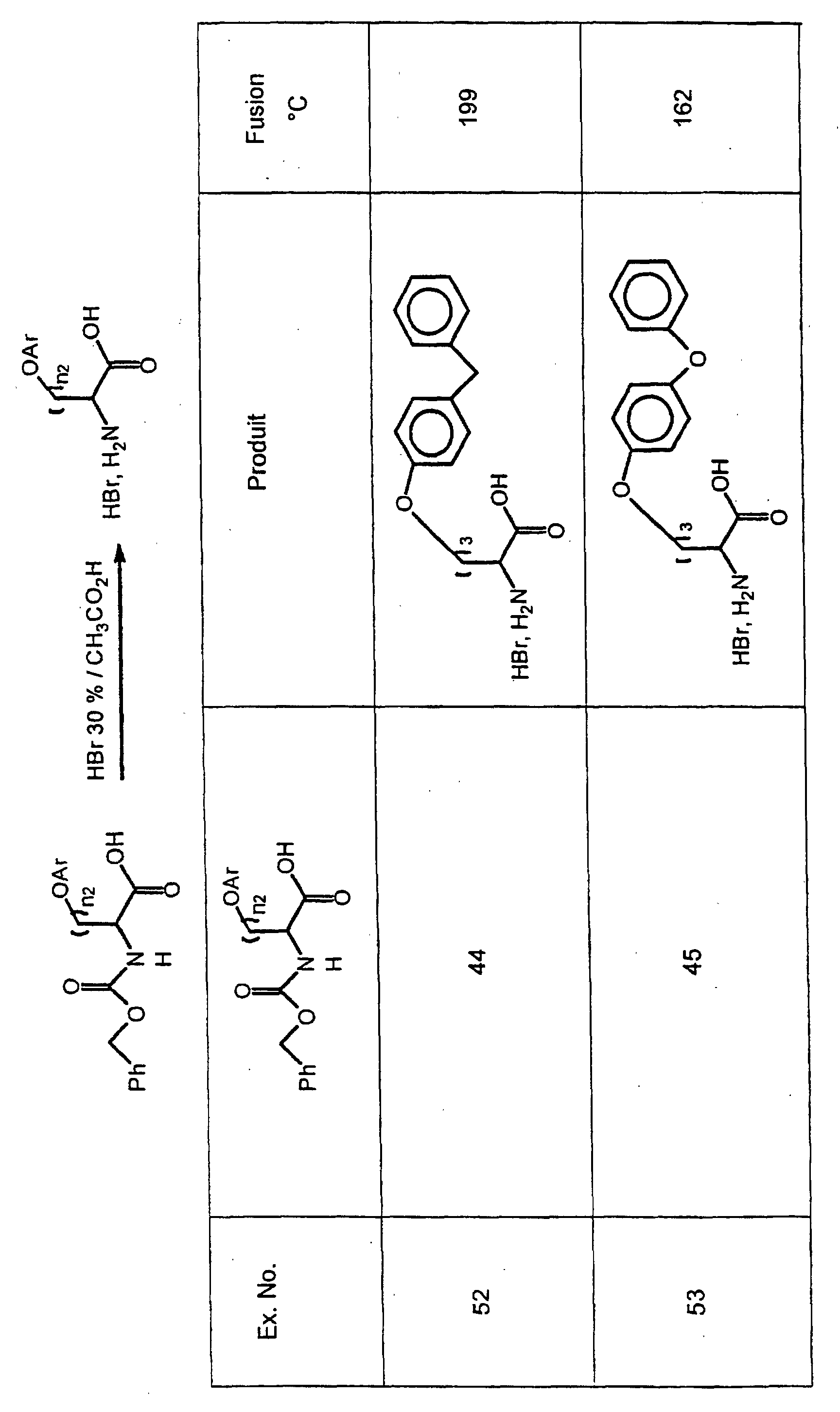

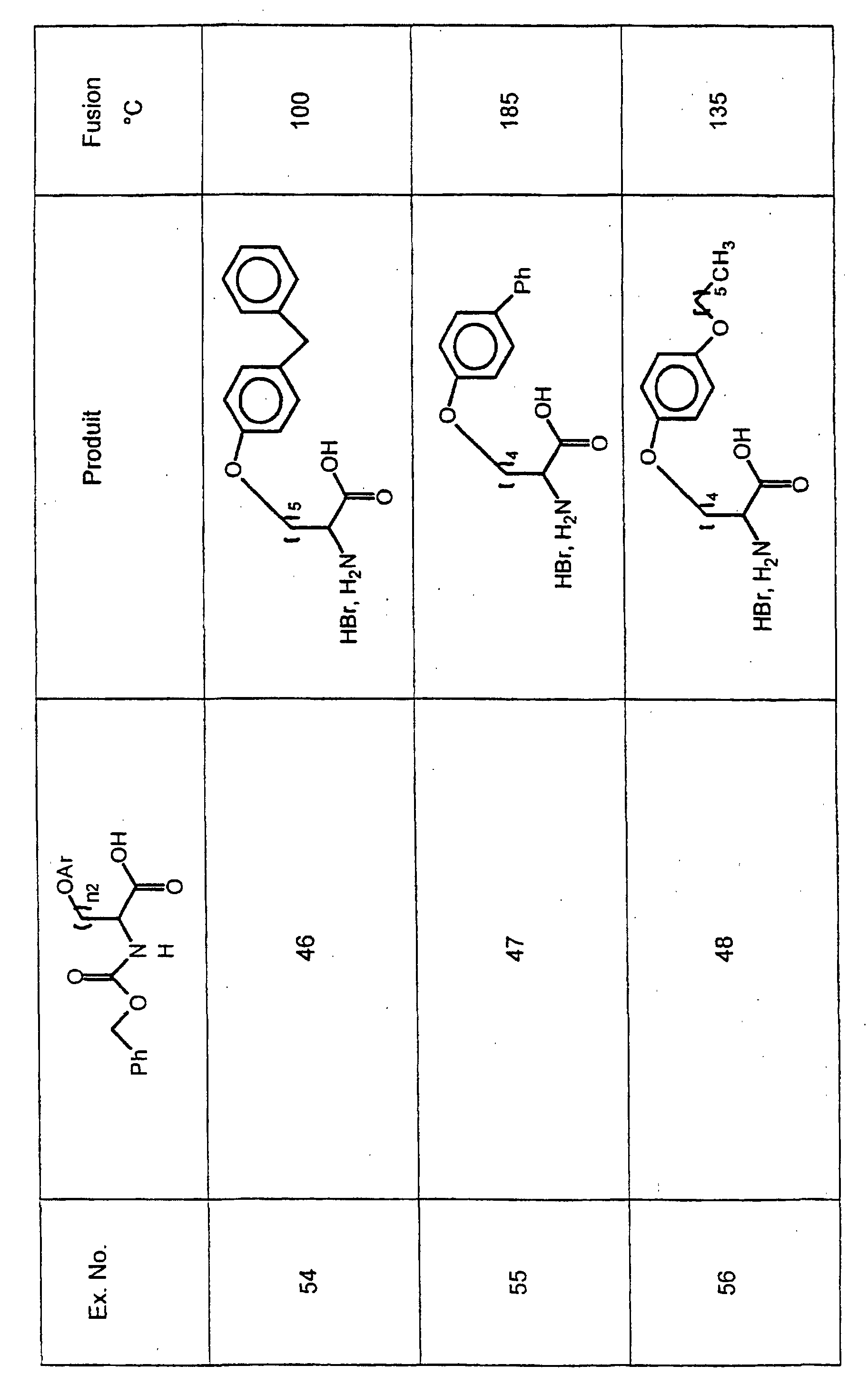

### Exemple 59

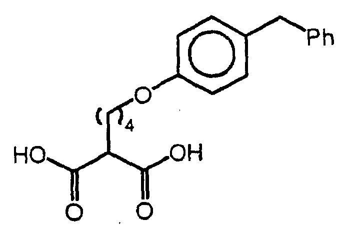

9,25 g (23,24 mmol) du diester malonique de l'exemple 18 sont dilués avec 10 ml d'eau. On ajoute 2,32 g (58,00 mmol) de pastilles de soude.

On agite et porte au reflux pendant 1 h 30.

On dilue avec de l'eau et lave avec de l'Et₂0 (1 fois 15 ml).

La phase aqueuse est refroidie et acidifiée par une solution aqueuse d'acide chlorhydrique concentré jusqu'à pH = 1. On extrait à l'Et₂O (2 fois 25 ml). Les phases éthérées sont réunies, séchées sur MgSO₄, filtrées et concentrées. On obtient 7,92 g (100 %) de solide blanc.

### Exemple 60

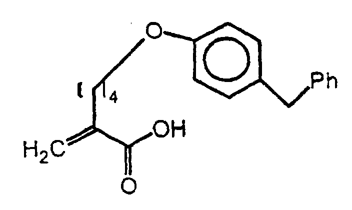

A une solution de 7,92 g (23,15 mmol) du diacide malonique de l'exemple 59 dans 50 ml d'AcOEt, on ajoute 1,69 g (23,15 mmol) de diéthylamine puis 1,04 g (30,40 mmol) de paraformaldéhyde.

On porte au reflux pendant 30 minutes.

La solution est ensuite refroidie à l'aide d'un bain de glace et d'eau, diluée avec 10 ml d'eau puis acidifiée avec une solution d'HCl 3N jusqu'à pH = 1. La phase aqueuse est éliminée. La phase organique est lavée avec de l'eau (une fois 10 ml), séchée sur MgSO₄, filtrée et concentrée.

On obtient 6,04 g (84 %) de solide blanc.

### Exemple 61

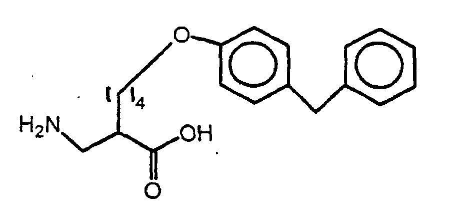

On porte au reflux une solution de 0,44 g (19,13 mmol) de sodium dans 15 ml d'EtOH anhydre. On ajoute à cette solution 1,35 g (19,42 mmol) de chlorhydrate d'hydroxylamine dans 1 ml d'eau chaude. On glace à 5°C, filtre et lave le précipité avec 2 ml d'EtOH anhydre.

On ajoute au filtrat 3 g (9,70 mmol) d'acide acrylique de l'exemple 60. On agite et porte au reflux pendant 24 heures.

On filtre, lave à l'eau, à l'EtOH puis à l'éther éthylique. On obtient 0,71 g de solide blanc (22 %) (Fusion > 200°C). La RMN¹H est en accord avec la structure chimique.

### Exemple 62

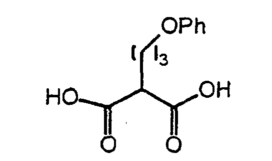

Le dérivé chloré de l'exemple 15 réagit avec le malonate de diéthyle selon le même procédé que celui décrit à l'exemple 18. Le malonate ainsi obtenu est saponifié selon le même mode opératoire que celui décrit à l'exemple 59 pour conduire au composé désiré.

### Exemple 63

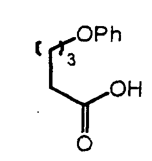

Le diacide de l'exemple 62 est décarboxylé par chauffage à 140°C jusqu'à la disparition du dégagement gazeux pour conduire à l'acide désiré.

### Exemple 64

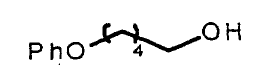

A une suspension de 7,35 g (193,67 mmol) de LiAlH₄ dans 210 ml d'Et₂O anhydre, on ajoute une solution de 31,34 g (161,36 mmol) d'acide de l'exemple 63 dans 138 ml d'Et₂O anhydre. On agite une nuit à température ambiante.

On glace à 5°C et ajoute successivement 5,25 ml d'eau, 5,25 ml de NaOH 15 % et 15,75 ml d'eau. Après 2 heures d'agitation on filtre, rince à l'éther éthylique et concentre le filtrat.

On obtient 19,97 g (68 %) d'alcool.

### Exemple 65

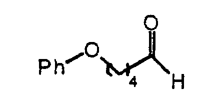

A une solution de 48 g (222,67mmol) de PCC dans 220 ml de CH₂Cl₂ refroidie à 0°C, on ajoute une solution de 19,77 g (110,8 mmol) de l'alcool de l'exemple 64 en solution dans 135 ml de CH₂Cl₂. On agite pendant 3 heures à température ambiante, filtre sur célite, évapore à sec et purifie par chromatographie éclair (éther éthylique/heptane 4/6). On obtient 9,94 g (55,77 mmol) d'aldéhyde.

### Exemple 66

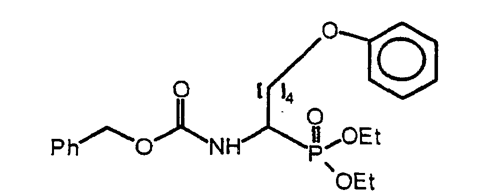

On mélange 6,75 g (44,6 mmol) de carbonate de benzyle, 6,5 g (44,6 mmol) de diéthyle phosphite et 33,5 ml de chlorure d'acétyle. On refroidit à -5°C et ajoute, goutte à goutte, 9,94 g (55,77 mmol) d'aldéhyde de l'exemple 65. On agite pendant une heure à 0°C puis une nuit à température ambiante.

On élimine l'excès de chlorure d'acétyle par évaporation sous vide puis on reprend le résidu avec 50 ml de CH₂Cl₂. On lave successivement avec de l'eau (une fois 30 ml), avec une solution aqueuse saturée de bisulfite de sodium (2 fois 30 ml), avec une solution aqueuse saturée de NaHCO₃ (3 fois 30 ml) et avec de l'eau (une fois 60 ml). La phase organique est séchée sur MgSO₄, filtrée et concentrée. On obtient 20,19 g de produit brut que l'on chromatographie sur silice (éluant Et₂O).

On récupère ainsi 10,93 g (54,5 %) de produit.

### Exemple 67

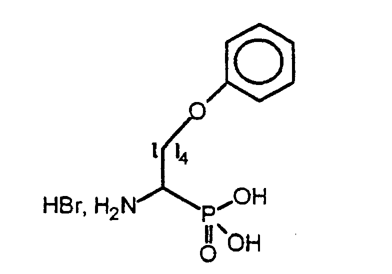

Dans un ballon on introduit 1,3 g (2,4 mmol) du phosphonate de l'exemple 66 et 3,5 ml d'une solution d'HBr gazeux à 30 % dans l'acide acétique. On maintient l'agitation pendant 24 heures.

On évapore à sec, triture le résidu huileux dans de l'Et₂O anhydre, on ajoute de l'eau et filtre le solide formé. On sèche sous vide sur P₂O₅. On obtient 0,62 g (1,44 mmol) de solide blanc.

Fusion : > 250°C.

La RMN¹H est en accord avec la structure chimique.

### Exemple 68

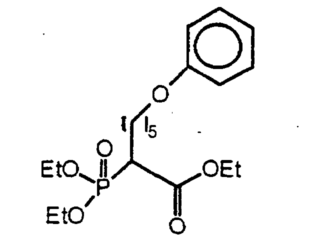

On met en solution 4,9 g (21,9 mmol) de triéthylphosphonoacétate dans 21 ml de DMF anhydre. On glace à 0°C et ajoute 0,56 g (21,9 mmol) de NaH par portions. On agite pendant 15 minutes à 0°C.

On ajoute une solution de 5,33 g (21,9 mmol) de dérivé bromé de l'exemple 16 dans 13 ml de DMF anhydre. On agite une nuit à température ambiante.

On dilue avec Et₂O, lave à l'eau, sèche la phase organique sur MgSO₄, filtre et concentre. On obtient 6,9 g de résidu huileux que l'on purifie par chromatographie sur silice (éluant Et₂O).

5,33 g (63 %) d'huile sont obtenus.

Les exemples 69 à 71 sont obtenus selon le même mode opératoire que celui décrit pour l'exemple 68.

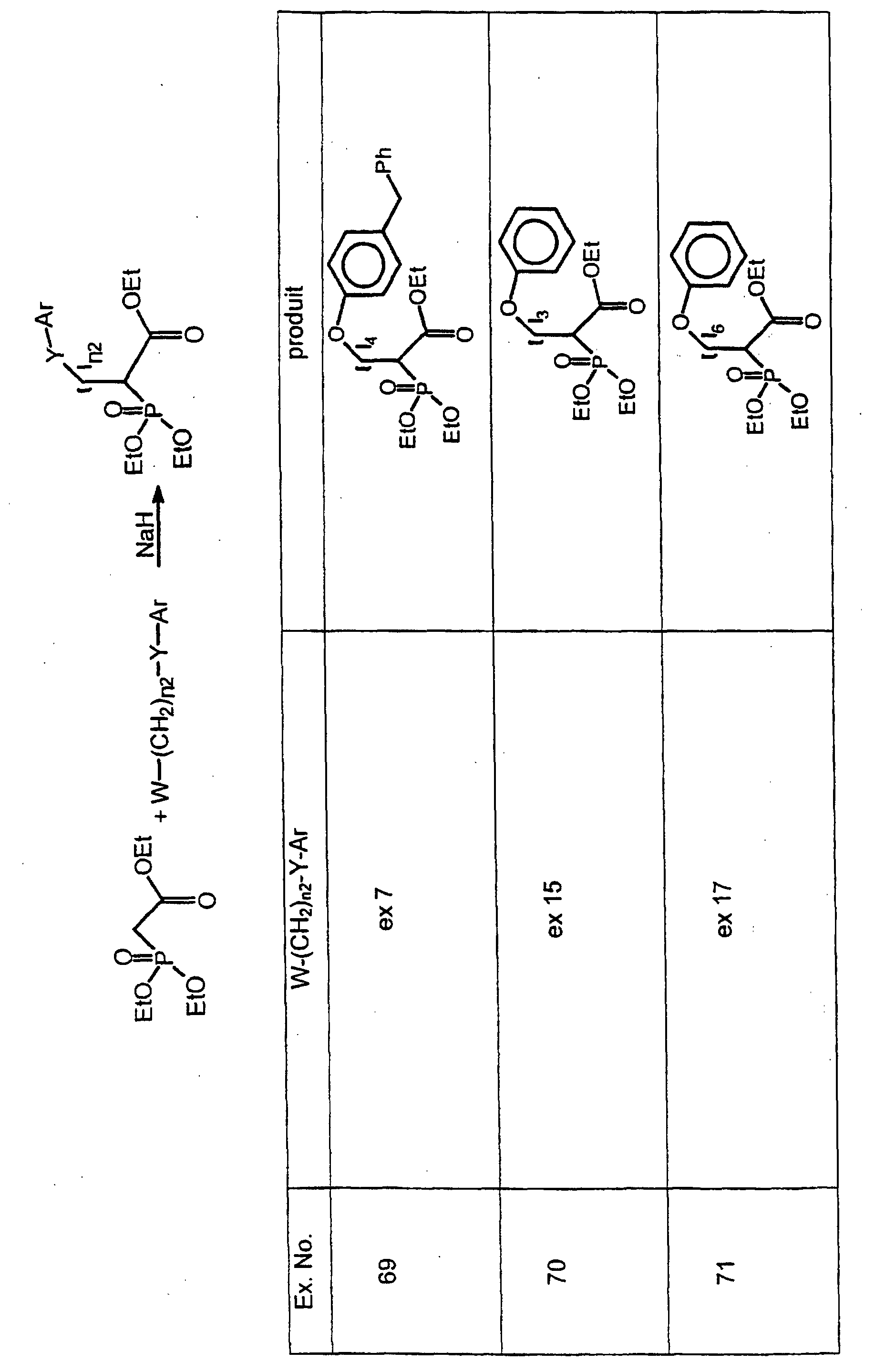

### Exemple 72

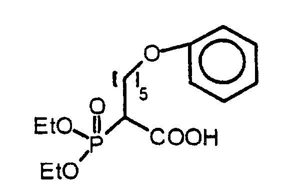

Une solution de 5,33 g (13,8 mmol) de dérivé de l'exemple 68 dans 32 ml de MeOH est agitée avec 20,7 ml de LiOH M. On chauffe pendant 1 heure au reflux.

On évapore à sec, ajoute de l'eau et lave avec de l'Et₂O. On acidifie la phase aqueuse avec une solution d'HCl N et l'extrait à l'Et₂O. Les phases éthérées sont réunies, séchées sur MgSO₄, filtrées et concentrées. On obtient 3,93 g (79 %) d'acide désiré.

Les exemples 73 à 75 sont obtenus selon le même mode opératoire que celui décrit pour l'exemple 72.

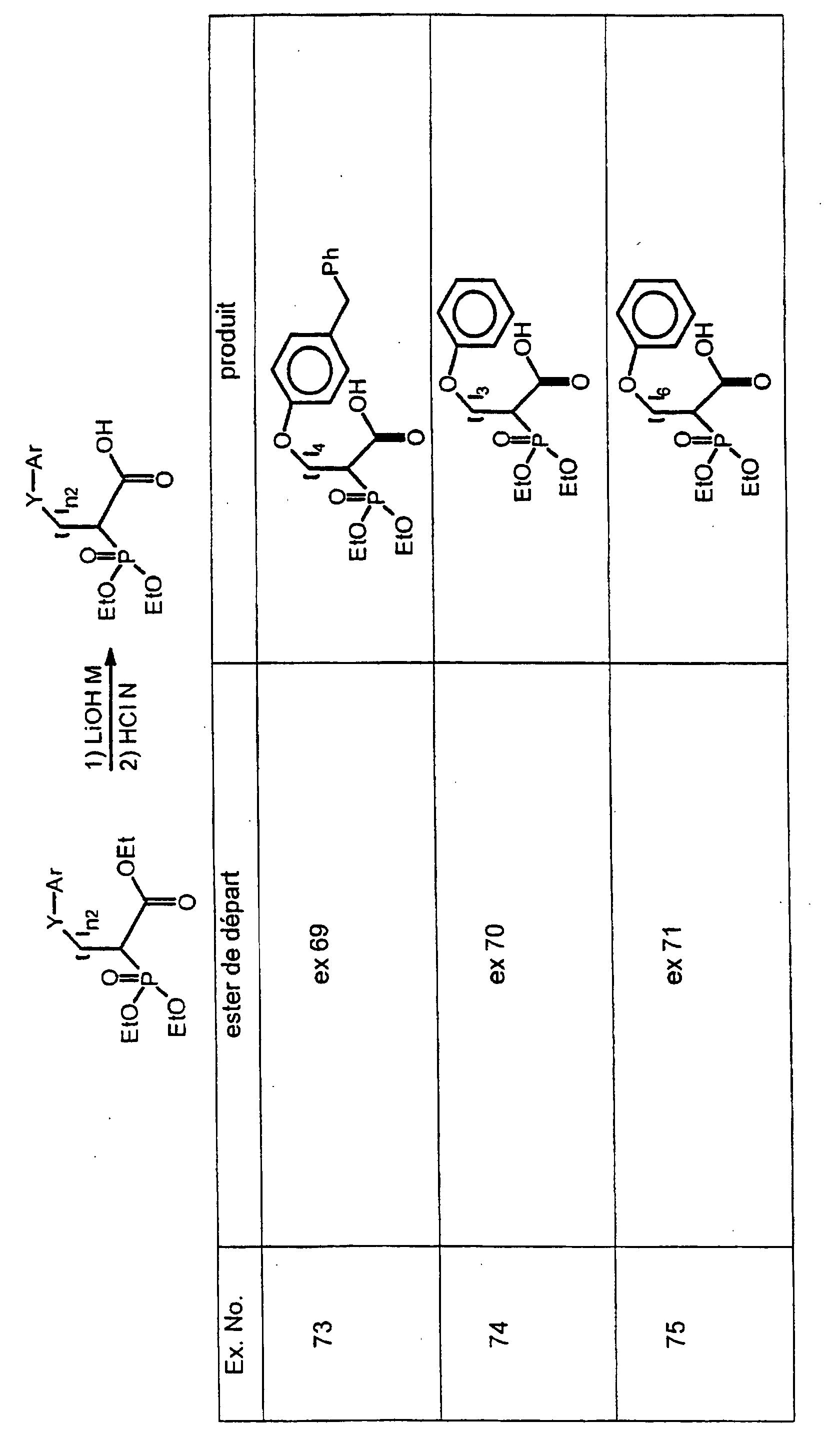

### Exemple 76

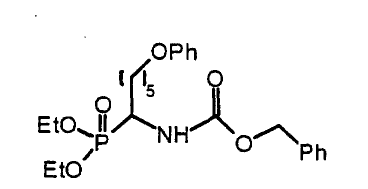

L'acide de l'exemple 72 est transformé en carbamate 76 selon le même mode opératoire que celui décrit à l'exemple 34.

Les exemples 77 à 79 sont obtenus selon le même mode opératoire que celui décrit à l'exemple 34.

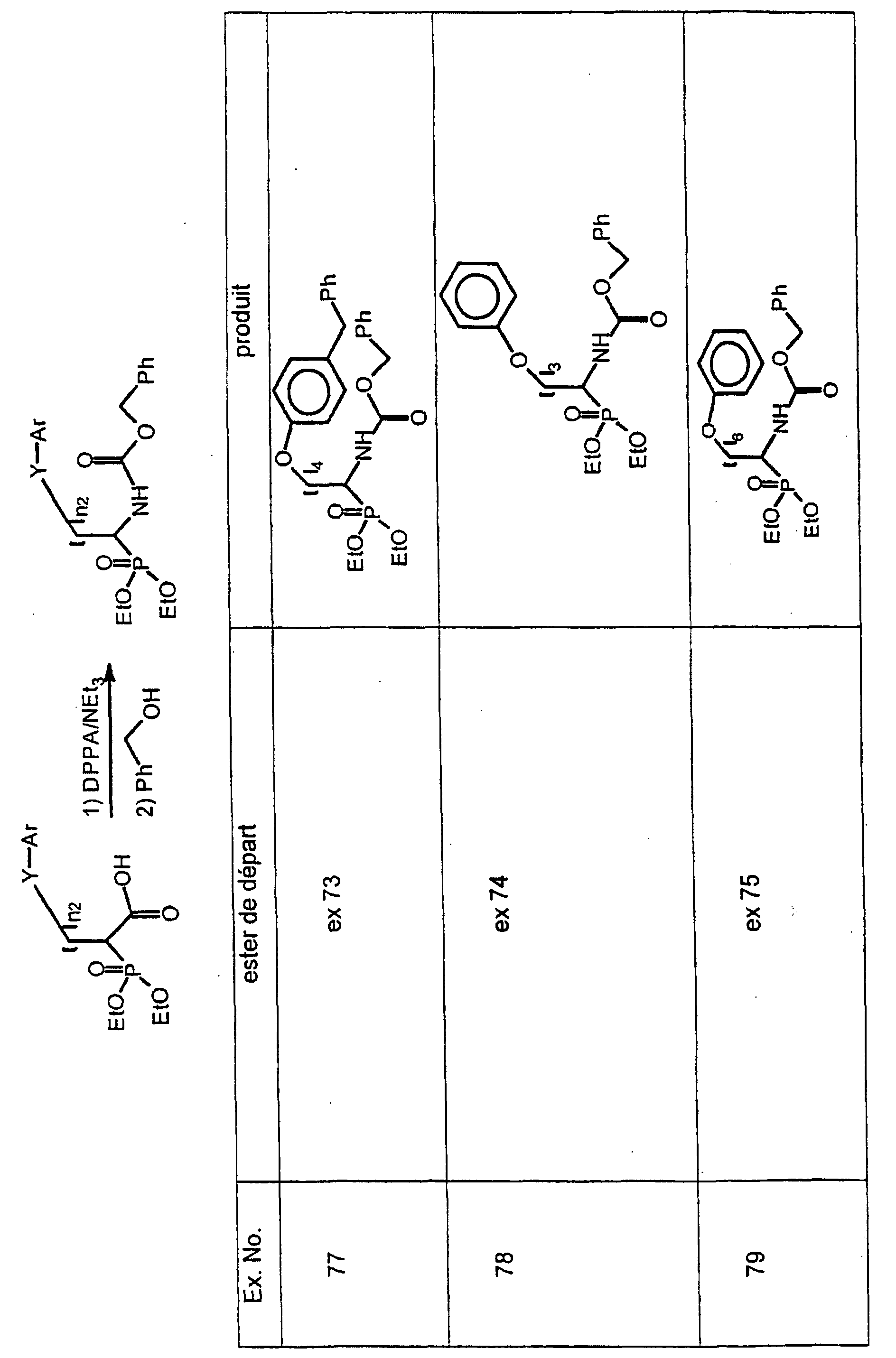

### Exemple 80

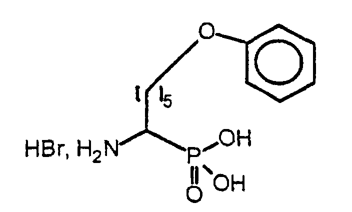

Le phosphonate de l'exemple 76 est transformé en dérivé aminophosphonique selon le même mode opératoire que celui décrit à l'exemple 67 (Fusion > 250°C).

La RMN¹H est en accord avec la structure chimique.

Les exemples 81 à 83 sont obtenus selon le même mode opératoire que celui décrit à l'exemple 67.

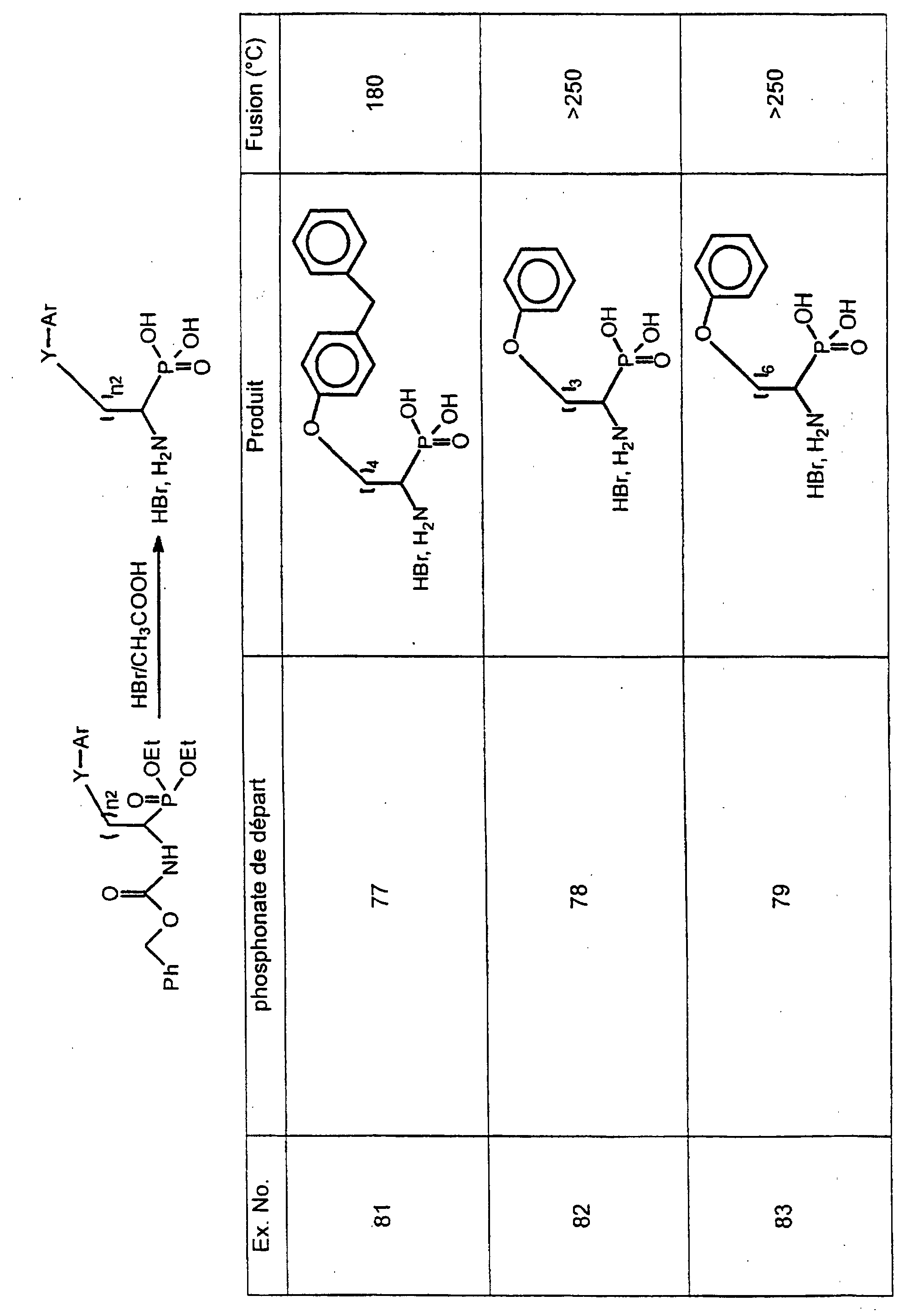

### Exemple 84

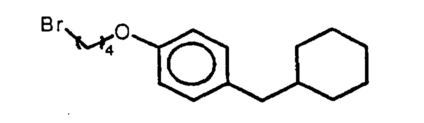

Le composé de l'exemple 84 est préparé à partir du 1,4-dibromobutane et du 4-(cyclohexylméthyl)-phénol (Helv. Chem. Acta. Vol 77, (1994), 1241 et 1255) selon le même mode opératoire que celui décrit pour l'exemple 7 (méthode b).

### Exemple 85

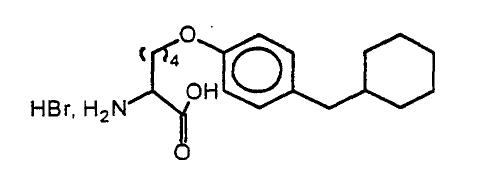

Le produit de l'exemple 85 est préparé selon la même suite réactionnelle que celle utilisée pour la synthèse de l'exemple 51.

Fusion : 121°C

### Exemple 86

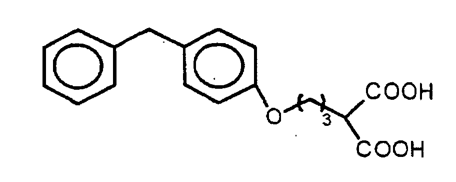

Le diester de l'exemple 19 est saponifié selon le même mode opératoire que celui décrit à l'exemple 59.

### Exemple 87

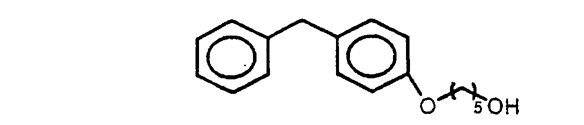

Le diacide de l'exemple 86 est décarboxylé à 130°C pendant 30 minutes.

A une suspension de 1,64 g (1,2 équivalents) de LiAIH₄ dans 47 ml d'Et₂O anhydre on ajoute une solution de 10,27 g (36,1 mmol) de l'acide obtenu après décarboxylation dans 30 ml d'Et₂O anhydre. On agite pendant une nuit à température ambiante.

Après hydrolyse et filtration on obtient 7,68 g (28,4 mmol) de l'alcool désiré.

### Exemple 88

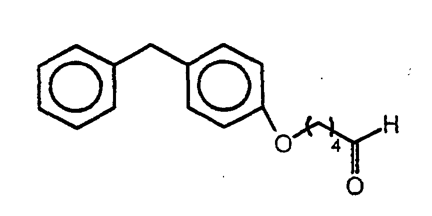

A 12,25 g (2 équivalents) de pyridinium chlorochromate en solution dans 56 ml de CH₂Cl₂, on ajoute à 0°C 7,68 g (28,4 mmol) de l'alcool précédent en solution dans 35 ml de CH₂Cl₂. Après 3 heures à température ambiante, on filtre sur silice et purifie par chromatographie éclair (éluant 7/3 heptane/Et₂O).

On obtient 4,63 g (17,25 mmol) d'aldéhyde.

### Exemple 89

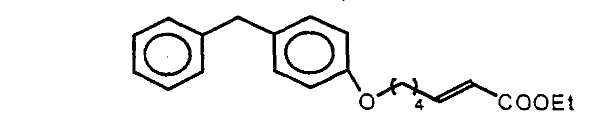

A une solution de 3,77 g (1,2 équivalents) de triméthyl-phosphonoacétate dans 52 ml de THF anhydre, on ajoute à 0°C, 523 mg (1,2 équivalents) de NaH. On agite pendant 15 minutes à 0°C puis on ajoute une solution de 4,63 g (17,25 mmol) d'aldéhyde de l'exemple 88 dans 20 ml de THF anhydre et on agite pendant 4 heures à température ambiante.

On évapore, ajoute de l'eau, extrait à Et₂O, sèche sur MgSO₄ puis on évapore. On purifie par chromatographie sur silice (éluant 1/9 Et₂O/heptane).

On obtient 2,56 g (7,89 mmol) de l'ester désiré.

### Exemple 90

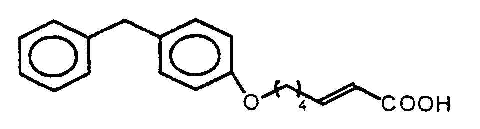

A 2,56 g (7,9 mmol) de l'ester précédent en solution dans 26 ml de MeOH, on ajoute 16 ml de NaOH N. On chauffe pendant une heure au reflux, acidifie par HCl N, extrait à l'Et₂O, sèche sur MgSO₄ filtre et concentre.

On obtient 2,36 g (7,6 mmol) d'acide acrylique désiré.

### Exemple 91

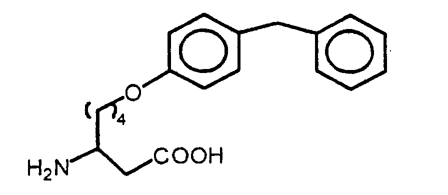

L'acide précédent est traité selon le même mode opératoire que celui décrit à l'exemple 61.

Fusion : 205°C.

La RMN ¹H est en accord avec la structure chimique.

### Exemple 92

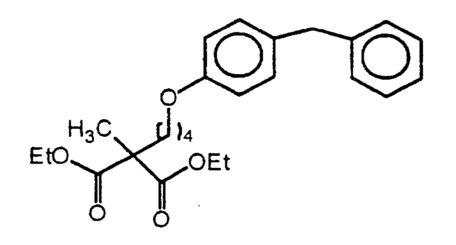

Le méthyl malonate de diéthyle est alkylé avec le dérivé bromé de l'exemple 7 selon le même mode opératoire que celui décrit à l'exemple 18.

### Exemple 93

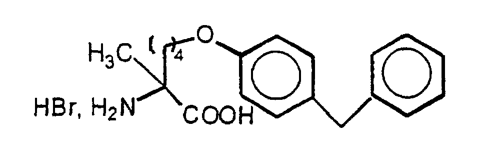

Le produit de l'exemple 92 est traité selon la même suite réactionnelle que celle utilisée pour la synthèse de l'exemple 51.

Fusion : 196°C.

### Exemple 94

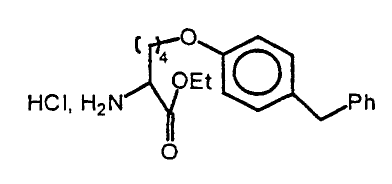

Le carbamate de l'exemple 38 est hydrogéné selon le même processus que celui décrit pour l'exemple 42 pour conduire à l'exemple 94.

Fusion > 250°C.

### Exemple 95

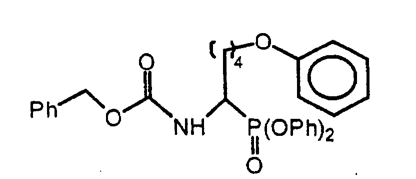

On utilise le même mode opératoire que celui décrit à l'exemple 66 à ceci près que la diéthyle phosphite est remplacée par la diphénylphosphite.

### Exemple 96

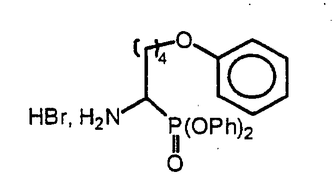

Le produit de l'exemple précédent, 0,5 g (1 mmol), est agité dans 2 ml HBr/CH₃COOH à 30 % pendant 2 heures.

On évapore à sec et triture dans Et₂O sec jusqu'à la précipitation du sel.

On obtient après filtration et séchage 0,4 g du produit désiré.

La RMN¹H est en accord avec la structure chimique.

### Exemple 97

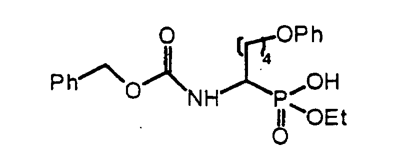

A 1 g (2,22 mmol) de produit de l'exemple 66, on ajoute 0,3 g de NBu₄Br et 8 ml de NaOH 2N. On agite pendant 2 jours à température ambiante.

On dilue avec de l'eau, lave avec de l'Et₂O. La phase aqueuse est acidifiée par HCl N puis H₂SO₄ concentré. On extrait par Et₂O, sèche sur MgSO₄ et on évapore. On obtient 0,41 g de produit désiré.

### Exemple 98

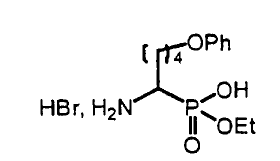

Le produit de l'exemple 97 est déprotégé selon le même mode opératoire que celui décrit à l'exemple 96.

### Exemple 99

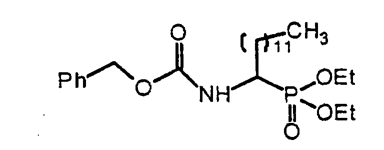

On utilise le même mode opératoire que celui décrit à l'exemple 66 à ceci près que l'aldéhyde de l'exemple 65 est remplacé par du tridécanal.

### Exemple 100

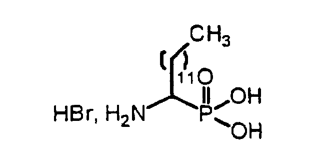

Le produit de l'exemple 99 est déprotégé selon le même mode opératoire que celui décrit à l'exemple 67.

Fusion : 252°C.

La RMN ¹H est en accord avec la structure chimique.

### Exemple 101

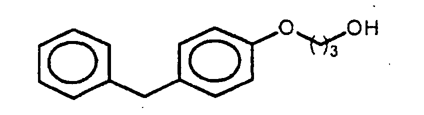

Dans un erienmeyer, on introduit successivement 4,4 g (31,67 mmol) de 3-bromo 1-propanol, 4,9 g (26,5 mmol) de 4-hydroxydiphénylméthane, 11 g (79,59 mmol) de K₂CO₃ en poudre et 45 ml de DMF anhydre.

On agite pendant une nuit à température ambiante.

On filtre et reprend le filtrat avec 30 ml d'acétate d'éthyle. La phase organique est lavée avec de l'eau (2 fois 10 ml) puis avec une solution aqueuse saturée de NaCl (1 fois 10 ml), séchée sur MgSO₄, filtrée et concentrée.

On purifie le résidu par chromatographie éclair sur silice avec le mélange éther éthylique/heptane (50/50). On obtient 5,35 g (22,07 mmol) de produit attendu.

### Exemple 102

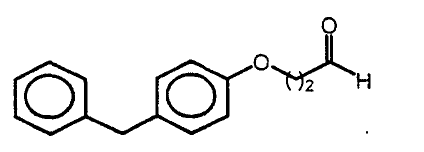

L'alcool de l'exemple 101 est oxydé selon le même mode opératoire que celui décrit à l'exemple 88.

### Exemple 103

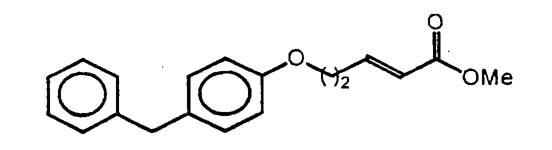

A une solution de 3,56 g (14,8 mmol) d'aldéhyde de l'exemple 102 dans 15 ml de THF anhydre, on ajoute successivement 2,97 g (1,1 équivalents) de triméthyl phosphonoacétate et 0,69 g (1,1 équivalents) de lithine. On agite pendant une nuit à température ambiante sous argon. On ajoute 100 mi d'éther éthylique et on lave la phase organique avec de l'eau (2 fois 10 ml) et avec une solution aqueuse saturée de NaCl (1 fois 10 ml). On sèche la phase organique sur tamis moléculaire, on filtre et on concentre. On purifie par chromatographie sur silice (éluant, Et₂O/heptan 1/9). On obtient 2,27 g (7,66 mmol) de l'ester désiré.

### Exemple 104

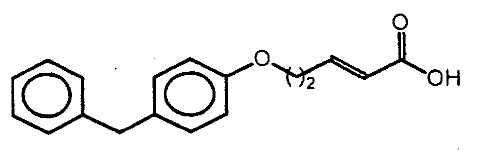

L'ester de l'exemple 103 est saponifié selon le même mode opératoire que celui décrit à l'exemple 90.

### Exemple 105

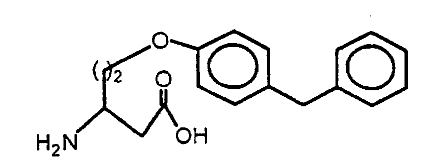

L'acide précédent est traité selon le même mode opératoire que celui décrit à l'exemple 61.

Fusion : 240°C

La RMN ¹H est en accord avec la structure chimique.

### Exemple 106

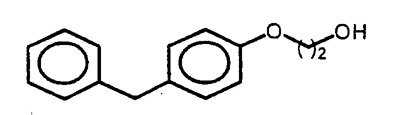

Dans un ballon tricol, on ajoute successivement 10 g (54,28 mmol) de 4-hydroxydiphénylméthane, 1,86 g (0,1 équivalent) de nBu₄NBᵣ, 7,2 g (1,5 équivalents) d'éthylène carbonate et 100 ml de DMF anhydre. On chauffe à 140°C sous argon pendant 4 heures. On revient à température ambiante, on ajoute 100 ml d'éther éthylique et on lave la phase organique avec de l'eau (3 fois 40 ml) puis avec une solution aqueuse saturée de NaCl (1 fois 20 ml). La phase organique est séchée sur tamis moléculaire, filtrée et concentrée. On purifie le résidu par chromatographie éclair sur silice avec un mélange éther-éthylique/heptane (50/50).

On obtient 6,4 g de produit attendu.

### Exemple 107

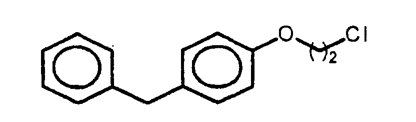

A 3,41 g (15 mmol) d'alcool de l'exemple 106, on ajoute, à une température d'environ +5°C, 2,14 g (1,2 équivalents) de SOCl₂ puis 76 mg (1,1 mmol) d'imidazole. On agite 15 minutes à température ambiante puis 4 heures à 100°C. On revient ensuite à température ambiante, on ajoute 20 ml d'eau, on neutralise la phase aqueuse avec du NaHCO₃ et on extrait avec de l'éther éthylique (2 fois 20 ml). La phase organique est séchée sur tamis moléculaire, filtrée et concentrée. On obtient 3,45 g (13,98 mmol) du dérivé chloré attendu.

### Exemple 108

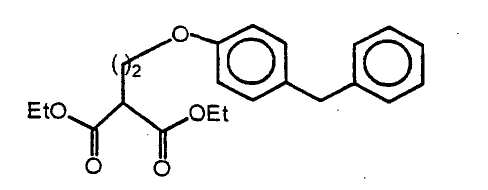

Le diester de l'exemple 108 est préparé selon le même mode opératoire que celui décrit à l'exemple 18, mais en partant du dérivé chloré 107.

### Exemple 109

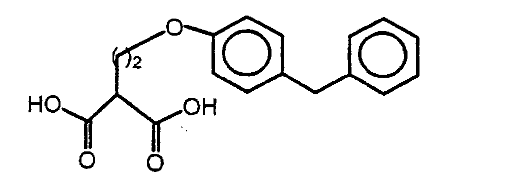

Le diester de l'exemple 108 est saponifié selon le même mode opératoire que celui décrit à l'exemple 59.

### Exemple 110

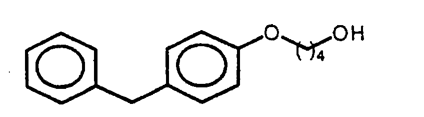

Le diacide de l'exemple 109 est décarboxylé et réduit selon le même mode opératoire que celui décrit à l'exemple 87.

### Exemple 111

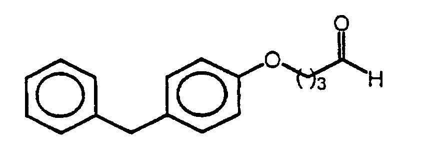

L'alcool de l'exemple 110 est oxydé selon le même mode opératoire que celui décrit à l'exemple 88.

### Exemple 112

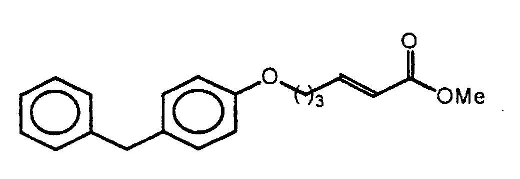

L'aldéhyde de l'exemple 111 est transformé en ester 112 par une réaction de Wittig Horner selon le même mode opératoire que celui décrit à l'exemple 103.

### Exemple 113

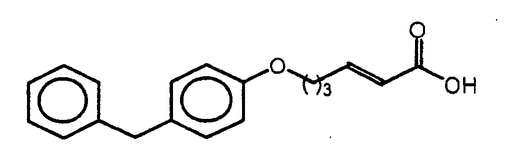

L'ester de l'exemple 112 est saponifié selon le même mode opératoire que celui décrit à l'exemple 90.

### Exemple 114

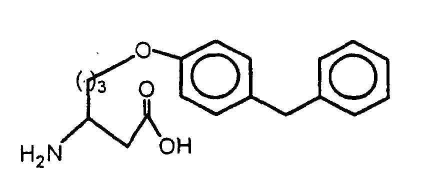

L'acide 113 est traité selon le même mode opératoire que celui décrit à l'exemple 61.

Fusion : 226°C

La RMN ¹H est en accord avec la structure chimique.

### ACTIVITE BIOLOGIQUE

### Essais biologiques des composés selon l'invention

### 1) Inhibition de l'activité aminopeptidase de la LTA₄ hydrolase recombinante

Les composés ont été testés en utilisant la LTA₄ hydrolase humaine recombinante (Minami et coll., FEBS Letters, 1988, 229 : 279). La LTA₄ hydrolase exprimée par E. Coli JM109 est purifiée principalement selon Minami et coll. (J. Biol. Chem., 1987, 262: 13873).

L'inhibition de l'activité aminopeptidase de l'enzyme est mesurée au moyen d'une méthode fluorimétrique en microplaques 96 puits. L'enzyme recombinante (0,5 µg dans 50 µl de Tris-HCl 50 mM pH 7,4) est préincubée 10 minutes à 37°C en présence d'inhibiteur et de dithiothréitol (DTT, 10⁻⁵M). Le substrat Alanyl-amido-méthylcoumarine (Ala-AMC, 25 µM -Tris HCl 50 mM, pH 7,4) est ajouté et l'incubation poursuivie 15 minutes à 37°C. La libération d'AMC est mesurée par fluorimétrie.

Afin d'évaluer la spécificité des composés selon l'invention, certains d'entre eux ont aussi été testés pour leur capacité à inhiber l'activité de l'aminopeptidase M membranaire (EC 3.4.11.2). Le même test est réalisé avec 0,1 µg d'aminopeptidase M (Pierce, USA).

### 2) Inhibition de la biosynthèse de LTB₄ in vitro

La biosynthèse de LTB₄ est mesurée dans le sang total humain en présence d'inhibiteurs de la LTA₄ hydrolase selon l'invention. Un échantillon de 50 µl de sang prélevé sur héparinate de sodium est préincubé 10 minutes à 37°C en présence d'inhibiteur (Tris-HCl 50 mM, NaCl 0,15 M, DTT 10⁻⁵M, pH 7,4).

Le substrat LTA₄ a été préparé extemporanément par hydrolyse alcaline du LTA₄ méthyl ester (Cayman Chemical Co., USA). Après 10 minutes d'incubation en présence de LTA₄ (1 µM dans Tris-HCl 50 mM, NaCl 0,15 M, BSA, 0,5 %, pH 7,4), la réaction est stoppée par dilution au 1/20^{éme} dans du tampon phosphate de potassium 0,1M, NaN₂, 1,5 mM, NaCl 0,4 M, EDTA 1 mM, BSA 0,1 %, pH 7,4 -4°C).

Le LTB₄ est dosé par enzymo-immunodosage (Cayman Chemical Co, USA).

### 3) Inhibition de la biosynthèse de LTB₄ ex vivo

Les composés inhibiteurs de la LTA₄ hydrolase selon l'invention sont mis en suspension dans la méthylcellulose 1,25 % et adminitrés aux souris par voie orale à la dose de 10 mg/kg. Trente minutes après, les souris sont sacrifiées et le sang prélevé sur héparinate de lithium. Le sang est alors comme précédemment incubé 10 minutes à 37°C en présence de LTA₄ puis le LTB₄ formé, dosé par enzymo-immunodosage.

Les composés de l'invention se sont montrés actifs à faible concentration in vitro (par exemple le Ki du composé 51 était de 32 nM) et à faible dose par voie orale (< 1 mg/kg, voire < 0,1 mg/kg).

Les composés selon l'invention, en particulier ceux répondant à l'une des formules (II) et (VI) permettent l'inhibition de la LTA₄ hydrolase in vitro et in vivo. Ils permettent également d'inhiber la biosynthèse de LTB₄, ce qui en fait des composés intéressants en thérapeutique humaine.

Les composés selon l'invention peuvent être administrés notamment par voie orale.

Ils présentent une bonne biodisponibilité ainsi qu'une faible toxicité.

Les composés selon l'invention, en particulier les composés de type aminophosphonate, possèdent une longue durée d'action.

C'est ainsi que ces composés exercent pendant une durée de plus de 24 heures une inhibition totale de l'activité LTA₄ hydrolase sanguine après administration par voie orale à des doses de 1 à 10 mg/kg chez le rat.

## Revendications

1. Composés répondant à la formule (I) suivante : 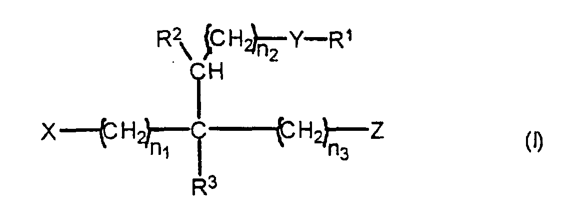 dans laquelle
- X est choisi parmi les groupes suivants :
i)
-NH₂
ii) 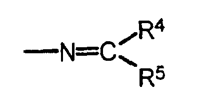
- n₁ et n₃ sont égaux à 0 ou 1, avec (n₁+n₃) égal à 0 ou 1,
- n₂ varie de 0 à 10
- Y est choisi parmi les groupes suivants :
i)
―O―
ii)
―CH₂―
iii)
―S―
iv)
―NH―
v)
―OCH₂―
- R¹ est choisi parmi les groupes suivants :
i) un atome d'hydrogène
ii) un groupe alkyle
iii) un groupe cycloalkyle
iv) un groupe phényle, non substitué, ou mono ou polysubstitué avec des substituants choisis parmi les atomes d'halogène et les groupes CF₃, alkyle contenant de 1 à 10 atomes de carbone, alcoxy contenant de 1 à 10 atomes de carbone, NH₂, NO₂, CN, OH, CO₂H, OPh, OCH₂Ph, SCH₃, SCH₂CH₃ et NHCOR⁶.
v) un groupe α- ou β-naphtyle
vi) un groupe anthracène
vii)
―A²―(CH₂)ₙ₄―A¹
où
n₄ varie de 0 à 4.
A¹ et A² sont indépendamment choisis parmi les groupes suivants :
a) cycloalkyle
b) phényle, non substitué, ou mono ou polysubstitué avec des substituants choisis parmi les atomes d'halogène et les groupes CF₃, alkyle contenant de 1 à 10 atomes de carbone, et alcoxy contenant de 1 à 10 atomes de carbone,
c) 2-, 3- ou 4-pyridyle
d) 2- ou 3-thiényle
e) 2- ou 3-furyle
f) 2-, 3- ou 4-pipéridyle
g) cycloalcène
viii) un groupe 2-, 3- ou 4-pyridyle
ix) un groupe 2- ou 3-thiényle
x) un groupe 2- ou 3-furyle
xi) 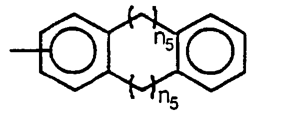
- Z est choisi parmi les groupes suivants :
i)
―COOR⁷
ii) 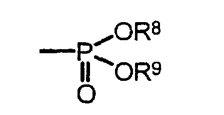
iii) 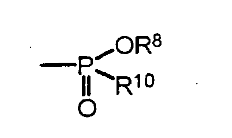
iv) 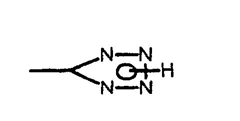
v)
―SO₃H ;
vi)
―SO₂NHR¹¹
vii)
―CONHSO₂R¹¹
- R² et R³ sont indépendamment choisis parmi les groupes suivants :
i) un atome d'hydrogène
ii) un groupe alkyle contenant de 1 à 10 atomes de carbone
iii) un groupe alkyle contenant de 1 à 10 atomes de carbone substitué par un atome d'halogène
iv) un groupe CF₃
v) un atome d'halogène
- R⁴et R⁵ sont indépendamment choisis parmi un atome d'hydrogène, un groupe alkyle contenant de 1 à 10 atomes de carbone, un groupe phényle non substitué ou substitué par un atome d'halogène, un groupe CF₃, un groupe alkyle contenant de 1 à 10 atomes de carbone, un groupe alcoxy contenant de 1 à 10 atomes de carbone et un groupe OH.
- n₅ varie de 0 à 2
- R⁶ représente un groupe alkyle contenant de 1 à 10 atomes de carbone
- R⁷ représente un atome d'hydrogène, un groupe alkyle contenant de 1 à 10 atomes de carbone un groupe ―(CH₂)ₙ₆―Ph, n₆ variant de 0 à 4 et Ph étant un groupe phényle non substitué ou mono ou polysubstitué par un atome d'halogène, un groupe CF₃, un alkyle contenant de 1 à 10 atomes de carbone, un alcoxy contenant de 1 à 10 atomes de carbone ou un groupe OH.
- R⁸ et R⁹ sont indépendamment choisis parmi un atome d'hydrogène, un groupe phényle, un groupe alkyle contenant de 1 à 10 atomes de carbone et un groupe acéthylthioalkylène contenant de 1 à 10 atomes de carbone,
- R¹⁰ représente un groupe alkyle contenant de 1 à 10 atomes de carbone, un groupe ―(CH₂)ₙ₇―Ph, n₇ variant de 1 à 6 et Ph étant un groupe phényle non substitué ou mono ou polysubstitué par un atome d'halogène, un groupe CF₃, un alkyl contenant de 1 à 10 atomes de carbone ou un alcoxy contenant de 1 à 10 atomes de carbone.
- R¹¹ représente un groupe alkyle contenant de 1 à 10 atomes de carbone ou un groupe phényle,
ainsi que leurs isomères, diastéréoisomères et énantiomères et leurs sels thérapeutiquement acceptables,
avec comme conditions que :
(α) si Z est un groupement de type COOR⁷ et n₁=n₃=0 et R²=H et R¹ est un groupe iv) de type phényl non substitué, ou mono ou polysubstitué, alors n₂ ne peut être égal à 1, et
(β) ledit composé n'est pas l'acide α-amino-β-phénoxy-propionique, l'acide 3-amino-7-phényl-heptanoïque, l'acide 3-amino-6-phénoxy-hexanoïque, l'acide α-amino-6-phényl-hexanoïque, ni l'acide α-amino-5-phénoxy-pentanoïque.

2. Composés selon la revendication 1, **caractérisés en ce que** R² et/ou R³ représente un atome d'hydrogène.

3. Composés selon la revendication 2, **caractérisés en ce que** R² et R³ représentent un atome d'hydrogène.

4. Composés selon la revendication 1, **caractérisés en ce que** R² et/ou R³ est différent de l'hydrogène.

5. Composés selon l'une des revendications 1 à 4, **caractérisés en ce que** n₁ et n₃ sont égaux à 0.

6. Composés selon l'une des revendications 1 à 4, **caractérisés en ce que** n₁ ou n₃ est différent de 0.

7. Composés selon l'une des revendications 1 à 6, **caractérisés en ce que** X représente NH₂.

8. Composés selon l'une des revendications 1 à 7, **caractérisés en ce que** X représente NH₂ et/ou Z représente COOH.

9. Composés selon l'une des revendications 1 à 8, **caractérisés en ce qu'**ils répondent à la formule (II) suivante : 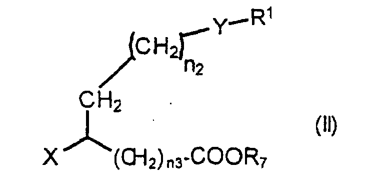 X, n₂, n₃, Y, R¹ et R⁷ ont la signification donnée à la revendication 1.

10. Composés selon l'une des revendications 1 à 9, **caractérisés en ce qu'**ils répondent à la formule (III) suivante : 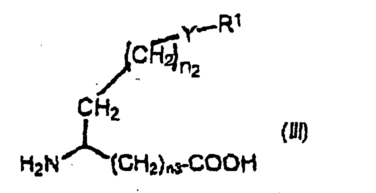 Y, n₂, n₃, et R¹ ont la signification indiquée à la revendication 1.

11. Composés selon l'une des revendications 1 à 10, **caractérisés en ce qu'**ils répondent à la formule (IV) suivante : 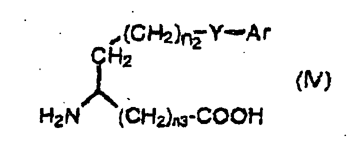 où Y, n₂ et n₃ ont la signification donnée précédemment et Ar symbolise le groupe R¹ représentant un groupe phényle iv) éventuellement substitué tel que défini à la revendication 1 ou R¹ représentant un groupe vii) -A²-(CH₂)ₙ₄-A¹, A² étant un groupe phényle (b) éventuellement substitué tel que défini à la revendication 1.

12. Composés selon l'une des revendications 1 à 7, **caractérisés en ce que** X représente NH₂ et/ou Z représente 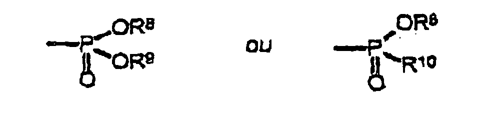 R⁸, R⁹ étant un atome d'hydrogène et R¹⁰ ayant la signification donnée à la revendication 1.

13. Composés selon la revendication 14, **caractérisés en ce que** X représente NH₂ et Z représente -PO(OH)₂.

14. Composés selon l'une des revendications 1 à 7, 12 ou 13, **caractérisés en ce qu'**ils répondent à la formule (V) suivante : 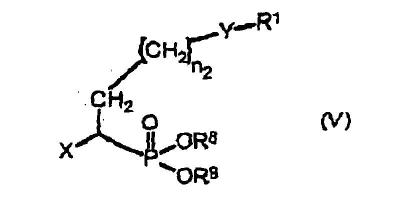 X, n₂, Y, R¹, R⁸ et R⁹ ont la signification donnée à la revendication 1.

15. Composés selon l'une des revendications 1 à 7 et 12 à 14,
**caractérisés en ce qu'**ils répondent à la formule (VI) suivante : 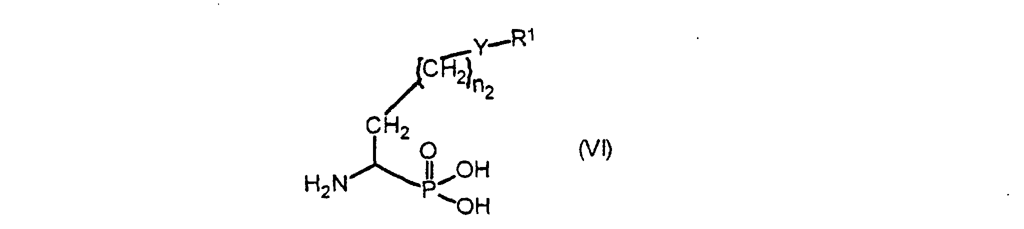 Y, n₂ et R¹ ont la signification indiquée à la revendication 1.

16. Composés selon l'une des revendications 1 à 7 et 12 à 15, **caractérisés en ce qu'**ils répondent à la formule (VII) suivante : 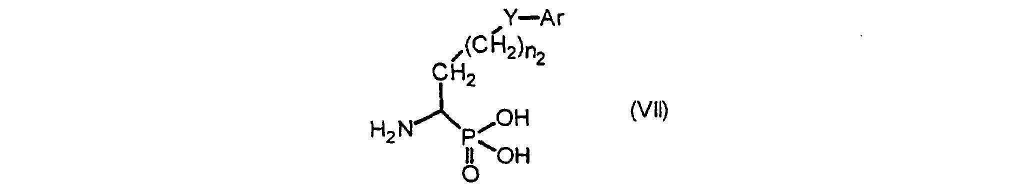 où Y et n₂ sont tels que définis précédemment, et Ar symbolise R¹ représentant un groupe phényle iv) éventuellement substitué tel que défini à la revendication 1 ou R¹ représentant un groupe vii) -A²-(CH₂)ₙ₄-A¹, A² étant un groupe phényle (b) éventuellement substitué tel que défini en revendication 1.

17. Composés selon l'une des revendications 1 à 7, **caractérisés en ce qu'**ils répondent à la formule (VIII) suivante : 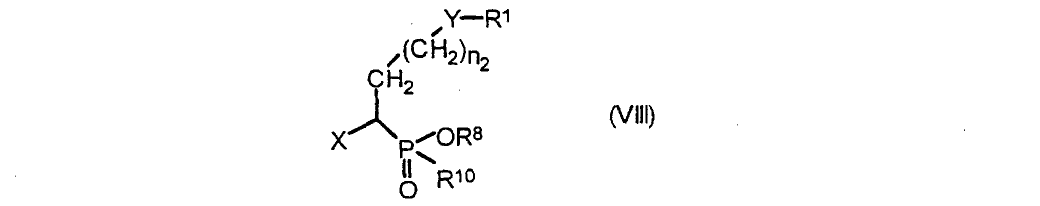 où X, Y, n₂, R¹, R⁸ et R¹⁰ ont la signification indiquée à la revendication 1.

18. Composés selon l'une des revendications 1 à 17, **caractérisés en ce que** n₂ varie de 2 à 5.

19. Composés selon la revendication 18, **caractérisés en ce que** n₂ est égal à 3.

20. Composés selon l'une des revendications 1 à 19, caracténsés en ce que Y représente un atome d'oxygène.

21. Composés selon l'une des revendications 1 à 19, **caractérisés en ce que** Y représente ―CH₂―.

22. Composés selon l'une des revendicadans 1 à 21, **caractérisés en ce que** R¹ représente un groupe phényle non suastitué.

23. Composés selon l'une des revendications 1 à 10 et 18 à 21 et 31, **caractérisés en ce que** R¹ représente un groupe phényle mono- ou polysubstitué par un groupe choisi parmi les groupes alkyle contenant de 1 à 10 atomes de carbone, alcoxy contenant de 1 à 10 atomes de carbone OPh et OCH₂Ph, de préférence OPh.

24. Composés selon l'une des revendications 1 à 21, **caractérisés en ce que** R¹ représente un motif -A²-(CH₂)ₙ₄-A¹.

25. Composés selon la revendication 24, **caractérisés en ce que** A² représente un groupe phényle, de préférence non substitué.

26. Composés selon l'une des revendications 24 et 25. **caractérisés en ce que** n₄ est égal à 0 ou 1.

27. Composés selon l'une des revendications 1 à 13, 21 à 29 et 24 à 26, **caractérisés en ce que** A¹ est un groupe phényle, cycloalkyle ou cycloalcène.

28. Composés selon l'une des revendications 24 à 27, **caractérisés en ce que** R¹ représente un groupe phényle substitué par un groupe Ph, CH₂Ph, CH₂-cycloalkyle ou CH₂-cycloalcène, de préférence CH₂Ph ou CH₂-cycloalkyle.

29. Composés selon l'une des revendications 24 à 26, **caractérisés en ce que** A¹ est un groupe phényle.

30. Composés selon l'une des revendications 24 à 26 et 29, **caractérisés en ce que** R¹ représente un groupe phényle substitué par un groupe Ph ou CH₂Ph, de préférence CH₂Ph.

31. Composés selon l'une des revendications 1 à 21, **caractérisés en ce que** R¹ représente un atome d'hydrogène ou un groupe alkyle contenant de 1 à 10 atomes de carbone.

32. Composés selon l'une des revendications 1 à 21,
**caractérisés en ce que** R¹ représente 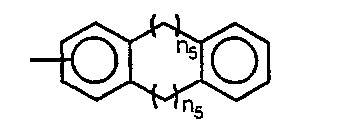 n₅ étant tel que défini à la revendication 1.

33. Composés selon l'une des revendications 1 à 11 et 18 à 32, **caractérisés en ce qu'**ils sont choisis parmi :
1) Chlorhydrate de la -(S)-O-4-benzyl phénoxy sérine
2) Bromhydrate de l'acide 2-(RS)-amino-6-(4 benzyl phénoxy) hexanoique
3) Bromhydrate de l'acide 2-(RS)-amino-5-(4 benzyl phénoxy) pentanoique
4) Bromhydrate de l'acide 2-(RS)-amino-5-(4-phénoxy-phénoxy) pentanoique
5) Bromhydrate de l'acide 2-(RS)-amino-7-(4-benzyl-phénoxy) heptanoique
6) Bromhydrate de l'acide 2-(RS)-amino-6-(4-phényl-phénoxy)-hexanoïque
7) Bromhydrate de l'acide 2-(RS)-amino-6-(4-hexyloxy-phénoxy)-hexanoïque
8) Bromhydrate de l'acide 2-(RS)-amino-8-(4-benzyl-phénoxy)-octanoïque
9) Bromhydrate de l'acide 2-(RS)-amino-6-(4-phénoxy-phénoxy)-hexanoïque
10) Acide 2-(RS)-aminométhyl-6-(4-benzyl-phénoxy)-hexanoïque
16) Bromhydrate de l'acide 2-(RS)-amino-6-(4 cyclohexylméthyl-phénoxy)-hexanoïque
17) Acide 3-(RS)-amino-7-(4-benzyl-phénoxy)-heptanoïque
18) Bromhydrate de l'acide 2-(RS)-amino-2-méthyl-6(4-benzyl-phénoxy)hexanoïque.
20) Acide 3-(RS)-amino-5-(4-benzyl-phénoxy)-pentanoïque
21) Acide 3-(RS)-amino-6-(4-benzyl-phénoxy)-hexanoïque

34. Composés selon l'une des revendications 1 à 7, 12 à 32, **caractérisés en ce qu'**ils sont choisis par :
11) Bromhydrate de l'acide 1-(RS)-amino-5-(phénoxy)-pentyl-phosphonique
12) Bromhydrate de l'acide 1-(RS)-amino-6-(phénoxy)-hexyl-phosphonique
13) Bromhydrate de l'acide 1-(RS)-amino-5-(4-benzyl-phénoxy)-pentyl-phosphonique
14) Bromhydrate de l'acide 1-(RS)-amino-4-(phénoxy)-butyl-phosphonique
15) Bromhydrate de l'acide 1-(RS)-amino-7-(phénoxy)-heptyl-phosphonique.
19) Bromhydrate de l'acide 1-(RS)-amino-tridécanyl phosphonique.

35. Composés selon l'une des revendications 1 à 6, à 10, 12, 14, 17 à 32, **caractérisés en ce que** X représente 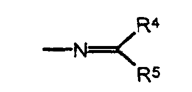 R⁴ et R⁵ ayant la signification donnée à la revendication 1.

36. Composés selon l'une des revendications 1 à 7, 17 à 32 et 35, **caractérisés en ce que** R⁷ est différent de l'hydrogène.

37. Composés selon l'une quelconque des revendications 1 à 7, 12, 14, 17 à 32 ou 35, **caractérisés en ce que** R⁸ et/ou R⁹ sont indépendants et différents de l'hydrogène.

38. Composés selon l'une quelconque des revendications 1 à 7, 17 à 32 ou 35, **caractérisés en ce que** R⁸ et/ou R¹⁰ sont indépendants et R⁸ différent de l'hydrogène.

39. Composés selon l'une quelconque des revendications 35 à 38, caractérisés en qu'ils sont choisis parmi
22) chlorhydrate du 2-(RS)-amino-7-(4 benzyl phénoxy) heptanoate d'éthyle
23) chlorhydrate du 2-(RS)-amino-6-(4-benzyl-phénoxy) hexanoate d'éthyle
24) bromhydrate du diphényl 1-amino-5-phénoxy-pentyl-phosphonate
25) bromhydrate de l'éthyl-hydrogène-1-amino-5 phénoxy-pentyl-phosphonate.

40. Composition pharmaceutique **caractérisée en ce qu'**elle comprend à titre de principe actif, une quantité thérapeutiquement efficace d'un composé selon l'une des revendications 1 à 39, en combinaison avec un véhicule ou excipient physiologiquement acceptable.

41. Médicament agissant comme inhibiteur de l'activité de la LTA₄ hydrolase, **caractérisé en ce qu'**il comprend, à titre de principe actif, un composé selon l'une des revendications 1 à 39.

42. Médicament destiné à un traitement anti-inflammatoire, **caractérisé en ce qu'**il comprend, à titre de principe actif, un composé selon l'une des revendications 1 à 39.

43. Médicament destiné à un traitement anti-arthritique, **caractérisé en ce qu'**il comprend, à titre de principe actif, un composé selon l'une des revendications 1 à 39.

44. Médicament destiné à un traitement anti-psoriasique, **caractérisé en ce qu'**il comprend, à titre de principe actif, un composé selon l'une des revendications 1 à 39.

45. Médicament destiné à un traitement hépato-protecteur, **caractérisé en ce qu'**il comprend, à titre de principe actif, un composé selon l'une des revendications 1 à 39.

46. Médicament destiné à un traitement antimitotique, **caractérisé en ce qu'**il comprend, à titre de principe actif, un composé selon l'une des revendications 1 à 39.

47. Médicament destiné au traitement d'une surproduction de LTB₄, induite notamment par des inhibiteurs de cyclooxygénase, **caractérisé en ce qu'**il comprend, à titre de principe actif, un composé selon l'une des revendications 1 à 39.

48. Utilisation d'un composé selon l'une des revendications 1 à 39, d'un composé de formule (1) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné à inhiber l'activité de la LTA₄ hydrolase.

49. Utilisation d'un composé selon l'une des revendications 1 à 39, d'un composé de formule (1) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné à un traitement anti-inflammatoire.

50. Utilisation d'un composé selon l'une des revendications 1 à 39, d'un composé de formule (1) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné à un traitement anti-arthritique.

51. Utilisation d'un composé selon l'une des revendications 1 à 39, d'un composé de formule (I) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné à un traitement anti-psoriasique.

52. Utilisation d'un composé selon l'une des revendications 1 à 39, d'un composé de formule (I) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné à un traitement hépato-protecteur.

53. Utilisation d'un composé selon l'une des revendications 1 à 39, d'un composé de formule (I) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné à un traitement antimitotique.

54. Utilisation d'un composé selon l'une des revendications 1 à 39, d'un composé de formule (I) où Z est un groupement de type COOR⁷, n₁=n₃=O, R²=H, R¹ est un groupe iv) de type phényl non substitué ou mono ou polysubstitué et n₂=1, de l'acide α-amino-β-phénoxy-propionique, de l'acide 3-amino-7-phényl-heptanoïque, de l'acide 3-amino-6-phénoxy-hexanoïque, de l'acide α-amino-6-phényl-hexanoïque, ou de l'acide α-amino-5-phénoxy-pentanoïque, pour la fabrication d'un médicament destiné au traitement d'une surproduction de LTB₄ induite notamment par un inhibiteur de cyclooxygénase.

55. Composition pharmaceutique, **caractérisée en ce qu'**elle comprend, à titre de principe actif, une quantité thérapeutiquement efficace d'un composé selon l'une quelconque des revendications 1 à 52 et une quantité thérapeutiquement efficace d'un inhibiteur de cyclooxygénase, éventuellement en combinaison avec un véhicule ou excipient physiologiquement acceptable.

## Patentansprüche

1. Verbindungen der folgenden Formel (I): 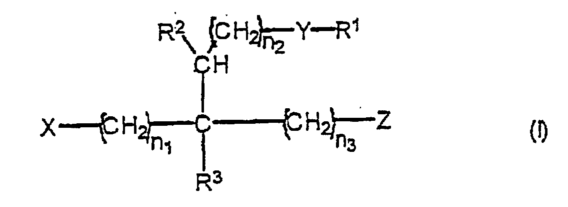 in der
- X ausgewählt ist aus den folgenden Gruppen:
i)
-NH₂
ii) 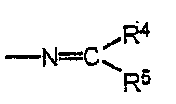
- n₁ und n₃ 0 oder 1 bedeuten, mit der Maßgabe, daß (n₁+n₃) 0 oder 1 ist,
- n₂ einen Wert von 0 bis 10 aufweist,
- Y ausgewählt ist aus den folgenden Gruppen:
i)
-O-
ii)
-CH₂-
iii)
-S-
iv)
-NH-
v)
-OCH₂-
- R¹ ausgewählt ist aus den folgenden Gruppen:
i) dem Wasserstoffatom
ii) Alkylgruppen
iii) Cycloalkylgruppen
iv) Phenylgruppen, die nichtsubstituiert sind oder einfach oder mehrfach substituiert sind mit Substituenten ausgewählt aus Halogenatomen, CF₃-Gruppen, Alkylgruppen, die 1 bis 10 Kohlenstoffatome enthalten, und Alkoxygruppen, die 1 bis 10 Kohlenstoffatome enthalten, Gruppen der Formeln NH₂, NO₂, CN, OH, CO₂H, OPh, OCH₂Ph, SCH₃, SCH₂CH₃ und NHCOR⁶,
v) α- oder β-Naphthylgruppen,
vi) Antracengruppen,
vii) Gruppen der Formel -A²-(CH₂)ₙ₄-A¹, in der
n₄ einen Wert von 0 bis 4 aufweist,
A¹ und A² unabhängig voneinander ausgewählt sind aus den folgenden Gruppen:
a) Cycloalkylgruppen
b) Phenylgruppen, die nicht substituiert sind oder einfach oder mehrfach substituiert sind durch Substituenten ausgewählt aus Halogenatomen, CF₃-Gruppen und Alkylgruppen, die 1 bis 10 Kohlenstoffatome enthalten, und Alkoxygruppen, die 1 bis 10 Kohlenstoffatome enthalten,
c) 2-, 3- oder 4-Pyridyl
d) 2- oder 3-Thienyl
e) 2- oder 3-Furyl
f) 2-, 3- oder 4-Piperidyl
g) Cycloalken
viii) 2-, 3- oder 4-Pyridylgruppen
ix) 2- oder 3-Thienylgruppen
x) 2- oder 3-Furylgruppen
xi) 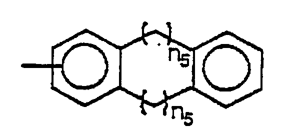
- Z ausgewählt ist aus den folgenden Gruppen:
i)
-COOR⁷
ii) 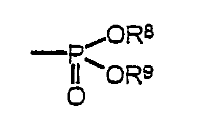
iii) 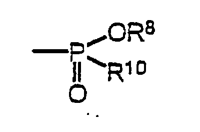
iv) 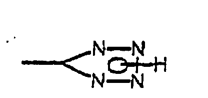
v)
-SO₃H;
vi)
-SO₂NHR¹¹
vii)
-CONHSO₂R¹¹
- R² und R³ unabhängig voneinander ausgewählt sind aus den foglenden Gruppen:
i) Wasserstoffatomen
ii) Alkylgruppen, die 1 bis 10 Kohlenstoffatome enthalten
iii) Alkylgruppen, die 1 bis 10 Kohlenstoffatome enthalten und durch ein Halogenatom substituiert sind
iv) CF₃-Gruppen
v) Halogenatomen
- R⁴ und R⁵ unabhängig voneinander ausgewählt sind aus Wasserstoffatomen, Alkylgruppen, die 1 bis 10 Kohlenstoffatome enthalten, Phenylgruppen, die nicht substituiert sind oder durch ein Halogenatom, eine CF₃-Gruppe, eine Alkylgruppe, die 1 bis 10 Kohlenstoffatome enthält, eine Alkoxygruppe, die 1 bis 10 Kohlenstoffatome enthält, oder eine OH-Gruppe substituiert sind,
- n₅ einen Wert von 0 bis 2 aufweist,
- R⁶ eine Alkylgruppe bedeutet, die 1 bis 10 Kohlenstoffatome enthält
- R⁷ ein Wasserstoffatom, eine Alkylgruppe, die 1 bis 10 Kohlenstoffatome enthält, eine Gruppe -(CH₂)ₙ₆-Ph bedeutet, worin n₆ einen Wert von 0 bis 4 aufweist und Ph eine Phenylgruppe bedeutet, die nicht substituiert ist oder einfach oder mehrfach substituiert ist durch ein Halogenatom, eine Gruppe CF₃, eine Alkylgruppe, die 1 bis 10 Kohlenstoffatome enthält, eine Alkoxygruppe, die 1 bis 10 Kohlenstoffatome enthält, oder eine OH-Gruppe,
- R⁸ und R⁹ unabhängig voneinander ausgewählt sind aus Wasserstoffatomen, Phenylgruppen, Alkylgruppen, die 1 bis 10 Kohlenstoffatome enthalten, und Acetylthioalkylengruppen, die 1 bis 10 Kohlenstoffatome enthalten,
- R¹⁰ eine Alkylgruppe, die 1 bis 10 Kohlenstoffatome enthält, eine Gruppe -(CH₂)ₙ₇-Ph bedeutet, worin n₇ einen Wert von 1 bis 6 aufweist und Ph eine Phenylgruppe darstellt, die nicht substituiert ist oder einfach oder mehrfach substituiert ist durch ein Halogenatom, eine CF₃-Gruppe, eine Alkylgruppe, die 1 bis 10 Kohlenstoffatome aufweist, oder eine Alkoxygruppe, die 1 bis 10 Kohlenstoffatome aufweist,
- R¹¹ eine Alkylgruppe, die 1 bis 10 Kohlenstoffatome enthält, oder eine Phenylgruppe bedeutet,
sowie deren Isomere, Diastereoisomere und Enantiomere sowie ihre therapeutisch annehmbaren Salze,
mit der Maßgabe, daß, wenn:
(α) wenn Z eine Gruppe des Typs COOR⁷ darstellt und n₁ = n₃ = 0 und R² = H bedeuten und R¹ eine nichtsubstituierte oder einfach oder mehrfach substituierte Phenylgruppe des Typs iv) darstellt, dann n₂ nicht den Wert 1 besitzt, und
(β) die Verbindung nicht α-Amino-β-phenoxy-propionsäure, 3-Amino-7-phenylheptansäure, 3-Amino-6-phenoxy-hexansäure, α-Amino-6-phenyl-hexansäure noch α-Amino-5-phenoxy-pentansäure ist.

2. Verbindungen nach Anspruch 1, **dadurch gekennzeichnet, daß** R² und/oder R³ Wasserstoffatome bedeuten.

3. Verbindungen nach Anspruch 2, **dadurch gekennzeichnet, daß** R² und R³ Wasserstoffatome bedeuten.

4. Verbindungen nach Anspruch 1, **dadurch gekennzeichnet, daß** R² und/oder R³ von Wasserstoff verschieden sind.

5. Verbindungen nach einem der Ansprüche 1 bis 4, **dadurch gekennzeichnet, daß** n₁ und n₃ den Wert 0 besitzen.

6. Verbindungen nach einem der Ansprüche 1 bis 4, **dadurch gekennzeichnet, daß** n₁ oder n₃ von 0 verschieden ist.

7. Verbindungen nach einem der Ansprüche 1 bis 6, **dadurch gekennzeichnet, daß** X NH₂ bedeutet.

8. Verbindungen nach einem der Ansprüche 1 bis 7, **dadurch gekennzeichnet, daß** X NH₂ und/oder Z COOH bedeuten.

9. Verbindungen nach einem der Ansprüche 1 bis 8, **dadurch gekennzeichnet, daß** sie der folgenden Formel (II) entsprechen: 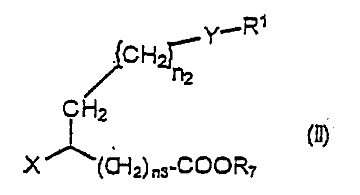 in der X, n₂, n₃, Y, R¹ und R⁷ die in Anspruch 1 angegebenen Bedeutungen besitzen.

10. Verbindungen nach einem der Ansprüche 1 bis 9, **dadurch gekennzeichnet, daß** sie der foglenden Formel (III) entsprechen: 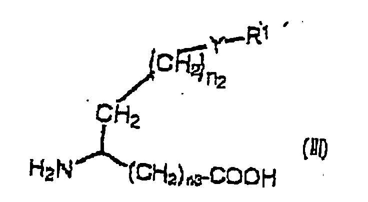 in der Y, n₂, n₃ und R¹ die in Anspruch 1 angegebenen Bedeutungen besitzen.

11. Verbindungen nach einem der Ansprüche 1 bis 10, **dadurch gekennzeichnet, daß** sie der folgenden Formel (IV) entsprechen: 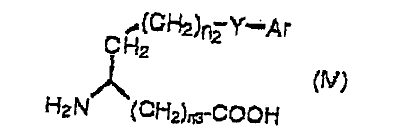 in der Y, n₂ und n₃ die oben angegebenen Bedeutungen besitzen und Ar für eine Gruppe R¹ steht, die eine gegebenenfalls substituierte Phenylgruppe iv), wie sie in Anspruch 1 definiert ist, bedeutet, oder R¹ eine Gruppe vii) -A²-(CH₂)ₙ₄-A¹ bedeutet, in der A² eine gegebenenfalls substituierte Phenylgruppe (b) darstellt, wie sie in Anspruch 1 definiert ist.

12. Verbindungen nach einem der Ansprüche 1 bis 7, **dadurch gekennzeichnet, daß** X NH₂ und/oder Z 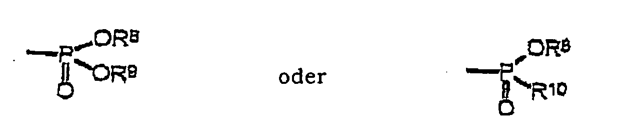 bedeuten, worin R⁸ und R⁹ Wasserstoffatome darstellen und R¹⁰ die in Anspruch 1 angegebenen Bedeutungen besitzt.

13. Verbindungen nach Anspruch 12, **dadurch gekennzeichnet, daß** X NH₂ und Z -PO(OH)₂ bedeuten.

14. Verbindungen nach einem der Ansprüche 1 bis 7, 12 oder 13, **dadurch gekennzeichnet, daß** sie der folgenden Formel (V) entsprechen: 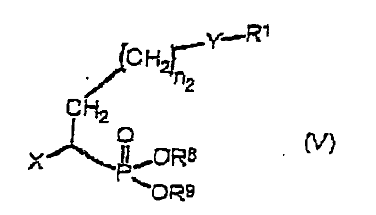 in der X, n₂, Y, R¹, R⁸ und R⁹ die in Anspruch 1 angegebenen Bedeutungen besitzen.

15. Verbindungen nach einem der Ansprüche 1 bis 7 und 12 bis 14, **dadurch gekennzeichnet, daß** sie der folgenden Formel (VI) entsprechen: 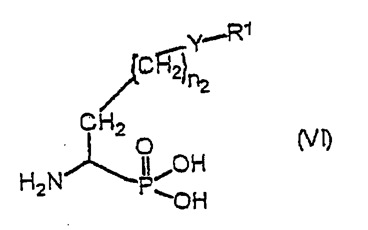 in Y, n₂ und R¹ die in Anspruch 1 angegebenen Bedeutungen besitzen.

16. Verbindungen nach einem der Ansprüche 1 bis 7 und 12 bis 15, **dadurch gekennzeichnet, daß** sie der folgenden Formel (VII) entsprechen: 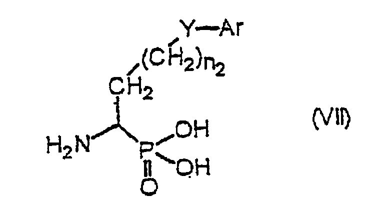 in der Y und n₂ die oben angegebenen Bedeutungen besitzen und Ar für R¹ steht, die eine gegebenenfalls substituierte Phenylgruppe iv) bedeutet, wie sie in Anspruch 1 definiert ist, oder R¹ eine Gruppe vii) -A²-(CH₂)ₙ₄-A¹ bedeutet, in der A² eine gegebenenfalls substituierte Phenylgruppe (b) darstellt, wie sie in Anspruch 1 definiert ist.

17. Verbindungen nach einem der Ansprüche 1 bis 7, **dadurch gekennzeichnet, daß** sie der folgenden Formel (VIII) entsprechen: 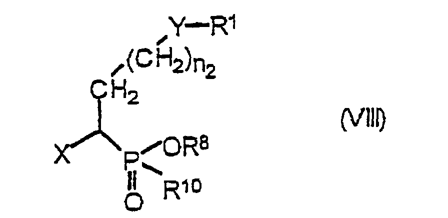 in der X, Y, n₂, R¹, R⁸ und R¹⁰ die in Anspruch 1 angegebenen Bedeutungen besitzen.

18. Verbindungen nach einem der Ansprüche 1 bis 17, **dadurch gekennzeichnet, daß** n₂ einen Wert von 2 bis 5 besitzt.

19. Verbindungen nach Anspruch 18, **dadurch gekennzeichnet, daß** n₂ einen Wert von 3 besitzt.

20. Verbindungen nach einem der Ansprüche 1 bis 19, **dadurch gekennzeichnet, daß** Y ein Sauerstoffatom bedeutet.

21. Verbindungen nach einem der Ansprüche 1 bis 19, **dadurch gekennzeichnet, daß** Y -CH₂- bedeutet.

22. Verbindungen nach einem der Ansprüche 1 bis 21, **dadurch gekennzeichnet, daß** R¹ eine nichtsubstituierte Phenylgruppe bedeutet.

23. Verbindungen nach einem der Ansprüche 1 bis 10 und 18 bis 21 und 31, **dadurch gekennzeichnet, daß** R¹ eine Phenylgruppe darstellt, die einfach oder mehrfach substituiert ist durch eine Gruppe ausgewählt aus Alkylgruppen, die 1 bis 10 Kohlenstoffatome enthalten, Alkoxygruppen, die 1 bis 10 Kohlenstoffatome enthalten, Gruppen OPh und OCH₂Ph, vorzugsweise OPh.

24. Verbindungen nach einem der Ansprüche 1 bis 21, **dadurch gekennzeichnet, daß** R¹ einen Rest -A²-(CH₂)ₙ₄-A¹ bedeutet.

25. Verbindungen nach Anspruch 24, **dadurch gekennzeichnet, daß** A² eine vorzugsweise nichtsubstituierte Phenylgruppe darstellt.

26. Verbindungen nach einem der Ansprüche 24 und 25, **dadurch gekennzeichnet, daß** n₄ einen Wert von 0 oder 1 besitzt.

27. Verbindungen nach einem der Ansprüche 1 bis 13. 21 bis 29 und 24 bis 26, **dadurch gekennzeichnet, daß** A¹ eine Phenylgruppe, Cycloalkylgruppe oder Cycloalkengruppe darstellt.

28. Verbindungen nach einem der Ansprüche 24 bis 27, **dadurch gekennzeichnet, daß** R¹ eine Phenylgruppe darstellt, die durch eine Gruppe Ph, CH₂Ph, CH₂-Cycloalkyl oder CH₂-Cycloalken, vorzugsweise CH₂Ph oder CH₂-Cycloalkyl substituiert ist.

29. Verbindungen nach einem der Ansprüche 24 bis 26, **dadurch gekennzeichnet, daß** A¹ eine Phenylgruppe darstellt.

30. Verbindungen nach einem der Ansprüche 24 bis 26 und 29, **dadurch gekennzeichnet, daß** R¹ eine Phenylgruppe darstellt, die durch eine Gruppe Ph oder CH₂Ph, vorzugsweise CH₂Ph, substituiert ist.

31. Verbindungen nach einem der Ansprüche 1 bis 21, **dadurch gekennzeichnet, daß** R¹ ein Wasserstoffatom oder eine Alkylgruppe, die 1 bis 10 Kohlenstoffatome enthält, bedeutet.

32. Verbindungen nach einem der Ansprüche 1 bis 21, **dadurch gekennzeichnet, daß** R¹ 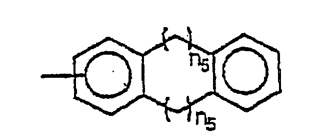 bedeutet, worin n₅ die in Anspruch 1 angegebenen Bedeutungen besitzt.

33. Verbindungen nach einem der Ansprüche 1 bis 11 und 18 bis 32, **dadurch gekennzeichnet, daß** sie ausgewählt sind aus:
1) -(S)-O-4-Benzyl-phenoxy-serin-Hydrochlorid
2) 2-(RS)-Amino-6-(4-benzyl-phenoxy)-hexansäure-Hydrobromid
3) 2-(RS)-Amino-5-(4-benzyl-phenoxy)-pentansäure-Hydrobromid
4) 2-(RS)-Amino-5-(4-phenoxy-phenoxy)-pentansäure-Hydrobromid
5) 2-(RS)-Amino-7-(4-benzyl-phenoxy)-heptansäure-Hydrobromid
6) 2-(RS)-Amino-6-(4-phenyl-phenoxy)-hexansäure-Hydrobromid
7) 2-(RS)-Amino-6-(4-hexyloxy-phenoxy)-hexansäure-Hydrobromid
8) 2-(RS)-Amino-8-(4-benzyl-phenoxy)-octansäure-Hydrobromid
9) 2-(RS)-Amino-6-(4-phenoxy-phenoxy)-hexansäure-Hydrobromid
10) 2-(RS)-Aminomethyl-6-(4-benzyl-phenoxy)-hexansäure
16) 2-(RS)-Amino-6-(4-cyclohexylmethyl-phenoxy)-hexansäure-Hydrobromid
17) 3-(RS)-Amino-7-(4-benzyl-phenoxy)-heptansäure
18) 2-(RS)-Amino-2-methyl-6-(4-benzyl-phenoxy)-hexansäure-Hydrobromid
20) 3-(RS)-Amino-5-(4-benzyl-phenoxy)-pentansäure
21) 3-(RS)-Amino-6-(4-benzyl-phenoxy)-hexansäure.

34. Verbindungen nach einem der Ansprüche 1 bis 7 und 12 bis 32, **dadurch gekennzeichnet, daß** sie ausgewählt sind aus:
11) 1-(RS)-Amino-5-(phenoxy)-pentyl-phosphonsäure-Hydrobromid
12) 1-(RS)-Amino-6-(phenoxy)-hexyl-phosphonsäure-Hydrobromid
13) 1-(RS)-Amino-5-(4-benzyl-phenoxy)-pentyl-phosphonsäure-Hydrobromid
14) 1-(RS)-Amino-4-(phenoxy)-butyl-phosphonsäure-Hydrobromid
15) 1-(RS)-Amino-7-(phenoxy)-heptyl-phosphonsäure-Hydrobromid
19) 1-(RS)-Amino-tridecanyl-phosphonsäure-Hydrobromid.

35. Verbindungen nach einem der Ansprüche 1 bis 6, 10, 12, 14 und 17 bis 32, **dadurch gekennzeichnet, daß** X 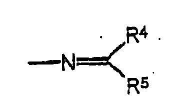 bedeutet, worin R⁴ und R⁵ die in Anspruch 1 angegebenen Bedeutungen besitzen.

36. Verbindungen nach einem der Ansprüche 1 bis 7, 17 bis 32 und 35, **dadurch gekennzeichnet, daß** R⁷ von Wasserstoff verschieden ist.

37. Verbindungen nach einem der Ansprüche 1 bis 7, 12, 14, 17 bis 32 und 35, **dadurch gekennzeichnet, daß** R⁸ und/oder R⁹ unabhängig voneinander und von Wasserstoff verschieden sind.

38. Verbindungen nach einem der Ansprüche 1 bis 7, 17 bis 32 und 35, **dadurch gekennzeichnet, daß** R⁸ und/oder R^{1β} unabhängig voneinander sind und R⁸ von Wasserstoff verschieden ist.

39. Verbindungen nach einem der Ansprüche 35 bis 38, **dadurch gekennzeichnet, daß** sie ausgewählt sind aus:
22) 2-(RS)-Amino-7-(4-benzyl-phenoxy)-heptansäureethylester-Hydrochlorid
23) 2-(RS)-Amino-6-[4-benzyl-phenoxy)-hexanosäureethylester-Hydrochlorid
24) Diphenyl-1-amino-5-phenoxy-pentyl-phosphonat-Hydrobromid
25) Ethyl-hydrogen-1-amino-5-phenoxy-pentyl-phosphonat-Hydrobromid.

40. Pharmazeutische Zubereitung, **dadurch gekennzeichnet, daß** sie als Wirkstoff eine therapeutisch wirksame Menge einer Verbindung nach einem der Ansprüche 1 bis 39 in Kombination mit einem physiologisch annehmbaren Trägermaterial oder Bindemittel umfaßt.

41. Arzneimittel wirkend als Inhibitor der LTA₄-Hydrolase-Aktivität, **dadurch gekennzeichnet, daß** es als Wirkstoff eine Verbindung nach einem der Ansprüche 1 bis 39 umfaßt.

42. Arzneimittel zur anti-inflammatorischen Behandlung, **dadurch gekennzeichnet, daß** es als Wirkstoff eine Verbindung nach einem der Ansprüche 1 bis 39 umfaßt.

43. Arzneimittel zur Anti-Arthritis-Behandlung, **dadurch gekennzeichnet, daß** es als Wirkstoff eine Verbindung nach einem der Ansprüche 1 bis 39 umfaßt.

44. Arzneimittel zur Anti-Psoriasis-Behandlung, **dadurch gekennzeichnet, daß** es als Wirkstoff eine Verbindung nach einem der Ansprüche 1 bis 39 umfaßt.

45. Arzneimittel zur Leberschutz-Behandlung, **dadurch gekennzeichnet, daß** es als Wirkstoff eine Verbindung nach einem der Ansprüche 1 bis 39 umfaßt.

46. Arzneimittel zur antimitotischen Behandlung, **dadurch gekennzeichnet, daß** es als Wirkstoff eine Verbindung nach einem der Ansprüche 1 bis 39 umfaßt.

47. Arzneimittel zur Behandlung der LTB₄-Überproduktion, die normalerweise durch Cyclooxyenase-Inhibitoren ausgelöst wird, **dadurch gekennzeichnet, daß** es als Wirkstoff eine Verbindung nach einem der Ansprüche 1 bis 39 umfaßt.

48. Verwendung einer Verbindung nach einem der Ansprüche 1 bis 39 einer Verbindung der Formel (I), in der Z eine Gruppe des Typs COOR⁷ bedeutet, n₁ = n₃ = 0, R² = H, R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur Inhibierng der LTA₄-Hydrolase-Aktivität.

49. Verwendung einer Verbindung nach einem der Ansprüche 1 bis 39 einer Verbindung der Formel (I), in der Z eine Gruppe des Typs COOR⁷ bedeutet, n₁ = n₃ = 0, R² = H, R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur anti-inflammatorischen Behandlung.

50. Verwendung einer Verbindung nach einem der Ansprüche 1 bis 39 einer Verbindung der Formel (I), in der Z eine Gruppe des Typs COOR⁷ bedeutet, n₁ = n₃ = 0, R² = H, R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur Anti-Arthritis-Behandlung.

51. Verwendung einer Verbindung nach einem der Ansprüche 1 bis 39 einer Verbindung der Formel (I), in der Z eine Gruppe des Typs COOR⁷ bedeutet, n₁ = n₃ = O, R² = H, R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur Anti-Psoriasis-Behandlung.

52. Verwendung einer Verbindung nach einem der Ansprüche 1 bis 39 einer Verbindung der Formel (I), in der Z eine Gruppe des Typs COOR⁷ bedeutet, n₁ = n₃ = 0, R² = H, R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur Leberschutz-Behandlung.

53. Verwendung einer Verbindung nach einem der Ansprüche 1 bis 39 einer Verbindung der Formel (I), in der Z eine Gruppe des Typs COOR⁷ bedeutet, n₁ = n₃ = 0, R² = H, R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur antimitotischen Behandlung.

54. Verwendung einer Verbindung nach einem der Ansprüche 1 bis 39 einer Verbindung der Formel (I), in der Z eine Gruppe des Typs COOR⁷ bedeutet, n₁ = n₃ = 0, R² = H. R¹ eine nichtsubstituierte, einfach oder mehrfach substituierte Phenylgruppe des Typs iv) bedeutet und n₂ = 1 ist, der α-Amino-β-phenoxy-propionsäure, der 3-Amino-7-phenyl-heptansäure, der 3-Amino-6-phenoxy-hexansäure, der α-Amino-6-phenyl-hexansäure oder der α-Amino-5-phenoxy-pentansäure für die Herstellung eines Arzneimittels zur Behandlung einer LTB₄-Überproduktion, die insbesondere durch einen Cyclooxygenase-Inhibitor ausgelöst worden ist.

55. Pharmazeutische Zubereitung, **dadurch gekennzeichnet, daß** sie als Wirkstoff eine therapeutisch wirksame Menge einer Verbindung nach einem der Ansprüche 1 bis 52 und eine therapeutisch wirksame Menge eines Cyclooxygenase-Inhibitors, gegebenenfalls in Kombination mit einem physiologisch annehmbaren Trägermaterial oder Bindemittel umfaßt.

## Claims

1. Compounds complying with the following formula (I): 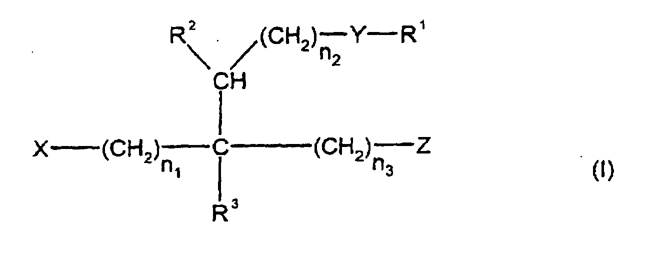 wherein
- X is selected from the following groups:
i)
―NH₂
ii) 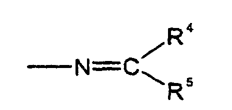
- n₁ and n₃ are equal to 0 or 1, with (n₁+n₃) equal to 0 or 1
- n₂ varies from 0 to 10
- Y is selected from the following groups:
i)
―O―
ii)
―CH₂―
iii)
―S―
iv)
―NH―
v)
―OCH₂―
- R¹ is selected from the following groups:
i) a hydrogen atom
ii) an alkyl group
iii) a cycloalkyl group
iv) a phenyl group, unsubstituted or mono- or poly-substituted with substituents selected from halogen atoms and CF₃ groups, alkyl containing 1 to 10 carbon atoms, alkoxy containing 1 to 10 carbon atoms, NH₂, NO₂, CN, OH, CO₂H, OPh, OCH₂Ph, SCH₃, SCH₂CH₃ and NHCOR⁶
v) an α- or β-naphthyl group
vi) an anthracene group
vii)
―A²―(CH₂)ₙ₄―A¹
where
n₄ varies from 0 to 4
A¹ and A² are independently selected from the following groups:
a) cycloalkyl
b) phenyl, unsubstituted or mono- or poly-substituted with substituents selected from halogen atoms and CF₃ groups, alkyl containing 1 to 10 carbon atoms and alkoxy containing 1 to 10 carbon atoms
c) 2-, 3- or 4-pyridyl
d) 2- or 3-thienyl
e) 2- or 3-furyl
f) 2-, 3- or 4-piperidyl
g) cycloalkene
viii) a 2-, 3- or 4-pyridyl group
ix) a 2- or 3-thienyl group
x) a 2- or 3-furyl group
xi) 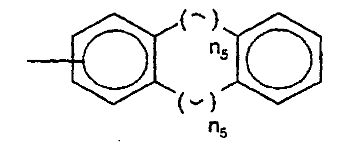
- Z is selected from the following groups:
i)
―COOR⁷
ii) 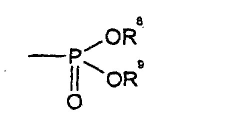
iii) 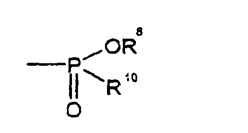
iv) 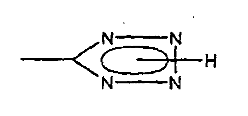
v)
―SO₃H
vi)
―SO₂NHR¹¹
vii)
―CONHSO₂R¹¹
- R² and R³ are independently selected from the following groups:
i) a hydrogen atom
ii) an alkyl group containing 1 to 10 carbon atoms
iii) an alkyl group containing 1 to 10 carbon atoms substituted by a halogen atom
iv) a CF₃ group
v) a halogen atom
- R⁴ and R⁵ are independently selected from a hydrogen atom, an alkyl group containing 1 to 10 carbon atoms, a phenyl group unsubstituted or substituted by a halogen atom, a CF₃ group, an alkyl group containing 1 to 10 carbon atoms, an alkoxy group containing 1 to 10 carbon atoms and an OH group;
- n₅ varies from 0 to 2
- R⁶ represents an alkyl group containing 1 to 10 carbon atoms
- R⁷ represents a hydrogen atom, an alkyl group containing 1 to 10 carbon atoms, a ―(CH₂)ₙ₆―Ph group, wherein n₆ varies from 0 to 4 and Ph is a phenyl group unsubstituted or mono- or poly-substituted by a halogen atom, a CF₃ group, an alkyl group containing 1 to 10 carbon atoms or an OH group;
- R⁸ and R⁹ are independently selected from a hydrogen atom, a phenyl group, an alkyl group containing 1 to 10 carbon atoms and an acetyl thioalkene group containing 1 to 10 carbon atoms
- R¹⁰ represents an alkyl group containing 1 to 10 carbon atoms, a ―(CH₂)ₙ₇―Ph group, wherein n₇ varies from 1 to 6 and Ph is a phenyl group unsubstituted or mono- or poly-substituted by a halogen atom, a CF₃ group, an alkyl group containing 1 to 10 carbon atoms or an alkoxy group containing 1 to 10 carbon atoms;
- R¹¹ represents an alkyl group containing 1 to 10 carbon atoms or a phenyl group;
as well as their isomers, diastereoisomers and enantiomers and their therapeutically acceptable salts,
with the following conditions:
(α) if Z is a group of the type COOR⁷ and n₁=n₃=0 and R²=H and R¹ is a iv) group of the unsubstituted or mono- or poly-substituted phenyl type, then n₂ cannot be equal to 1, and
(β) said compound is not α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid.

2. Compounds according to Claim 1, **characterised in that** R² and/or R³ represent a hydrogen atom.

3. Compounds according to Claim 2, **characterised in that** R² and R³ represent a hydrogen atom.

4. Compounds according to Claim 1, **characterised in that** R² and/or R³ are different from hydrogen.

5. Compounds according to one of Claims 1 to 4, **characterised in that** n₁ and n₃ are equal to 0.

6. Compounds according to one of Claims 1 to 4, **characterised in that** n₁ or n₃ is different from 0.

7. Compounds according to one of Claims 1 to 6, **characterised in that** X represents NH₂.

8. Compounds according to one of Claims 1 to 7, **characterised in that** X represents NH₂ and/or Z represents COOH.

9. Compounds according to one of Claims 1 to 8, **characterised in that** they comply with the following formula (II): 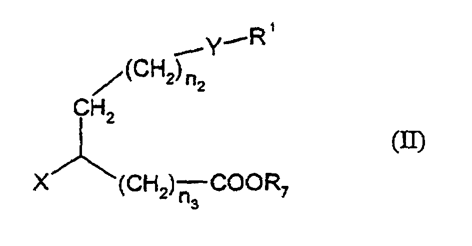 X₁, n₂, n₃, Y, R¹ and R⁷ having the meaning given in Claim 1.

10. Compounds according to one of Claims 1 to 9, **characterised in that** they comply with the following formula (III): 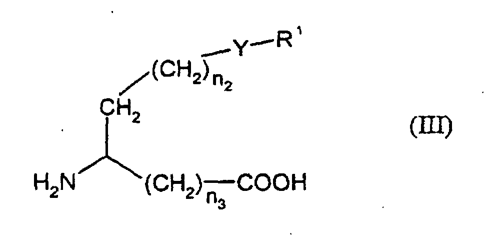 Y, n₂, n₃ and R¹ having the meaning given in Claim 1.

11. Compounds according to one of Claims 1 to 10, **characterised in that** they comply with the following formula (IV): 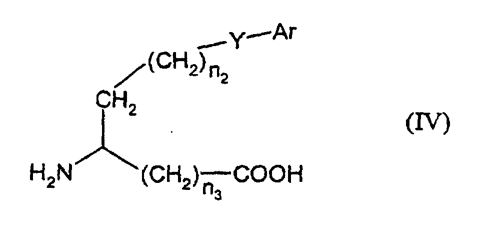 where Y, n₂ and n₃ have the meaning given above and Ar symbolises the R¹ group representing a possibly substituted phenyl group iv) as defined in Claim 1, or R¹ representing a vii) ―A²―(CH₂)ₙ₄―A¹ group, A² being a possibly substituted phenyl group (b) as defined in Claim 1.

12. Compounds according to one of Claims 1 to 7, **characterised in that** X represents NH₂ and/or Z represents 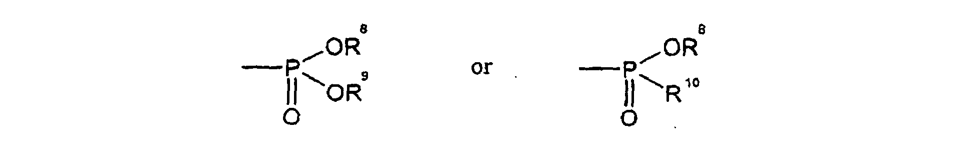 wherein R⁸, R⁹ are a hydrogen atom and R¹⁰ has the meaning given in Claim 1.

13. Compounds according to Claim 14, **characterised in that** X represents NH₂ and Z represents -PO(OH)₂.

14. Compounds according to one of Claims 1 to 7, 12 or 13, **characterised in that** they comply with the following formula (V): 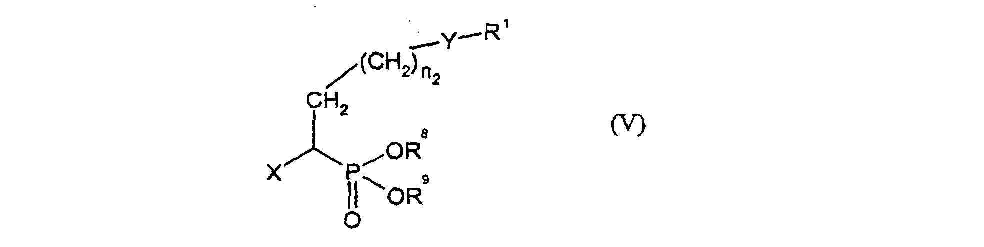 X, n₂, Y, R¹, R⁸ and R⁹ having the meaning given in Claim 1.

15. Compounds according to one of Claims 1 to 7 and 12 to 14, **characterised in that** they comply with the following formula (VI): 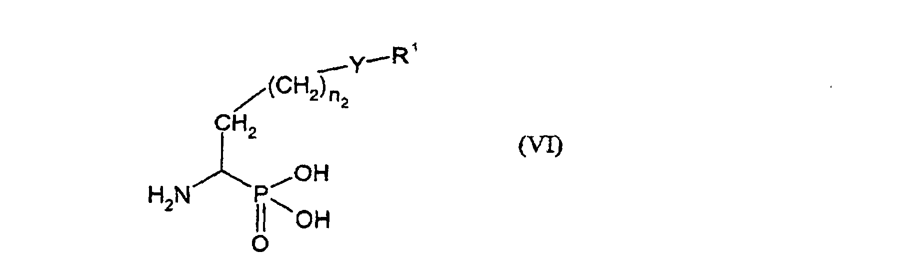 Y, n₂ and R¹ having the meaning indicated in Claim 1.

16. Compounds according to one of Claims 1 to 7 and 12 to 15, **characterised in that** they comply with the following formula (VII): 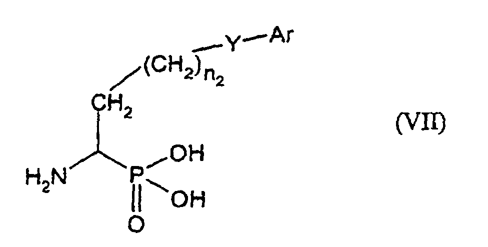 where Y and n₂ are as defined above, and Ar symbolises R¹ representing a possibly substituted phenyl group iv) as defined in Claim 1, or R¹ representing a vii) ―A²―(CH₂)ₙ₄―A¹ group, A² being a possibly substituted phenyl group (b) as defined in Claim 1.

17. Compounds according to one of Claims 1 to 7, **characterised in that** they comply with the following formula (VIII): 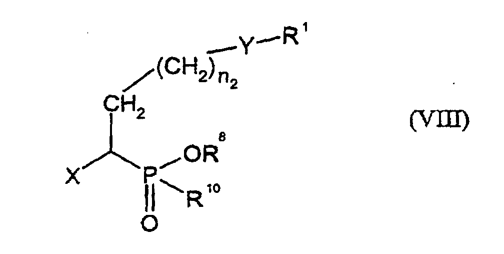 where X, Y, n₂, R¹, R⁸ and R¹⁰ have the meaning indicated in Claim 1.

18. Compounds according to one of Claims 1 to 17, **characterised in that** n₂ varies from 2 to 5.

19. Compounds according to Claim 18, **characterised in that** n₂ is equal to 3.

20. Compounds according to one of Claims 1 to 19, **characterised in that** Y represents an oxygen atom.

21. Compounds according to one of Claims 1 to 19, **characterised in that** Y represents ―CH₂―.

22. Compounds according to one of Claims 1 to 21, **characterised in that** R¹ represents an unsubstituted phenyl group.

23. Compounds according to one of Claims 1 to 10 and 18 to 21 and 31, **characterised in that** R¹ represents a phenyl group mono- or poly-substituted by a group selected from the alkyl group containing 1 to 10 carbon atoms, alkoxy group containing 1 to 10 carbon atoms, OPh and OCH₂Ph groups, preferably OPh.

24. Compounds according to one of Claims 1 to 21, **characterised in that** R¹ represents an ―A²―(CH₂)ₙ₄―A¹ unit.

25. Compounds according to Claim 24, **characterised in that** A² represents a phenyl group, preferably an unsubstituted phenyl group.

26. Compounds according to one of Claims 24 and 25, **characterised in that** n₄ is equal to 0 or 1.

27. Compounds according to one of Claims 1 to 13, 21 to 29 and 24 to 26, **characterised in that** A¹ is a phenyl, cycloalkyl or cycloalkene group.

28. Compounds according to one of Claims 24 to 27, **characterised in that** R¹ represents a phenyl group substituted by a Ph, CH₂Ph, CH₂-cycloalkyl or CH₂-cycloalkene group, preferably CH₂Ph or CH₂-cycloalkyl.

29. Compounds according to one of Claims 24 to 26, **characterised in that** A¹ is a phenyl group.

30. Compounds according to one of Claims 24 to 26 and 29, **characterised in that** R¹ represents a phenyl group substituted by a Ph or CH₂Ph group, preferably CH₂Ph.

31. Compounds according to one of Claims 1 to 21, **characterised in that** R¹ represents a hydrogen atom or an alkyl group containing 1 to 10 carbon atoms.

32. Compounds according to one of Claims 1 to 21, **characterised in that** R¹ represents 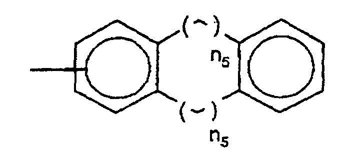 n₅ being defined as in Claim 1.

33. Compounds according to one of Claims 1 to 11 and 18 to 32, **characterised in that** they are selected from:
1) -(S)-O-4-benzyl phenoxy serine hydrochloride
2) 2-(RS)-amino-6-(4 benzyl phenoxy) hexanoic acid hydrobromide
3) 2-(RS)-amino-5-(4 benzyl phenoxy) pentanoic acid hydrobromide
4) 2-(RS)-amino-5-(4-phenoxy phenoxy) pentanoic acid hydrobromide
5) 2-(RS)-amino-7-(4-benzyl phenoxy) heptanoic acid hydrobromide
6) 2-(RS)-amino-6-(4-phenyl phenoxy) hexanoic acid hydrobromide
7) 2-(RS)-amino-6-(4-hexyloxy phenoxy) hexanoic acid hydrobromide
8) 2-(RS)-amino-8-(4-benzyl phenoxy) octanoic acid hydrobromide
9) 2-(RS)-amino-6-(4-phenoxy phenoxy) hexanoic acid hydrobromide
10) 2-(RS)-aminomethyl-6-(4-benzyl phenoxy) hexanoic acid
16) 2-(RS)-amino-6-(4-cyclohexylmethyl phenoxy) hexanoic acid hydrobromide
17) 3-(RS)-amino-7-(4-benzyl phenoxy) heptanoic acid
18) 2-(RS)-amino-2-methyl-6(4-benzyl phenoxy) hexanoic acid hydrobromide
20) 3-(RS)-amino-5-(4-benzyl phenoxy) pentanoic acid
21) 3-(RS)-amino-6-(4 benzyl phenoxy) hexanoic acid.

34. Compounds according to one of Claims 1 to 7, 12 to 32, **characterised in that** they are selected from:
11) 1-(RS)-amino-5-(phenoxy) pentyl phosphonic acid hydrobromide
12) 1-(RS)-amino-6-(phenoxy) hexyl phosphonic acid hydrobromide
13) 1-(RS)-amino-5-(4-benzyl phenoxy) pentyl phosphonic acid hydrobromide
14) 1-(RS)-amino-4-(phenoxy) butyl phosphonic acid hydrobromide
15) 1-(RS)-amino-7-(phenoxy) heptyl phosphonic acid hydrobromide
19) 1-(RS)-amino-tridecanyl phosphonic acid hydrobromide.

35. Compounds according to one of Claims 1 to 6, to 10, 12, 14, 17 to 32, **characterised in that** X represents 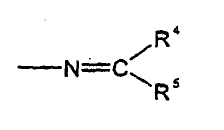 R⁴ and R⁵ having the meaning given in Claim 1.

36. Compounds according to one of Claims 1 to 7, 17 to 32 and 35, **characterised in that** R⁷ is different from hydrogen.

37. Compounds according to any one of Claims 1 to 7, 12, 14, 17 to 32 or 35, **characterised in that** R⁸ and/or R⁹ are independent and different from hydrogen.

38. Compounds according to any one of Claims 1 to 7, 17 to 32 or 35, **characterised in that** R⁸ and/or R¹⁰ are independent and R⁸ is different from hydrogen.

39. Compounds according to any one of Claims 35 to 38, **characterised in that** they are selected from
22) 2-(RS)-amino-7-(4 benzyl phenoxy) ethyl heptanoate hydrochloride
23) 2-(RS)-amino-6-(4-benzyl phenoxy) ethyl hexanoate hydrochloride
24) diphenyl 1-amino-5-phenoxy-pentyl phosphonate hydrobromide
25) ethyl-hydrogen-1-amino-5 phenoxy-pentyl phosphonate hydrobromide.

40. Pharmaceutical composition, **characterised in that** it contains as active principle a therapeutically effective quantity of a compound according to one of Claims 1 to 39 in combination with a physiologically acceptable vehicle or excipient.

41. Medication acting as inhibitor for LTA₄ hydrolase activity, **characterised in that** it contains as active principle a compound according to one of Claims 1 to 39.

42. Medication intended for an anti-inflammatory treatment, **characterised in that** it contains as active principle a compound according to one of Claims 1 to 39.

43. Medication intended for an anti-arthritic treatment, **characterised in that** it contains as active principle a compound according to one of Claims 1 to 39.

44. Medication intended for an anti-psoriasis treatment, **characterised in that** it contains as active principle a compound according to one of Claims 1 to 39.

45. Medication intended for a hepatoprotective treatment, **characterised in that** it contains as active principle a compound according to one of Claims 1 to 39.

46. Medication intended for an antimitotic treatment, **characterised in that** it contains as active principle a compound according to one of Claims 1 to 39.

47. Medication intended for treatment of an overproduction of LTB₄ induced in particular by cyclo-oxygenase inhibitors, **characterised in that** it contains as active principle a compound according to one of Claims 1 to 39.

48. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂=1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended to inhibit LTA₄ hydrolase activity.

49. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂ =1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended for an anti-inflammatory treatment.

50. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂ =1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended for an anti-arthritic treatment.

51. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂ =1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended for anti-psoriasis treatment.

52. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂ =1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended for a hepatoprotective treatment.

53. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂ =1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended for an antimitotic treatment.

54. Use of a compound according to one of Claims 1 to 39, of a compound with the formula (I) where Z is a group of the type COOR⁷, n₁=n₃=0, R²=H and R¹ is a phenyl group iv) of the unsubstituted or mono- or poly-substituted type and n₂ =1, of α-amino-β-phenoxy propionic acid, 3-amino-7-phenyl heptanoic acid, 3-amino-6-phenoxy hexanoic acid, α-amino-6-phenyl hexanoic acid or α-amino-5-phenoxy pentanoic acid, for the production of a medication intended for an overproduction of LTB₄ induced in particular by a cyclo-oxygenase inhibitor.

55. Pharmaceutical composition, **characterised in that** it contains as active principle a therapeutically effective quantity of a compound according to any one of Claims 1 to 52 and a therapeutically effective quantity of a cyclo-oxygenase inhibitor, possibly in combination with a physiologically acceptable vehicle or excipient.
